(19) Europäisches Patentamt
European Patent Office
Office européen des brevets

(11) EP 3 546 444 A1

(12) # EUROPEAN PATENT APPLICATION
published in accordance with Art. 153(4) EPC

(43) Date of publication:
**02.10.2019 Bulletin 2019/40**

(21) Application number: **17874935.4**

(22) Date of filing: **09.11.2017**

(51) Int Cl.:
***C07C 69/76*** *(2006.01)* ***C07C 69/75*** *(2006.01)*
***C07D 277/82*** *(2006.01)* ***C08F 20/38*** *(2006.01)*
***G02B 1/111*** *(2015.01)* ***G02B 5/30*** *(2006.01)*
***G02F 1/13363*** *(2006.01)* ***H01L 51/50*** *(2006.01)*
***H05B 33/02*** *(2006.01)*

(86) International application number:
**PCT/JP2017/040466**

(87) International publication number:
**WO 2018/096938 (31.05.2018 Gazette 2018/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.11.2016 JP 2016227091**
**29.08.2017 JP 2017164852**

(71) Applicant: **Zeon Corporation**
**Tokyo 100-8246 (JP)**

(72) Inventors:
- **SAKAMOTO, Kei**
  **Tokyo 100-8246 (JP)**
- **OKUYAMA, Kumi**
  **Tokyo 100-8246 (JP)**
- **MIMA, Takanori**
  **Tokyo 100-8246 (JP)**

(74) Representative: **Adam, Holger et al**
**Kraus & Weisert**
**Patentanwälte PartGmbB**
**Thomas-Wimmer-Ring 15**
**80539 München (DE)**

(54) **POLYMERIZABLE COMPOUND, POLYMERIZABLE COMPOSITION, POLYMER, OPTICAL FILM, OPTICALLY ANISOTROPIC BODY, POLARIZING PLATE, FLAT-PANEL DISPLAY DEVICE, ORGANIC ELECTROLUMINESCENCE DISPLAY DEVICE, ANTIREFLECTION FILM, AND COMPOUND**

(57) A polymerizable compound indicated by the following formula (I). In formula (I): Ar represents an optionally substituted aromatic hydrocarbon cyclic group/aromatic heterocyclic group; D represents an organic group having a carbon number of 1 to 67 and including an aromatic ring; $Z^1$, $Z^2$, and $Y^0$ to $Y^4$ each represent atomic bonding; $L^1$ and $L^2$ each represent a chain group; G represents an optionally substituted alicyclic group/aromatic group/alkylene group; $A^1$, $A^2$, $B^1$, and $B^2$ each represent an alicyclic group or an aromatic group; $P^1$ and $P^2$ each represent a hydrogen atom or a polymerizable group; and p and q are each, independently of one another, an integer of 0 to 2, with a proviso that cases in which p = 0 and q = 1 and in which p = 1 and q = 2 are excluded.

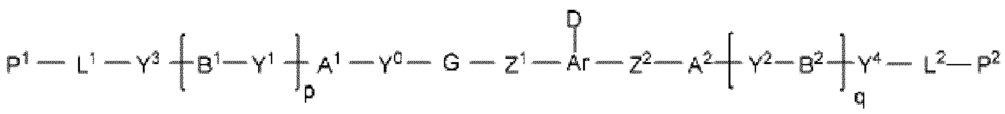

(I)

EP 3 546 444 A1

## Description

TECHNICAL FIELD

The present disclosure relates to an optical film and an optically anisotropic body, and to a polarizer, a flat panel display, an organic electroluminescence display, and an antireflection film in which the optically anisotropic body is used.

Moreover, the present disclosure relates to a polymer that can be used in production of the optical film and the optically anisotropic body, a polymerizable compound and a polymerizable composition containing the polymerizable compound that can be used in production of the polymer, and a compound that can be used in production of the polymerizable compound and the optical film.

BACKGROUND

Examples of retardation plates used in various devices such as flat panel displays include quarter-wave plates that convert linearly polarized light to circularly polarized light and half-wave plates that perform 90° conversion of the plane of vibration of linearly polarized light. Such retardation plates can accurately impart a retardation of 1/4λ or 1/2λ of the wavelength of light with respect to specific monochromatic light.

However, conventional retardation plates have a problem that polarized light that passes therethrough and is output therefrom is converted to colored polarized light. Specifically, since a constituent material of the retardation plate has a property of wavelength dispersion with respect to retardation, and a distribution arises in the polarization state of each wavelength for white light, which is a composite wave in which light in the visible region is mixed, it is impossible to achieve accurate adjustment to polarized light with a retardation of 1/4λ or 1/2λ over the entire wavelength region of input light.

In order to solve this problem, various retardation plates having a property referred to as "reverse wavelength dispersion" have been studied. These retardation plates are wideband retardation plates that can achieve uniform retardation with respect to light over a wide wavelength region.

On the other hand, enhanced performance and widespread use of mobile information terminals such as mobile personal computers and mobile phones has been accompanied by demand for thickness-reduction of flat panel displays to as great an extent as possible. Consequently, there has also been demand for thickness-reduction of retardation plates used as components thereof.

In terms of methods of achieving thickness-reduction, a method in which a retardation plate is produced by applying a polymerizable composition containing a low-molecular weight polymerizable compound onto a film substrate to form an optical film has been regarded as the most effective method in recent years. For this reason, there has been much development of polymerizable compounds that are capable of forming optical films that excel in terms of reverse wavelength dispersion, and also polymerizable compositions in which these compounds are used.

Specifically, polymerizable compounds and polymerizable compositions that are capable of forming optical films having excellent reverse wavelength dispersion have been proposed (for example, refer to PTL 1).

CITATION LIST

Patent Literature

PTL 1: WO 2014/010325 A1

SUMMARY

(Technical Problem)

In recent years, there has been a need to improve wavelength dispersion characteristics with respect to light of comparatively short wavelengths. However, it has not been possible to sufficiently improve wavelength dispersion characteristics at short wavelengths of optical films and the like obtained using conventional polymerizable compounds and polymerizable compositions such as described in PTL 1.

The present disclosure was completed in view of the circumstances set forth above and has an objective of providing a polymerizable compound that is useful in production of a polymerizable composition that is capable of forming an optical film or optically anisotropic body having good wavelength dispersion characteristics at short wavelengths.

Another objective of the present disclosure is to provide a polymerizable composition that is capable of forming an optical film or optically anisotropic body having good wavelength dispersion characteristics at short wavelengths.

Yet another objective of the present disclosure is to provide a compound that is useful in production of a polymerizable compound and an optical film.

(Solution to Problem)

As a result of diligent research conducted in order to solve the problem set forth above, the inventors discovered that by using a specific polymerizable compound indicated by formula (I), shown below, it is possible to form an optical film or the like having good wavelength dispersion characteristics at short wavelengths, and in this manner completed the present disclosure.

Accordingly, the present disclosure provides the following polymerizable compounds, polymerizable composition, polymer, optical films, optically anisotropic body, polarizer, flat panel display, organic electroluminescence display, antireflection film, and compounds.

[1] A polymerizable compound indicated by formula (I), shown below,

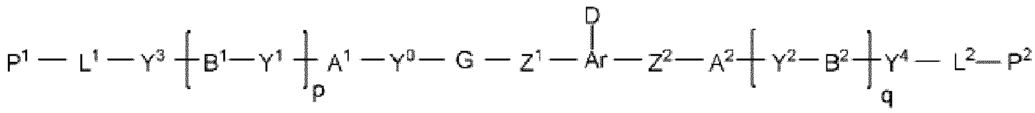

(I)

where, in formula (I):

Ar represents an optionally substituted aromatic hydrocarbon cyclic group or an optionally substituted aromatic heterocyclic group;

D represents an organic group having a carbon number of 1 to 67 and including at least one aromatic ring selected from the group consisting of an aromatic hydrocarbon ring and an aromatic heterocyclic ring;

$Y^0$, $Z^1$, and $Z^2$ each represent, independently of one another, a single bond, -O-, -O-$CH_2$-, -$CH_2$-O-, -O-$CH_2$-$CH_2$-, -$CH_2$-$CH_2$-O-, -C(=O)-O-, -O-C(=O)-, -C(=O)-S-, -S-C(=O)-, -$NR^{10}$-C(=O)-, -C(=O)-$NR^{10}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, -$CH_2$-C(=O)-O-, -O-C(=O)-$CH_2$-, -$CH_2$-O-C(=O)-, -C(=O)-O-$CH_2$-, or -C≡C-, where $R^{10}$ represents a hydrogen atom or an alkyl group having a carbon number of 1 to 6;

G represents an optionally substituted alicyclic group, an optionally substituted aromatic group, or an optionally substituted alkylene group;

$L^1$ and $L^2$ are each, independently of one another, an organic group that is either an alkylene group having a carbon number of 1 to 20 or a group in which at least one methylene group (-$CH_2$-) of an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups (-$CH_2$-) at both ends of $L^1$ and $L^2$ are not replaced by -O- or -C(=O)-;

$A^1$, $A^2$, $B^1$, and $B^2$ each represent, independently of one another, an optionally substituted alicyclic group or an optionally substituted aromatic group;

$Y^1$ to $Y^4$ each represent, independently of one another, a single bond, -O-, -C(=O)-, -C(=O)-O-, -O-C(=O)-, -$NR^{11}$-C(=O)-, -C(=O)-$NR^{11}$-, -O-C(=O)-O-, -$NR^{11}$-C(=O)-O-, -O-C(=O)-$NR^{11}$-, or -$NR^{11}$-C(=O)-$NR^{12}$-, where $R^{11}$ and $R^{12}$ each represent, independently of one another, a hydrogen atom or an alkyl group having a carbon number of 1 to 6;

one of $P^1$ and $P^2$ represents a hydrogen atom or a polymerizable group and the other of $P^1$ and $P^2$ represents a polymerizable group; and

p and q are each, independently of one another, an integer of 0 to 2, with a proviso that cases in which p = 0 and q = 1 and in which p = 1 and q = 2 are excluded.

[2] The polymerizable compound according to the foregoing [1], wherein Ar-D is indicated by any one of formulae (II-1) to (II-6), shown below,

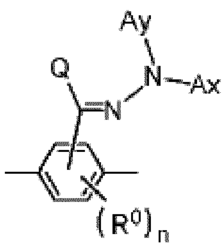

(II-1)

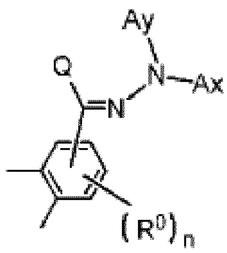

(II-2)

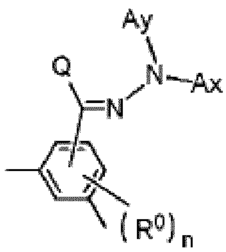

(II-3)

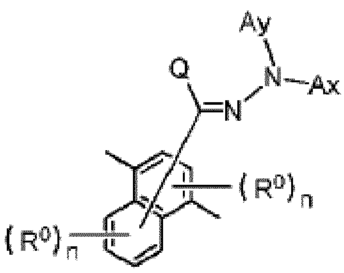

(II-4)

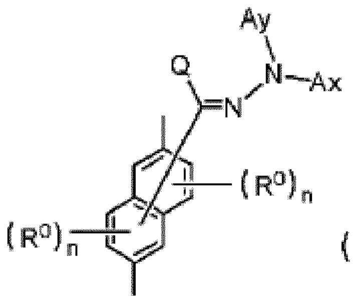

(II-5)

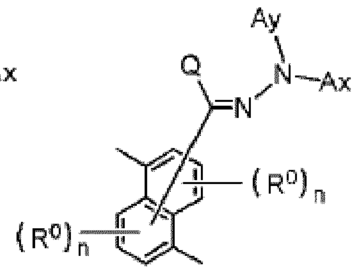

(II-6)

where, in formulae (II-1) to (II-6):

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted;
Ay represents a hydrogen atom or an optionally substituted organic group having a carbon number of 1 to 30;
Q represents a hydrogen atom or an alkyl group having a carbon number of 1 to 6; and
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-O-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4.

[3] The polymerizable compound according to the foregoing [2], wherein the polymerizable compound is indicated by formula (III-1) or (III-2), shown below,

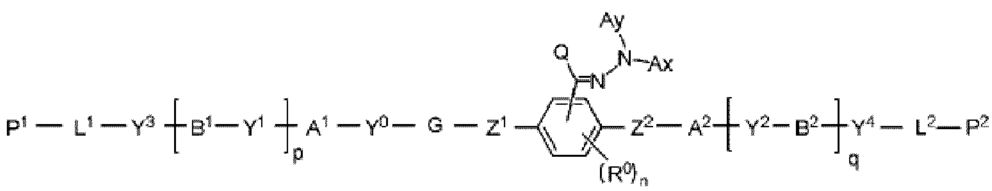

(III-1)

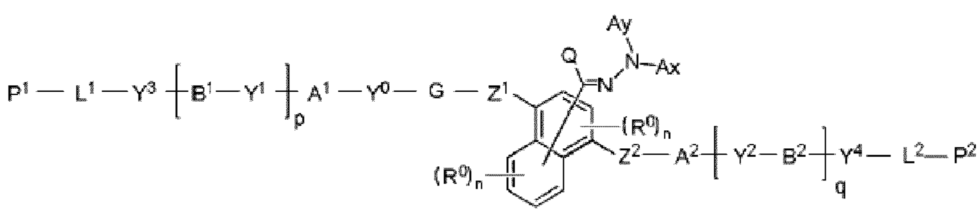

(III-2)

where, in formulae (III-1) and (III-2), $P^1$, $P^2$, $L^1$, $L^2$, $A^1$, $A^2$, $B^1$, $B^2$, $Y^0$ to $Y^4$, G, $Z^1$, $Z^2$, $R^0$, n, p, q, Ax, Ay, and Q have the same meaning as previously described.

[4] The polymerizable compound according to the foregoing [2] or [3], wherein Ay is a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 30, or an optionally substituted aromatic heterocyclic group having a carbon number of 2 to 30.

[5] The polymerizable compound according to any one of the foregoing [2] to [4], wherein Ax is indicated by formula (V), shown below,

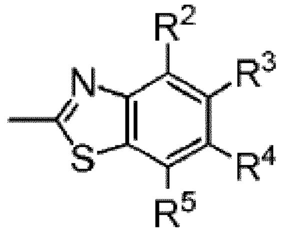

(V)

where, in formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, $C-R^2$ to $C-R^5$ may be the same or different, and one or more of ring constituents $C-R^2$ to $C-R^5$ may be replaced by a nitrogen atom.

[6] The polymerizable compound according to any one of the foregoing [1] to [5], wherein $P^1$ and $P^2$ are each, independently of one another, indicated by formula (IV), shown below,

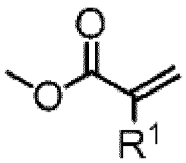

(IV)

where, in formula (IV), $R^1$ represents a hydrogen atom, a methyl group, or a chlorine atom.

[7] The polymerizable compound according to any one of the foregoing [1] to [6], wherein the polymerizable compound is indicated by formula (VI-1) or (VI-2), shown below,

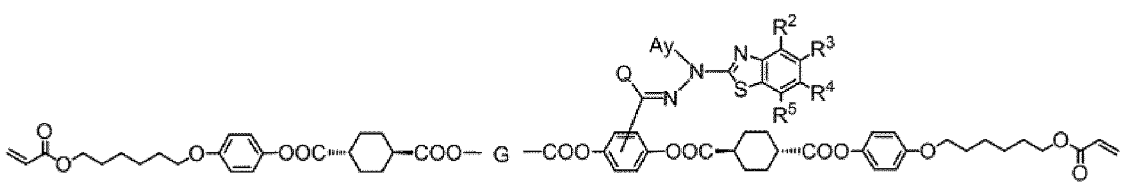

(VI-1)

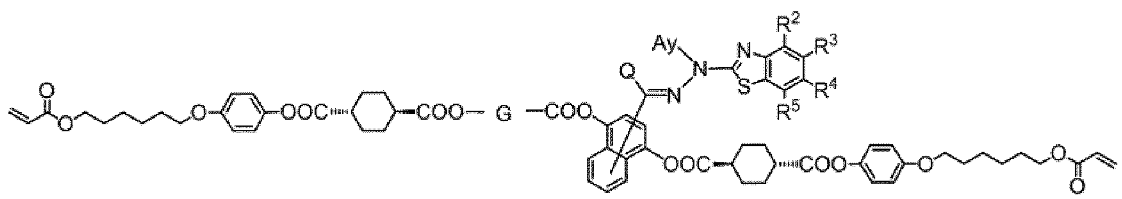

(VI-2)

where, in formulae (VI-1) and (VI-2):

$R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, $C-R^2$ to $C-R^5$ may be the same or different, and one or more of ring constituents $C-R^2$ to $C-R^5$ may be replaced by a nitrogen atom;
Ay represents a hydrogen atom or an optionally substituted organic group having a carbon number of 1 to 30;
Q represents a hydrogen atom or an alkyl group having a carbon number of 1 to 6; and
G represents an optionally substituted alicyclic group, an optionally substituted aromatic group, or an optionally substituted alkylene group.

[8] A polymerizable composition comprising:

the polymerizable compound according to any one of the foregoing [1] to [7]; and
a polymerization initiator.

[9] A polymer obtained by polymerizing the polymerizable compound according to any one of the foregoing [1] to [7].
[10] An optical film comprising the polymer according to the foregoing [9].
[11] An optical film comprising the polymerizable compound according to any one of the foregoing [1] to [7].
[12] An optically anisotropic body comprising a layer containing the polymer according to the foregoing [9].
[13] A polarizer comprising:

the optically anisotropic body according to the foregoing [12]; and
a polarizing film.

[14] A flat panel display comprising:

the polarizer according to the foregoing [13]; and
a liquid crystal panel.

[15] An organic electroluminescence display comprising:

the polarizer according to the foregoing [13]; and
an organic electroluminescence panel.

[16] An antireflection film comprising the polarizer according to the foregoing [13].
[17] A compound indicated by formula (VII-1) or (VII-2), shown below,

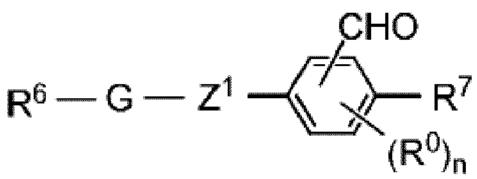

(VII-1)

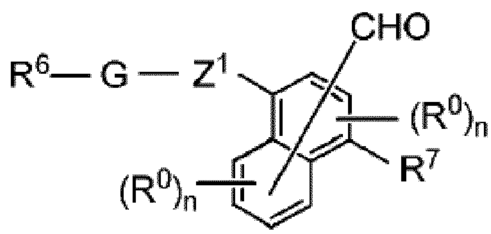

(VII-2)

where, in formulae (VII-1) and (VII-2):

$Z^1$ represents a single bond, -O-, $-O-CH_2-$, $-CH_2-O-$, $-O-CH_2-CH_2-$, $-CH_2-CH_2-O-$, -C(=O)-O-, -O-C(=O)-, -C(=O)-S-, -S-C(=O)-, $-NR^{10}-C(=O)-$, $-C(=O)-NR^{10}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$,

$-C(=O)-O-CH_2-CH_2-$, $-CH=CH-$, $-N=CH-$, $-CH=N-$, $-N=C(CH_3)-$, $-C(CH_3)=N-$, $-N=N-$, $-CH_2-C(=O)-O-$, $-O-C(=O)-CH_2-$, $-CH_2-O-C(=O)-$, $-C(=O)-O-CH_2-$, or $-C\equiv C-$, where $R^{10}$ represents a hydrogen atom or an alkyl group having a carbon number of 1 to 6;

G represents an optionally substituted alicyclic group, an optionally substituted aromatic group, or an optionally substituted alkylene group;

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4; and

$R^6$ and $R^7$ each represent, independently of one another, $-OR^e$, $-CH_2OR^e$, $-CH_2CH_2OR^e$, $-C(=O)-OR^e$, $-CH_2-C(=O)-OR^e$, $-CH_2CH_2-C(=O)-OR^e$, a hydroxy group, a carboxyl group, $-CH_2-C(=O)-OH$, $-CH_2CH_2-C(=O)-OH$, $-CH_2OH$, $-CH_2CH_2OH$, or an amino group, where $R^e$ represents a protecting group.

[18] A compound indicated by formula (VIII-1) or (VIII-2), shown below,

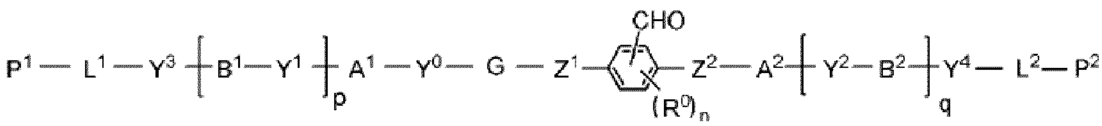

(VIII-1)

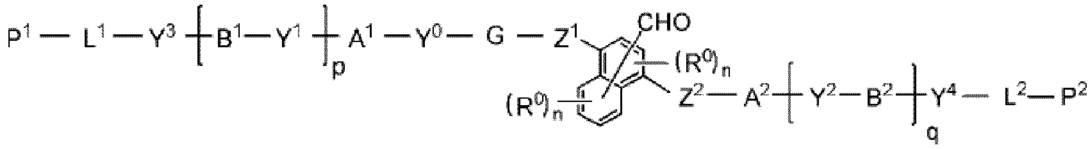

(VIII-2)

where, in formulae (VIII-1) and (VIII-2):

$Y^0$, $Z^1$, and $Z^2$ each represent, independently of one another, a single bond, -O-, $-O-CH_2-$, $-CH_2-O-$, $-O-CH_2-CH_2-$, $-CH_2-CH_2-O-$, $-C(=O)-O-$, $-O-C(=O)-$, $-C(=O)-S-$, $-S-C(=O)-$, $-NR^{10}-C(=O)-$, $-C(=O)-NR^{10}-$, $-CF_2-O-$, $-O-CF_2-$, $-CH_2-CH_2-$, $-CF_2-CF_2-$, $-O-CH_2-CH_2-O-$, $-CH=CH-C(=O)-O-$, $-O-C(=O)-CH=CH-$, $-CH_2-CH_2-C(=O)-O-$, $-O-C(=O)-CH_2-CH_2-$, $-CH_2-CH_2-O-C(=O)-$, $-C(=O)-O-CH_2-CH_2-$, $-CH=CH-$, $-N=CH-$, $-CH=N-$, $-N=C(CH_3)-$, $-C(CH_3)=N-$, $-N=N-$, $-CH_2-C(=O)-O-$, $-O-C(=O)-CH_2-$, $-CH_2-O-C(=O)-$, $-C(=O)-O-CH_2-$, or $-C\equiv C-$, where $R^{10}$ represents a hydrogen atom or an alkyl group having a carbon number of 1 to 6;

G represents an optionally substituted alicyclic group, an optionally substituted aromatic group, or an optionally substituted alkylene group;

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4;

$L^1$ and $L^2$ are each, independently of one another, an organic group that is either an alkylene group having a carbon number of 1 to 20 or a group in which at least one methylene group ($-CH_2-$) of an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-;

$A^1$, $A^2$, $B^1$, and $B^2$ each represent, independently of one another, an optionally substituted alicyclic group or an optionally substituted aromatic group;

$Y^1$ to $Y^4$ each represent, independently of one another, a single bond, -O-, -C(=O)-, -C(=O)-O-, -O-C(=O)-, $-NR^{11}-C(=O)-$, $-C(=O)-NR^{11}-$, -O-C(=O)-O-, $-NR^{11}-C(=O)-O-$, $-O-C(=O)-NR^{11}-$, or $-NR^{11}-C(=O)-NR^{12}-$, where $R^{11}$ and $R^{12}$ each represent, independently of one another, a hydrogen atom or an alkyl group having a carbon number of 1 to 6;
one of $P^1$ and $P^2$ represents a hydrogen atom or a polymerizable group and the other of $P^1$ and $P^2$ represents a polymerizable group; and
p and q are each, independently of one another, an integer of 0 to 2, with a proviso that cases in which p = 0 and q = 1 and in which p = 1 and q = 2 are excluded.

[19] A compound indicated by formula (X-1) or (X-2), shown below,

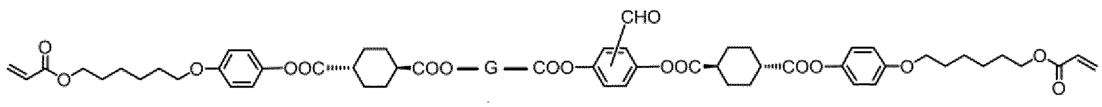

(X-1)

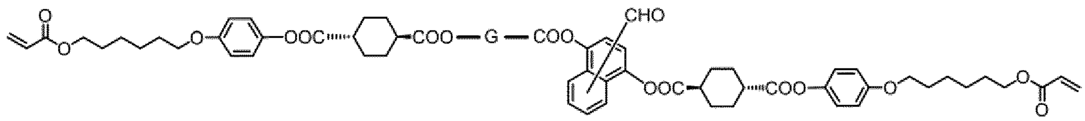

(X-2)

where, in formulae (X-1) and (X-2), G represents an optionally substituted alicyclic group, an optionally substituted aromatic group, or an optionally substituted alkylene group.
[20] A compound indicated by any one of formulae (XI-1) to (XI-7), shown below.

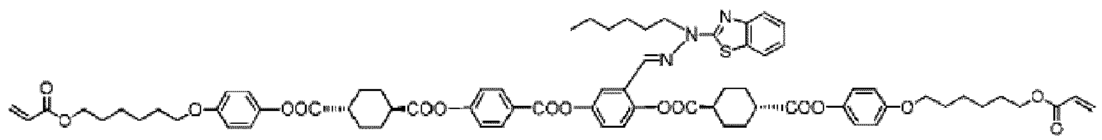

(XI-1)

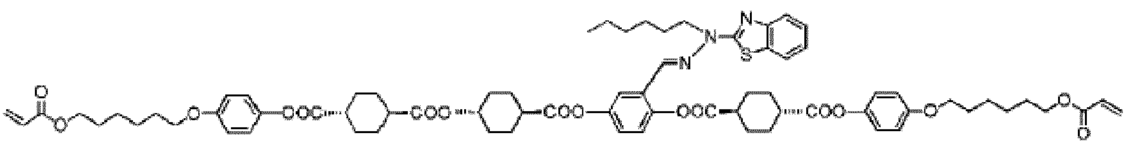

(XI-2)

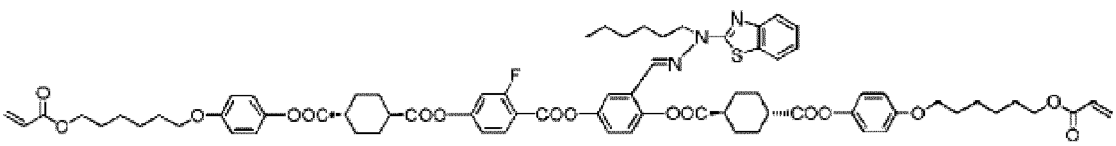

(XI-3)

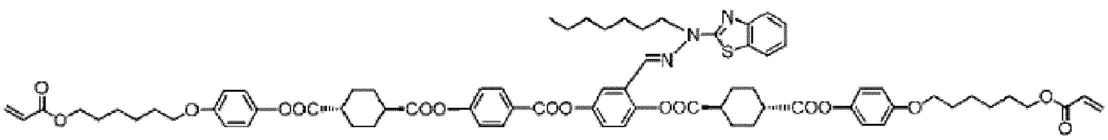

(XI-4)

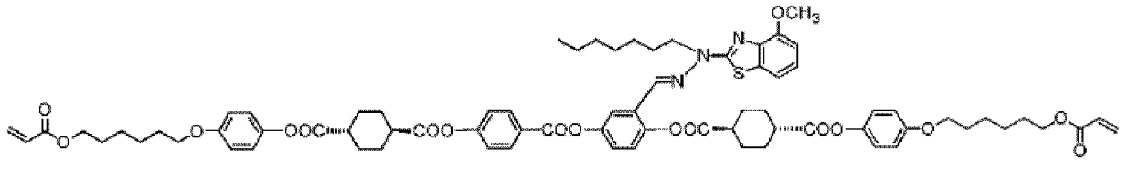

(XI-5)

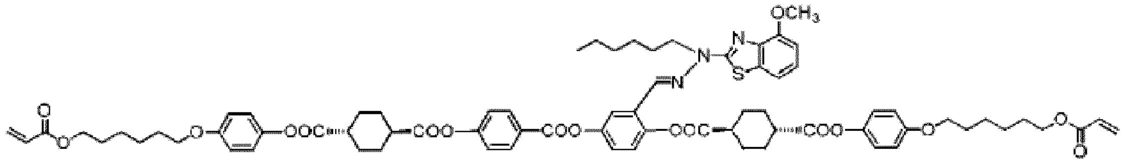

(XI-6)

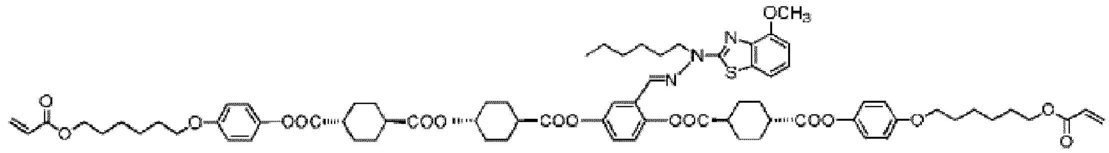

(XI-7)

(Advantageous Effect)

The present disclosure provides a polymerizable compound that is useful in production of an optical film and is useful in production of a polymerizable composition capable of forming an optical film or optically anisotropic body having good wavelength dispersion characteristics at short wavelengths.

Moreover, the present disclosure provides a polymerizable composition that is capable of forming an optical film or optically anisotropic body having good wavelength dispersion characteristics at short wavelengths.

Furthermore, the present disclosure provides a compound that is useful in production of the aforementioned polymerizable compound.

Also, the present disclosure provides an optical film and an optically anisotropic body having good wavelength dispersion characteristics at short wavelengths, and a polarizer, a flat panel display, an organic electroluminescence (EL) display, and an antireflection film in which the optical film and the optically anisotropic body are used.

DETAILED DESCRIPTION

The following provides a detailed description of the present disclosure. Note that the phrase "optionally substituted" as used in the present disclosure means "unsubstituted or having one or more substituents". Also note that in a case in which an organic group (for example, an alkyl group or an aromatic hydrocarbon cyclic group) included in a general formula has a substituent, the carbon number of the substituted organic group is taken to be exclusive of the carbon number of the substituent. For example, in a case in which an aromatic hydrocarbon cyclic group having a carbon number of 6 to 30 has a substituent, the carbon number of the aromatic hydrocarbon cyclic group having a carbon number of 6 to 30 is taken to be exclusive of the carbon number of the substituent. Moreover, the term "alkyl group" as used in the present disclosure refers to chain (linear or branched) saturated hydrocarbon groups and is not inclusive of "cycloalkyl groups", which are cyclic saturated hydrocarbon groups.

A presently disclosed polymerizable compound can be used in production of a presently disclosed polymerizable composition and a presently disclosed optical film, for example, but is not specifically limited to being used in this manner.

Moreover, the presently disclosed polymerizable composition can be used in production of a presently disclosed polymer, for example, but is not specifically limited to being using in this manner.

The presently disclosed polymer can be used as a constituent material of the presently disclosed optical film or as a constituent material of a layer included in a presently disclosed optically anisotropic body, for example, but is not specifically limited to being used in this manner. Moreover, the presently disclosed optically anisotropic body can be used in a presently disclosed polarizer, for example, but is not specifically limited to being used in this manner. Furthermore, the presently disclosed polarizer can be used in a presently disclosed flat panel display, a presently disclosed organic electroluminescence display, or a presently disclosed antireflection film, for example, but is not specifically limited to being used in this manner.

Also, a presently disclosed compound can be used in production of the presently disclosed polymerizable compound, for example, but is not specifically limited to being used in this manner.

(1) Polymerizable compound

The presently disclosed polymerizable compound is a compound indicated by the following formula (I) (hereinafter, also referred to as "polymerizable compound (I)") and can advantageously be used in production of a polymer, an optical film, and an optically anisotropic body described further below.

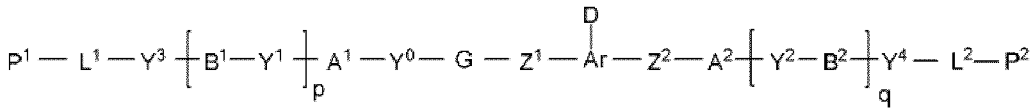

( I )

Note that by using the polymerizable compound (I), it is possible to obtain a polymerizable composition that can advantageously be used in production of an optical film or the like having good wavelength dispersion characteristics at short wavelengths as described further below.

Although the reason is not clear, this is thought to be due to the polymerizable compound (I) having an asymmetrical structure. When the polymerizable compound (I) is described as having an asymmetrical structure, this means that the two chain sections bonded to the aromatic hydrocarbon cyclic group or aromatic heterocyclic group (Ar) having D as a substituent in the polymerizable compound (I) differ from one another. Wavelength dispersion characteristics at short wavelengths can be improved through the polymerizable compound (I) having an asymmetrical structure.

In formula (I), Ar is an optionally substituted aromatic hydrocarbon cyclic group or an optionally substituted aromatic heterocyclic group. D is an organic group having a carbon number of 1 to 67 and including at least one aromatic ring selected from the group consisting of an aromatic hydrocarbon ring and an aromatic heterocyclic ring.

Examples of the aromatic hydrocarbon cyclic group of Ar include a 1,4-phenylene group, a 1,3-phenylene group, a 1,4-naphthylene group, a 2,6-naphthylene group, a 1,5-naphthylene group, an anthracenyl-9,10-diyl group, an anthracenyl-1,4-diyl group, and an anthracenyl-2,6-diyl group.

Of these aromatic hydrocarbon cyclic groups, a 1,4-phenylene group, a 1,4-naphthylene group, or a 2,6-naphthylene group is preferable, and a 1,4-phenylene group is particularly preferable.

Examples of the aromatic heterocyclic group of Ar include a benzothiazole-4,7-diyl group, a 1,2-benzisothiazole-4,7-diyl group, a benzoxazole-4,7-diyl group, an indonyl-4,7-diyl group, a benzimidazole-4,7-diyl group, a benzopyrazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo [1,2-b:5,4-b']dithiophenyl-4,8-diyl group, a benzo [1,2-b:4,5-b']dithiophenyl-4,8-diyl group, a benzo[1,2-b:5,4-b']difuranyl-4,8-diyl group, a benzo[1,2-b:4,5-b']difuranyl-4,8-diyl group, a benzo[2,1-b:4,5-b']dipyrrole-4,8-diyl group, a benzo[1,2-b:5,4-b']dipyrrole-4,8-diyl group, and a benzo[1,2-d:4,5-d']diimidazole-4,8-diyl group.

Of these aromatic heterocyclic groups, a benzothiazole-4,7-diyl group, a benzoxazole-4,7-diyl group, a 1-benzofuran-4,7-diyl group, a 2-benzofuran-4,7-diyl group, a benzo[1,2-d:4,5-d']dithiazolyl-4,8-diyl group, a benzo[1,2-d:5,4-d']dithiazolyl-4,8-diyl group, a benzothiophenyl-4,7-diyl group, a 1H-isoindole-1,3(2H)-dione-4,7-diyl group, a benzo[1,2-b:5,4-b']dithiophenyl-4,8-diyl group, a benzo[1,2-b:4,5-b']dithiophenyl-4,8-diyl group, a benzo[1,2-b:5,4-b']difuranyl-4,8-diyl group, or a benzo[1,2-b:4,5-b']difuranyl-4,8-diyl group is preferable.

The aromatic hydrocarbon cyclic group or aromatic heterocyclic group of Ar may have a subsequently described substituent $R^0$.

The term "aromatic ring" as used in the present description refers to a cyclic structure having aromaticity in the broad sense according to Huckel's law. In other words, "aromatic ring" refers to cyclic conjugated structures including 4n + 2 $\pi$-electrons and cyclic structures that display aromaticity through the contribution of a lone pair of electrons of a heteroatom such as sulfur, oxygen, or nitrogen to the $\pi$-electron system, representative examples of which include thiophenes, furans, and benzothiazoles.

The total number ($N_{Ar}$ + $N_D$) of the number of $\pi$-electrons included in Ar ($N_{Ar}$) and the number of $\pi$-electrons included in D ($N_D$) is normally 12 or more, preferably at least 12 and not more than 36, and more preferably at least 12 and not more than 30.

Examples of the aromatic hydrocarbon ring of D include a benzene ring, a naphthalene ring, an anthracene ring, a phenanthrene ring, a pyrene ring, and a fluorene ring.

Of these aromatic hydrocarbon rings, a benzene ring, a naphthalene ring, or an anthracene ring is preferable.

Examples of the aromatic heterocyclic ring of D include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an

oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a monocyclic aromatic heterocyclic ring such as a furan ring, a pyran ring, a thiophene ring, an oxazole ring, an oxadiazole ring, a thiazole ring, or a thiadiazole ring, or a fused ring aromatic heterocyclic ring such as a benzothiazole ring, a benzoxazole ring, a quinoline ring, a 1-benzofuran ring, a 2-benzofuran ring, a benzothiophene ring, a 1H-isoindole-1,3(2H)-dione ring, a benzo[c]thiophene ring, a thiazolopyridine ring, a thiazolopyrazine ring, a benzisoxazole ring, a benzoxadiazole ring, or a benzothiadiazole ring is preferable.

The organic group constituting D that has a carbon number of 1 to 67 and includes at least one aromatic ring selected from the group consisting of an aromatic hydrocarbon ring and an aromatic heterocyclic ring may be, but is not specifically limited to, an optionally substituted aromatic hydrocarbon cyclic group, an optionally substituted aromatic heterocyclic group, or a group represented by a formula: $-C(R^f)=N-N(R^g)R^h$ or a formula: $-C(R^f)=N-N=C(R^{f1})R^h$.

In the preceding formulae, $R^f$ and $R^{f1}$ each represent, independently of one another, a hydrogen atom or an alkyl group having a carbon number of 1 to 6 such as a methyl group, an ethyl group, a propyl group, or an isopropyl group.

Moreover, $R^g$ in the preceding formulae represents a hydrogen atom or an optionally substituted organic group having a carbon number of 1 to 30. Examples of the organic group having a carbon number of 1 to 30 and substituents thereof include the same specific examples as subsequently listed for an organic group of Ay having a carbon number of 1 to 30 and substituents thereof.

Furthermore, $R^h$ in the preceding formulae represents an organic group including at least one aromatic ring selected from the group consisting of an aromatic hydrocarbon ring having a carbon number of 6 to 30 and an aromatic heterocyclic ring having a carbon number of 2 to 30. Specific examples of the organic group including at least one aromatic ring selected from the group consisting of an aromatic hydrocarbon ring having a carbon number of 6 to 30 and an aromatic heterocyclic ring having a carbon number of 2 to 30 include the same specific examples as subsequently listed for an organic group of Ax including at least one aromatic ring selected from the group consisting of an aromatic hydrocarbon ring having a carbon number of 6 to 30 and an aromatic heterocyclic ring having a carbon number of 2 to 30.

Specific examples of aromatic hydrocarbon cyclic groups that may constitute D include a phenyl group, a naphthyl group, an anthracenyl group, a phenanthrenyl group, a pyrenyl group, and a fluorenyl group.

Of these aromatic hydrocarbon cyclic groups, a phenyl group, a naphthyl group, or an anthracenyl group is preferable.

Examples of aromatic heterocyclic groups that may constitute D include a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxadiazolyl group, a benzoxazolyl group, a benzothiadiazolyl group, a benzothiazolyl group, a benzothienyl group, a benzotriazinyl group, a benzotriazolyl group, a benzopyrazolyl group, a benzopyranonyl group, a dihydropyranyl group, a tetrahydropyranyl group, a dihydrofuranyl group, and a tetrahydrofuranyl group.

Of these aromatic heterocyclic groups, a monocyclic aromatic heterocyclic group such as a furanyl group, a pyranyl group, a thienyl group, an oxazolyl group, a furazanyl group, a thiazolyl group, or a thiadiazolyl group, or a fused ring aromatic heterocyclic group such as a benzothiazolyl group, a benzoxazolyl group, a quinolyl group, a 1-benzofuranyl group, a 2-benzofuranyl group, a benzothienyl group, a phthalimide group, a benzo[c]thienyl group, a thiazolopyridyl group, a thiazolopyrazinyl group, a benzisoxazolyl group, a benzoxadiazolyl group, or a benzothiadiazolyl group is preferable.

The aromatic hydrocarbon ring or aromatic heterocyclic ring of D and the aromatic hydrocarbon cyclic group or aromatic heterocyclic group constituting D may have one or more substituents.

Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon

number of 1 to 6 such as a trifluoromethyl group; N,N-dialkylamino groups having a carbon number of 1 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^{b1}$; $-O-C(=O)-R^{b1}$; $-C(=O)-O-R^{b1}$; and $-SO_2R^a$. $R^{b1}$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18. The optionally substituted aromatic hydrocarbon cyclic group having a carbon number of 5 to 18 is preferably an optionally substituted aromatic hydrocarbon cyclic group having a carbon number of 5 to 12. $R^a$ represents an alkyl group having a carbon number of 1 to 6 such as a methyl group or an ethyl group; or an aromatic hydrocarbon cyclic group having a carbon number of 6 to 20 that is optionally substituted with an alkyl group having a carbon number of 1 to 6 or an alkoxy group having a carbon number of 1 to 6 (for example, a phenyl group, a 4-methylphenyl group, or a 4-methoxyphenyl group). Of these examples, halogen atoms, a cyano group, a nitro group, alkyl groups having a carbon number of 1 to 6, alkoxy groups having a carbon number of 1 to 6, and haloalkyl groups having a carbon number of 1 to 6 are preferable as substituents of the aromatic hydrocarbon ring or aromatic heterocyclic ring of D or an aromatic ring included in the aromatic hydrocarbon cyclic group or aromatic heterocyclic group constituting D.

The aromatic hydrocarbon ring or aromatic heterocyclic ring of D and the aromatic hydrocarbon cyclic group or aromatic heterocyclic group constituting D may have a plurality of substituents selected from the substituents described above. In a case in which the aromatic hydrocarbon ring, aromatic heterocyclic ring, aromatic hydrocarbon cyclic group, or aromatic heterocyclic group includes a plurality of substituents, these substituents may be the same or different.

Examples of the alkyl group having a carbon number of 1 to 20 and substituents thereof in the optionally substituted alkyl group having a carbon number of 1 to 20 of $R^{b1}$, the alkenyl group having a carbon number of 2 to 20 and substituents thereof in the optionally substituted alkenyl group having a carbon number of 2 to 20 of $R^{b1}$, the cycloalkyl group having a carbon number of 3 to 12 and substituents thereof in the optionally substituted cycloalkyl group having a carbon number of 3 to 12 of $R^{b1}$, and the aromatic hydrocarbon cyclic group having a carbon number of 5 to 18 and substituents thereof in the optionally substituted aromatic hydrocarbon cyclic group having a carbon number of 5 to 18 of $R^{b1}$ include the same specific examples as subsequently listed for an alkyl group having a carbon number of 1 to 20 and substituents thereof in an optionally substituted alkyl group having a carbon number of 1 to 20 of $R^b$, an alkenyl group having a carbon number of 2 to 20 and substituents thereof in an optionally substituted alkenyl group having a carbon number of 2 to 20 of $R^b$, a cycloalkyl group having a carbon number of 3 to 12 and substituents thereof in an optionally substituted cycloalkyl group having a carbon number of 3 to 12 of $R^b$, and an aromatic hydrocarbon cyclic group having a carbon number of 5 to 18 and substituents thereof in an optionally substituted aromatic hydrocarbon cyclic group having a carbon number of 5 to 18 of $R^b$.

Examples of combinations of Ar and D (Ar-D) set forth above include a phenylene group substituted with a group represented by a formula: $-C(R^f)=N-N(R^g)R^h$ or a formula: $-C(R^f)=N-N=C(R^{f1})R^h$, a benzothiazole-4,7-diyl group substituted with a 1-benzofuran-2-yl group, a benzothiazole-4,7-diyl group substituted with a 5-(2-butyl)-1-benzofuran-2-yl group, a benzothiazole-4,7-diyl group substituted with a 4,6-dimethyl-1-benzofuran-2-yl group, a benzothiazole-4,7-diyl group substituted with a 6-methyl-1-benzofuran-2-yl group, a benzothiazole-4,7-diyl group substituted with a 4,6,7-trimethyl-1-benzofuran-2-yl group, a benzothiazole-4,7-diyl group substituted with a 4,5,6-trimethyl-1-benzofuran-2-yl group, a benzothiazole-4,7-diyl group substituted with a 5-methyl-1-benzofuran-2-yl group, a benzothiazole-4,7-diyl group substituted with a 5-propyl-1-benzofuran-2-yl group, a benzothiazole-4,7-diyl group substituted with a 7-propyl-1-benzofuran-2-yl group, a benzothiazole-4,7-diyl group substituted with a 5-fluoro-1-benzofuran-2-yl group, a benzothiazole-4,7-diyl group substituted with a phenyl group, a benzothiazole-4,7-diyl group substituted with a 4-fluorophenyl group, a benzothiazole-4,7-diyl group substituted with a 4-nitrophenyl group, a benzothiazole-4,7-diyl group substituted with a 4-trifluoromethylphenyl group, a benzothiazole-4,7-diyl group substituted with a 4-cyanophenyl group, a benzothiazole-4,7-diyl group substituted with a 4-methanesulfonyl-phenyl group, a benzothiazole-4,7-diyl group substituted with a thiophene-2-yl group, a benzothiazole-4,7-diyl group substituted with a thiophene-3-yl group, a benzothiazole-4,7-diyl group substituted with a 5-methylthiophene-2-yl group, a benzothiazole-4,7-diyl group substituted with a 5-chlorothiophene-2-yl group, a benzothiazole-4,7-diyl group substituted with a thieno[3,2-b]thiophene-2-yl group, a benzothiazole-4,7-diyl group substituted with a 2-benzothiazolyl group, a benzothiazole-4,7-diyl group substituted with a 4-biphenyl group, a benzothiazole-4,7-diyl group substituted with a 4-propylbiphenyl group, a benzothiazole-4,7-diyl group substituted with a 4-thiazolyl group, a benzothiazole-4,7-diyl group substituted with a 1-phenylethylene-2-yl group, a benzothiazole-4,7-diyl group substituted with a 4-pyridyl group, a benzothiazole-4,7-diyl group substituted with a 2-furyl group, a benzothiazole-4,7-diyl group substituted with a naphtho[1,2-b]furan-2-yl group, a 1H-isoindole-1,3(2H)-dione-4,7-diyl group substituted with a 5-methoxy-2-benzothiazolyl group, a 1H-isoindole-1,3(2H)-dione-4,7-diyl group substituted with a phenyl group, a 1H-isoindole-1,3(2H)-dione-4,7-diyl group substituted with a 4-nitrophenyl group, and a 1H-isoindole-1,3(2H)-dione-4,7-diyl group substituted with a 2-thiazolyl group. $R^f$, $R^{f1}$, $R^g$, and $R^h$ have the same meaning here as previously described.

Ar-D is preferably a group indicated by any one of the following formulae (II-1) to (II-6).

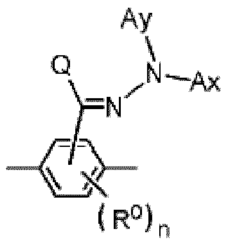

(II-1)

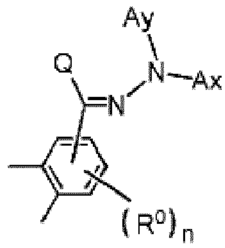

(II-2)

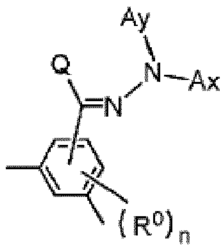

(II-3)

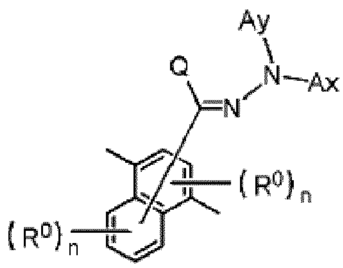

(II-4)

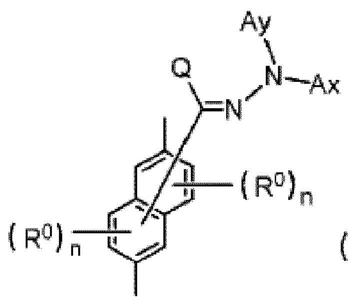

(II-5)

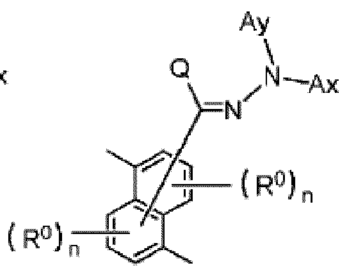

(II-6)

In formulae (II-1) to (II-6): Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted; Ay represents a hydrogen atom or an optionally substituted organic group having a carbon number of 1 to 30; and Q represents a hydrogen atom or an alkyl group having a carbon number of 1 to 6. Examples of the alkyl group having a carbon number of 1 to 6 of Q include a methyl group, an ethyl group, an n-propyl group, and an isopropyl.

$R^0$ represents a halogen atom; a cyano group; an alkyl group having a carbon number of 1 to 6 such as a methyl group, an ethyl group, a propyl group, an isopropyl group, a butyl group, a sec-butyl group, or a tert-butyl group; an alkenyl group having a carbon number of 2 to 6; a haloalkyl group having a carbon number of 1 to 6; an N,N-dialkylamino group having a carbon number of 2 to 12; an alkoxy group having a carbon number of 1 to 6; a nitro group; $-C(=O)-R^a$; $-O-C(=O)-R^a$; $-C(=O)-O-R^a$; or $-SO_2R^a$, where $R^a$ represents an alkyl group having a carbon number of 1 to 6 such as a methyl group or an ethyl group; or an aromatic hydrocarbon cyclic group having a carbon number of 6 to 20 that is optionally substituted with an alkyl group having a carbon number of 1 to 6 or an alkoxy group having a carbon number of 1 to 6 (for example, a phenyl group, a 4-methylphenyl group, or a 4-methoxyphenyl group). In a case in which a plurality of substituents is present, these substituents may be the same or different. From a viewpoint of improving solubility, a halogen atom, a cyano group, an alkyl group having a carbon number of 1 to 6, a haloalkyl group having a carbon number of 1 to 6, an alkoxy group having a carbon number of 1 to 6, or a nitro group is preferable as $R^0$.

Moreover, each n is independently an integer of 0 to 4. A case in which n = 0 is preferable.

Structures represented by the following formulae (II-7) to (II-23) are more preferable as Ar-D. Note that in the following formulae, $Z^1$ and $Z^2$ are also included for convenience in order to clarify the form of bonding. $Z^1$, $Z^2$, Ax, Ay, Q, $R^0$, and n in the formulae have the same meaning as previously described. Of the following formulae, formulae (II-7), (II-8), (11-16), and (II-18) are particularly preferable.

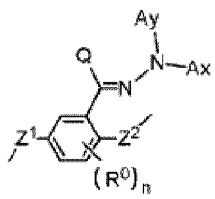 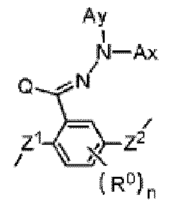 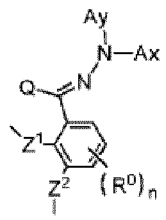 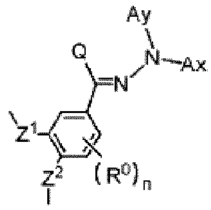

(II-7) (II-8) (II-9) (II-10)

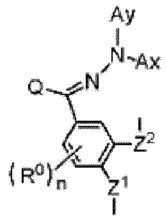 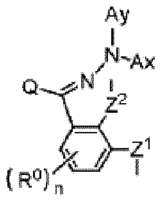 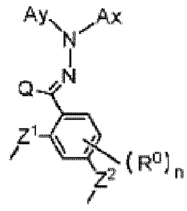

(II-11) (II-12) (II-13)

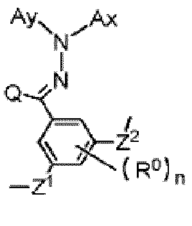 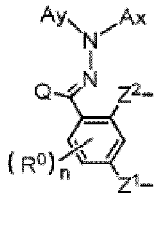 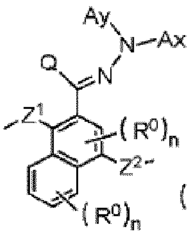 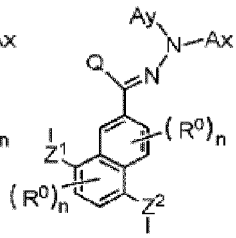 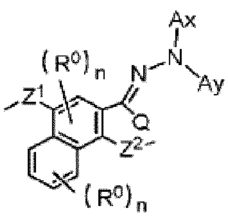

(II-14) (II-15) (II-16) (II-17) (II-18)

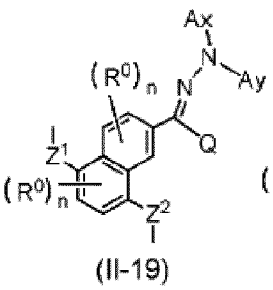 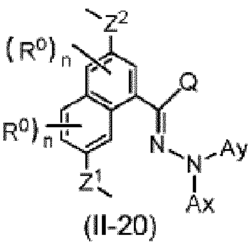 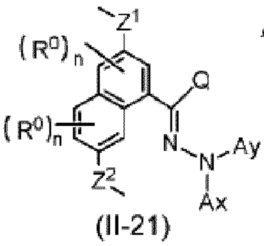 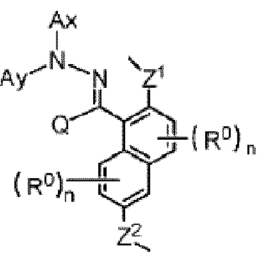

(II-19) (II-20) (II-21) (II-22)

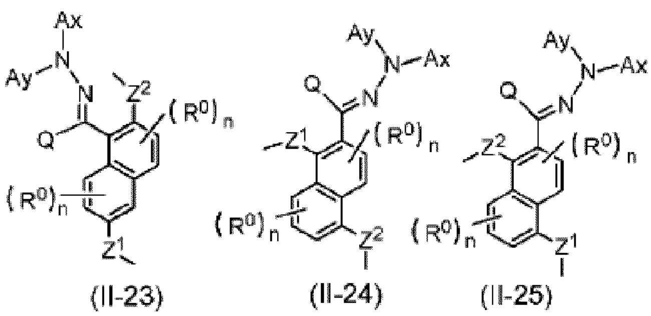

(II-23) (II-24) (II-25)

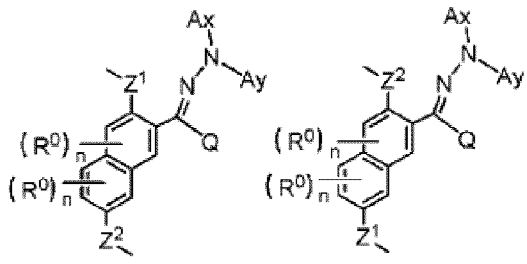

(II-26) (II-27)

The organic group of Ar that includes at least one aromatic ring selected from the group consisting of an aromatic hydrocarbon ring having a carbon number of 6 to 30 and an aromatic heterocyclic ring having a carbon number of 2 to 30 may include a plurality of aromatic rings and may include both an aromatic hydrocarbon ring and an aromatic heterocyclic ring. In a case in which the organic group includes a plurality of aromatic hydrocarbon rings or aromatic heterocyclic rings, these rings may be the same or different.

Examples of aromatic hydrocarbon rings that may be included in Ax include a benzene ring, a naphthalene ring, an anthracene ring, a phenanthrene ring, a pyrene ring, and a fluorene ring.

Of these aromatic hydrocarbon rings, a benzene ring, a naphthalene ring, or an anthracene ring is preferable.

Examples of aromatic heterocyclic rings that may be included in Ax include a 1H-isoindole-1,3(2H)-dione ring, a 1-benzofuran ring, a 2-benzofuran ring, an acridine ring, an isoquinoline ring, an imidazole ring, an indole ring, an oxadiazole ring, an oxazole ring, an oxazolopyrazine ring, an oxazolopyridine ring, an oxazolopyridazyl ring, an oxazolopyrimidine ring, a quinazoline ring, a quinoxaline ring, a quinoline ring, a cinnoline ring, a thiadiazole ring, a thiazole ring, a thiazolopyrazine ring, a thiazolopyridine ring, a thiazolopyridazine ring, a thiazolopyrimidine ring, a thiophene ring, a triazine ring, a triazole ring, a naphthyridine ring, a pyrazine ring, a pyrazole ring, a pyranone ring, a pyran ring, a pyridine ring, a pyridazine ring, a pyrimidine ring, a pyrrole ring, a phenanthridine ring, a phthalazine ring, a furan ring, a benzo[c]thiophene ring, a benzisoxazole ring, a benzisothiazole ring, a benzimidazole ring, a benzoxadiazole ring, a benzoxazole ring, a benzothiadiazole ring, a benzothiazole ring, a benzothiophene ring, a benzotriazine ring, a benzotriazole ring, a benzopyrazole ring, a benzopyranone ring, a dihydropyran ring, a tetrahydropyran ring, a dihydrofuran ring, and a tetrahydrofuran ring.

Of these aromatic heterocyclic rings, a monocyclic aromatic heterocyclic ring such as a furan ring, a pyran ring, a thiophene ring, an oxazole ring, an oxadiazole ring, a thiazole ring, or a thiadiazole ring, or a fused ring aromatic heterocyclic ring such as a benzothiazole ring, a benzoxazole ring, a quinoline ring, a 1-benzofuran ring, a 2-benzofuran ring, a benzothiophene ring, a 1H-isoindole-1,3(2H)-dione ring, a benzo[c]thiophene ring, a thiazolopyridine ring, a thiazolopyrazine ring, a benzisoxazole ring, a benzoxadiazole ring, or a benzothiadiazole ring is preferable.

The aromatic ring included in Ax is optionally substituted. Examples of possible substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-O-C(=O)-R^b$; $-C(=O)-O-R^b$; and $-SO_2R^a$. $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18. $R^a$ has the same meaning as previously described. Of these examples, halogen atoms, a cyano group, alkyl groups having a carbon number of 1 to 6, and alkoxy groups having a carbon number of 1 to 6 are preferable as substituents of the aromatic ring included in Ax.

Note that Ax may have a plurality of substituents selected from the substituents listed above. In a case in which Ax has a plurality of substituents, these substituents may be the same or different.

Examples of the alkyl group having a carbon number of 1 to 20 in the optionally substituted alkyl group having a carbon number of 1 to 20 of $R^b$ include a methyl group, an ethyl group, an n-propyl group, an isopropyl group, an n-butyl group, an isobutyl group, a 1-methylpentyl group, a 1-ethylpentyl group, a sec-butyl group, a t-butyl group, an n-pentyl group, an isopentyl group, a neopentyl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an n-octadecyl group, an n-nonadecyl group, and an n-icosyl group. The carbon number of the optionally substituted alkyl group having a carbon number of 1 to 20 is preferably 1 to 12, and more preferably 4 to 10.

Examples of the alkenyl group having a carbon number of 2 to 20 in the optionally substituted alkenyl group having a carbon number of 2 to 20 of $R^b$ include a vinyl group, a propenyl group, an isopropenyl group, a butenyl group, an isobutenyl group, a pentenyl group, a hexenyl group, a heptenyl group, an octenyl group, a nonenyl group, a decenyl group, an undecenyl group, a dodecenyl group, a tridecenyl group, a tetradecenyl group, a pentadecenyl group, a hexadecenyl group, a heptadecenyl group, an octadecenyl group, a nonadecenyl group, and an icosenyl group.

The carbon number of the optionally substituted alkenyl group having a carbon number of 2 to 20 is preferably 2 to 12.

Examples of possible substituents of the alkyl group having a carbon number of 1 to 20 or alkenyl group having a carbon number of 2 to 20 of $R^b$ include halogen atoms such as a fluorine atom and a chlorine atom; a cyano group; N,N-dialkylamino groups having a carbon number of 2 to 12 such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, a thiophenyl group, and a benzothiazole-2-ylthio group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; a benzofuryl group; a benzopyranyl group; a benzodioxolyl group; and a benzodioxanyl group. Of these examples, halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; and fluoroalkyl groups having a carbon number of 1 to 12 in which one or more hydrogen atoms are replaced by fluorine atoms such as a trifluoromethyl group, a pentafluoroethyl group, and $-CH_2CF_3$ are preferable as substituents of the alkyl group having a carbon number of 1 to 20 or alkenyl group having a carbon number of 2 to 20 of $R^b$.

The alkyl group having a carbon number of 1 to 20 or alkenyl group having a carbon number of 2 to 20 of $R^b$ may have a plurality of substituents selected from the substituents listed above. In a case in which the alkyl group having a carbon number of 1 to 20 or alkenyl group having a carbon number of 2 to 20 of $R^b$ has a plurality of substituents, these substituents may be the same or different.

Examples of the cycloalkyl group having a carbon number of 3 to 12 in the optionally substituted cycloalkyl group having a carbon number of 3 to 12 of $R^b$ include a cyclopropyl group, a cyclobutyl group, a cyclopentyl group, a cyclohexyl group, and a cyclooctyl group. Of these examples, a cyclopentyl group or a cyclohexyl group is preferable.

Examples of possible substituents of the cycloalkyl group having a carbon number of 3 to 12 of $R^b$ include halogen atoms such as a fluorine atom and a chlorine atom; a cyano group; N,N-dialkylamino groups having a carbon number of 2 to 12 such as a dimethylamino group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon cyclic groups having a carbon number of 6 to 20 such as a phenyl group and a naphthyl group. Of these examples, halogen atoms such as a fluorine atom and a chlorine atom; a cyano group; alkyl groups having a carbon number of 1 to 6 such as a methyl group, an ethyl group, and a propyl group; alkoxy groups having a carbon number of 1 to 6 such as a methoxy group, an ethoxy group, and an isopropoxy group; a nitro group; and aromatic hydrocarbon cyclic groups having a carbon number of 6 to 20 such as a phenyl group and a naphthyl group are preferable as substituents of the cycloalkyl group having a carbon number of 3 to 12 of $R^b$.

The cycloalkyl group having a carbon number of 3 to 12 of $R^b$ may have a plurality of substituents. In a case in which the cycloalkyl group having a carbon number of 3 to 12 of $R^b$ has a plurality of substituents, these substituents may be the same or different.

The aromatic hydrocarbon cyclic group having a carbon number of 5 to 18 in the optionally substituted aromatic hydrocarbon cyclic group having a carbon number of 5 to 18 of $R^b$ is preferably an aromatic hydrocarbon cyclic group having a carbon number of 5 to 12, specific examples of which include a phenyl group, a 1-naphthyl group, and a 2-naphthyl group. Of these examples, a phenyl group is preferable.

Examples of possible substituents of the optionally substituted aromatic hydrocarbon cyclic group having a carbon number of 5 to 18 include halogen atoms such as a fluorine atom and a chlorine atom; a cyano group; N,N-dialkylamino groups having a carbon number of 2 to 12 such as a dimethylamino group; alkoxy groups having a carbon

number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; $-OCF_3$; a benzofuryl group; a benzopyranyl group; a benzodioxolyl group; and a benzodioxanyl group. Of these examples, one or more substituents selected from halogen atoms such as a fluorine atom and a chlorine atom; a cyano group; alkoxy groups having a carbon number of 1 to 20 such as a methoxy group, an ethoxy group, an isopropoxy group, and a butoxy group; a nitro group; aromatic hydrocarbon cyclic groups having a carbon number of 6 to 20 such as a phenyl group and a naphthyl group; aromatic heterocyclic groups having a carbon number of 2 to 20 such as a furanyl group and a thiophenyl group; cycloalkyl groups having a carbon number of 3 to 8 such as a cyclopropyl group, a cyclopentyl group, and a cyclohexyl group; fluoroalkyl groups having a carbon number of 1 to 12 in which one or more hydrogen atoms are replaced by fluorine atoms such as a trifluoromethyl group, a pentafluoroethyl group, and $-CH_2CF_3$; and $-OCF_3$ are preferable as substituents of the aromatic hydrocarbon cyclic group having a carbon number of 5 to 18.

The aromatic hydrocarbon cyclic group having a carbon number of 5 to 18 may have a plurality of substituents. In a case in which the aromatic hydrocarbon cyclic group having a carbon number of 5 to 18 has a plurality of substituents, these substituents may be the same or different.

The aromatic ring included in Ax may have a plurality of substituents that are the same or different, and two substituents that are adjacent to one another may be bonded to form a ring. The ring that is formed may be a monocycle or a fused polycycle, and may be an unsaturated ring or a saturated ring.

Note that the "carbon number" of the organic group of Ax that includes at least one aromatic ring selected from the group consisting of an aromatic hydrocarbon ring having a carbon number of 6 to 30 and an aromatic heterocyclic ring having a carbon number of 2 to 30 is the carbon number of the aromatic hydrocarbon ring and/or aromatic heterocyclic ring itself and does not include carbon atoms of substituents.

Examples of the organic group of Ax that includes at least one aromatic ring selected from the group consisting of an aromatic hydrocarbon ring having a carbon number of 6 to 30 and an aromatic heterocyclic ring having a carbon number of 2 to 30 include the following groups 1) to 5).

1) A hydrocarbon cyclic group having a carbon number of 6 to 40 and including at least one aromatic hydrocarbon ring having a carbon number of 6 to 30
2) A heterocyclic group having a carbon number of 2 to 40 and including at least one aromatic ring selected from the group consisting of an aromatic hydrocarbon ring having a carbon number of 6 to 30 and an aromatic heterocyclic ring having a carbon number of 2 to 30
3) An alkyl group having a carbon number of 1 to 12 that is substituted with at least one of an aromatic hydrocarbon cyclic group having a carbon number of 6 to 30 and an aromatic heterocyclic group having a carbon number of 2 to 30
4) An alkenyl group having a carbon number of 2 to 12 that is substituted with at least one of an aromatic hydrocarbon cyclic group having a carbon number of 6 to 30 and an aromatic heterocyclic group having a carbon number of 2 to 30
5) An alkynyl group having a carbon number of 2 to 12 that is substituted with at least one of an aromatic hydrocarbon cyclic group having a carbon number of 6 to 30 and an aromatic heterocyclic group having a carbon number of 2 to 30

Specific examples of the aromatic hydrocarbon ring in the "hydrocarbon cyclic group having a carbon number of 6 to 40 and including at least one aromatic hydrocarbon ring having a carbon number of 6 to 30" mentioned above in 1) include the same specific examples as listed for aromatic hydrocarbon rings that may be included in Ax. Examples of the hydrocarbon cyclic group mentioned above in 1) include an aromatic hydrocarbon cyclic group having a carbon number of 6 to 30 (for example, a phenyl group, a naphthyl group, an anthracenyl group, a phenanthrenyl group, a pyrenyl group, or a fluorenyl group), an indanyl group, a 1,2,3,4-tetrahydronaphthyl group, and a 1,4-dihydronaphthyl group.

Specific examples of the aromatic hydrocarbon ring and the aromatic heterocyclic ring in the "heterocyclic group having a carbon number of 2 to 40 and including at least one aromatic ring selected from the group consisting of an aromatic hydrocarbon ring having a carbon number of 6 to 30 and an aromatic heterocyclic ring having a carbon number of 2 to 30" mentioned above in 2) include the same specific examples as listed for aromatic hydrocarbon rings and aromatic heterocyclic rings that may be included in Ax. Examples of the heterocyclic group mentioned above in 2) include

an aromatic heterocyclic group having a carbon number of 2 to 30 (for example, a phthalimide group, a 1-benzofuranyl group, a 2-benzofuranyl group, an acridinyl group, an isoquinolinyl group, an imidazolyl group, an indolinyl group, a furazanyl group, an oxazolyl group, an oxazolopyrazinyl group, an oxazolopyridinyl group, an oxazolopyridazinyl group, an oxazolopyrimidinyl group, a quinazolinyl group, a quinoxalinyl group, a quinolyl group, a cinnolinyl group, a thiadiazolyl group, a thiazolyl group, a thiazolopyrazinyl group, a thiazolopyridinyl group, a thiazolopyridazinyl group, a thiazolopyrimidinyl group, a thienyl group, a triazinyl group, a triazolyl group, a naphthyridinyl group, a pyrazinyl group, a pyrazolyl group, a pyranonyl group, a pyranyl group, a pyridyl group, a pyridazinyl group, a pyrimidinyl group, a pyrrolyl group, a phenanthridinyl group, a phthalazinyl group, a furanyl group, a benzo[c]thienyl group, a benzisoxazolyl group, a benzisothiazolyl group, a benzimidazolyl group, a benzoxazolyl group, a benzothiadiazolyl group, a benzothiazolyl group, a benzothiophenyl group, a benzotriazinyl group, a benzotriazolyl group, a benzopyrazolyl group, a benzopyranonyl group, a dihydropyranyl group, a tetrahydropyranyl group, a dihydrofuranyl group, or a tetrahydrofuranyl group), a 2,3-dihydroindolyl group, a 9,10-dihydroacridinyl group, and a 1,2,3,4-tetrahydroquinolyl group.

Specific examples of the alkyl group having a carbon number of 1 to 12 in the "alkyl group having a carbon number of 1 to 12 that is substituted with at least one of an aromatic hydrocarbon cyclic group having a carbon number of 6 to 30 and an aromatic heterocyclic group having a carbon number of 2 to 30" mentioned above in 3) include a methyl group, an ethyl group, a propyl group, and an isopropyl group. Specific examples of the aromatic hydrocarbon cyclic group having a carbon number of 6 to 30 and the aromatic heterocyclic group having a carbon number of 2 to 30 mentioned above in 3) include the same specific examples as listed for the aromatic hydrocarbon cyclic group having a carbon number of 6 to 30 and the aromatic heterocyclic group having a carbon number of 2 to 30 mentioned above in 1) and 2).

Specific examples of the alkenyl group having a carbon number of 2 to 12 in the "alkenyl group having a carbon number of 2 to 12 that is substituted with at least one of an aromatic hydrocarbon cyclic group having a carbon number of 6 to 30 and an aromatic heterocyclic group having a carbon number of 2 to 30" mentioned above in 4) include a vinyl group and an allyl group. Specific examples of the aromatic hydrocarbon cyclic group having a carbon number of 6 to 30 and the aromatic heterocyclic group having a carbon number of 2 to 30 mentioned above in 4) include the same specific examples as listed for the aromatic hydrocarbon cyclic group having a carbon number of 6 to 30 and the aromatic heterocyclic group having a carbon number of 2 to 30 mentioned above in 1) and 2).

Specific examples of the alkynyl group having a carbon number of 2 to 12 in the "alkynyl group having a carbon number of 2 to 12 that is substituted with at least one of an aromatic hydrocarbon cyclic group having a carbon number of 6 to 30 and an aromatic heterocyclic group having a carbon number of 2 to 30" mentioned above in 5) include an ethynyl group and a propynyl group. Specific examples of the aromatic hydrocarbon cyclic group having a carbon number of 6 to 30 and the aromatic heterocyclic group having a carbon number of 2 to 30 mentioned above in 5) include the same specific examples as listed for the aromatic hydrocarbon cyclic group having a carbon number of 6 to 30 and the aromatic heterocyclic group having a carbon number of 2 to 30 mentioned above in 1) and 2).

The organic groups listed above in 1) to 5) may have one or a plurality of substituents. In a case in which the organic group has a plurality of substituents, these substituents may be the same or different.

Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 2 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-O-C(=O)-R^b$; $-C(=O)-O-R^b$; and $-SO_2R^a$. $R^b$ and $R^a$ have the same meaning here as previously described and preferable examples thereof are also the same as previously described.

Of these examples, one or more substituents selected from halogen atoms, a cyano group, alkyl groups having a carbon number of 1 to 6, and alkoxy groups having a carbon number of 1 to 6 are preferable as substituents included in the organic groups listed above in 1) to 5).

Specific examples that are preferable as the organic group of Ax that includes at least one aromatic ring selected from the group consisting of an aromatic hydrocarbon ring having a carbon number of 6 to 30 and an aromatic heterocyclic ring having a carbon number of 2 to 30 are shown below. However, the following examples are not intended to be limiting. Note that "-" in the following formulae indicates atomic bonding with a N atom that extends from any position in a ring (i.e., a N atom that is bonded to Ax in formula (I)).

1) Specific examples of hydrocarbon cyclic groups having a carbon number of 6 to 40 and including at least one aromatic hydrocarbon ring having a carbon number of 6 to 30 include structures represented by the following formulae (1-1) to (1-21). Aromatic hydrocarbon cyclic groups having a carbon number of 6 to 30 that are represented by formulae (1-9) to (1-21) and the like are preferable.

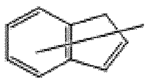 (1-1) 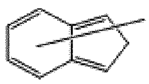 (1-2) 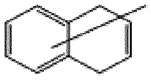 (1-3) 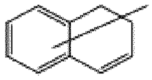 (1-4) 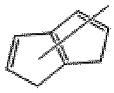 (1-5) 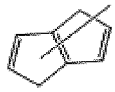 (1-6)

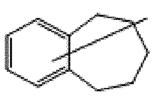 (1-7) 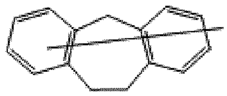 (1-8)

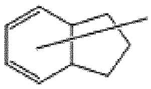 (1-9) 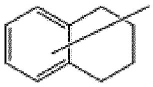 (1-10) 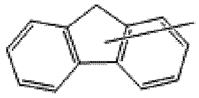 (1-11) 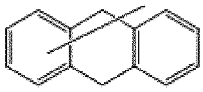 (1-12)

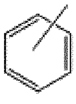 (1-13) 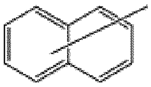 (1-14) 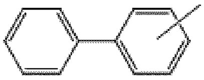 (1-15) 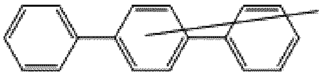 (1-16) 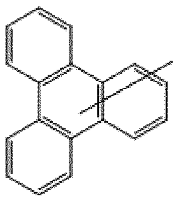 (1-21)

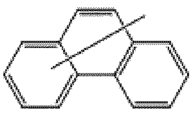 (1-17) 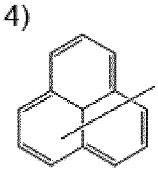 (1-18) 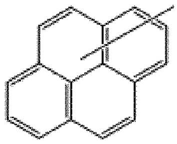 (1-19) 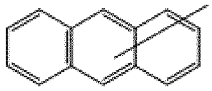 (1-20)

2) Specific examples of heterocyclic groups having a carbon number of 2 to 40 and including at least one aromatic ring selected from the group consisting of an aromatic hydrocarbon ring having a carbon number of 6 to 30 and an aromatic heterocyclic ring having a carbon number of 2 to 30 include structures represented by the following formulae (2-1) to (2-51). Aromatic heterocyclic groups having a carbon number of 2 to 30 that are represented by formulae (2-12) to (2-51) and the like are preferable.

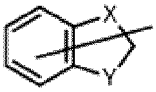 (2-1) 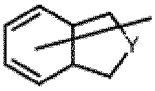 (2-2) 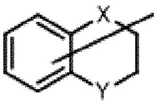 (2-3) 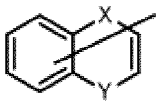 (2-4) 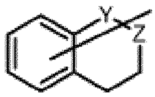 (2-5) 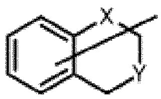 (2-6)

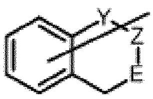 (2-7) 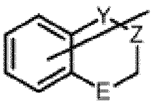 (2-8) 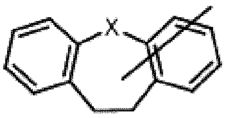 (2-9) 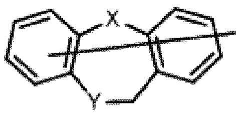 (2-10) 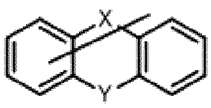 (2-11)

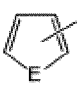 (2-12) 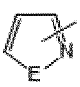 (2-13) 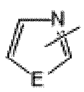 (2-14) 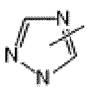 (2-15) 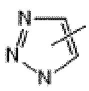 (2-16) 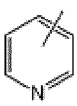 (2-17) 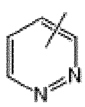 (2-18) 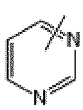 (2-19)

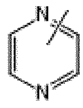
(2-20)
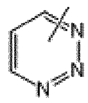
(2-21)
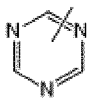
(2-22)
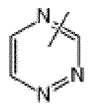
(2-23)
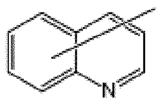
(2-24)
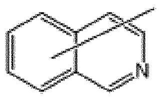
(2-25)
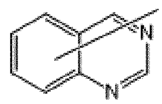
(2-26)

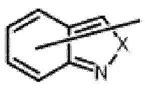
(2-27)
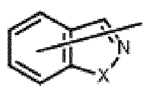
(2-28)
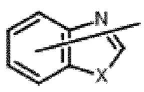
(2-29)
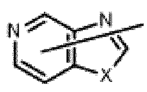
(2-30)
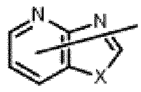
(2-31)
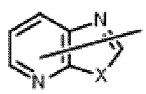
(2-32)
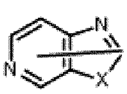
(2-33)

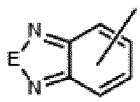
(2-34)
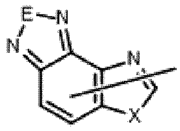
(2-35)
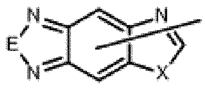
(2-36)
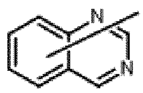
(2-37)
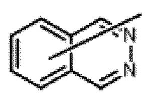
(2-38)
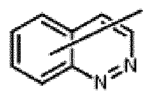
(2-39)

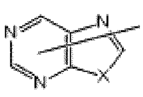
(2-40)
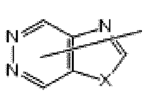
(2-41)
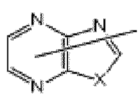
(2-42)
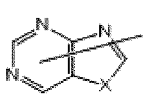
(2-43)
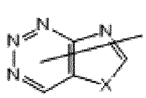
(2-44)
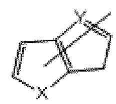
(2-45)
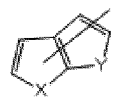
(2-46)

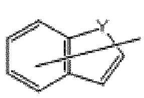
(2-47)
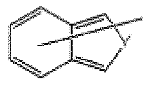
(2-48)
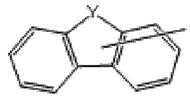
(2-49)
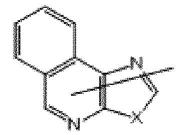
(2-50)
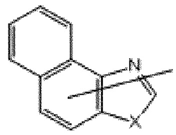
(2-51)

[In each formula: X represents $-CH_2-$, $-NR^c-$, an oxygen atom, a sulfur atom, -SO-, or $-SO_2-$;
Y and Z each represent, independently of one another, $-NR^c-$, an oxygen atom, a sulfur atom, -SO-, or $-SO_2-$; and
E represents $-NR^c-$, an oxygen atom, or a sulfur atom.

Moreover, $R^c$ represents a hydrogen atom or an alkyl group having a carbon number of 1 to 6 such as a methyl group, an ethyl group, or a propyl group. (However, in each formula, oxygen atoms, sulfur atoms, -SO-, and $-SO_2-$ are not located adjacently to one another.)]

3) Specific examples of alkyl groups having a carbon number of 1 to 12 that are substituted with at least one of an aromatic hydrocarbon cyclic group having a carbon number of 6 to 30 and an aromatic heterocyclic group having a carbon number of 2 to 30 include structures represented by the following formulae (3-1) to (3-8).

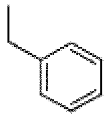
(3-1)
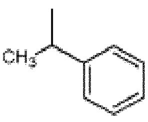
(3-2)
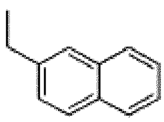
(3-3)
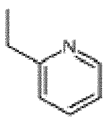
(3-4)
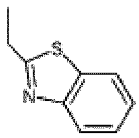
(3-5)

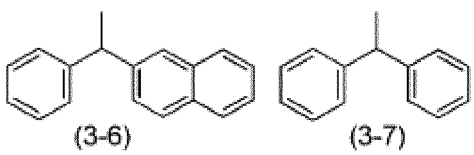
(3-6) (3-7)
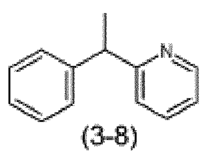
(3-8)

4) Specific examples of alkenyl groups having a carbon number of 2 to 12 that are substituted with at least one of an aromatic hydrocarbon cyclic group having a carbon number of 6 to 30 and an aromatic heterocyclic group having a carbon number of 2 to 30 include structures represented by the following formulae (4-1) to (4-5).

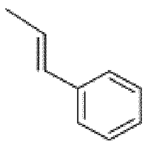
(4-1)

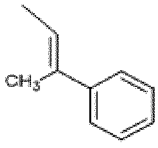
(4-2)

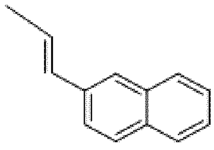
(4-3)

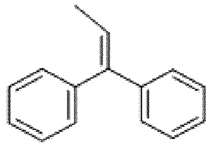
(4-4)

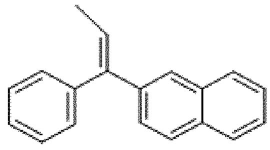
(4-5)

5) Specific examples of alkynyl groups having a carbon number of 1 to 12 that are substituted with at least one selected from the group consisting of an aromatic hydrocarbon ring and an aromatic heterocyclic ring include structures represented by the following formulae (5-1) and (5-2).

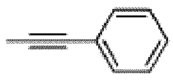
(5-1)

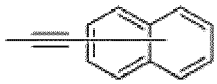
(5-2)

Note that the rings in these preferable specific examples of Ax may have one or a plurality of substituents. In a case in which a ring has a plurality of substituents, these substituents may be the same or different. Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group; N,N-dialkylamino groups having a carbon number of 1 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-OCF_3$; $-C(=O)-R^b$; $-O-C(=O)-R^b$; $-C(=O)-O-R^b$; and $-SO_2R^a$.

$R^b$ and $R^a$ have the same meaning here as previously described and preferable examples thereof are also the same as previously described. Of these examples, halogen atoms, a cyano group, alkyl groups having a carbon number of 1 to 6, and alkoxy groups having a carbon number of 1 to 6 are preferable as substituents of a ring included in Ax.

From among the examples of Ax described above, Ax is preferably an aromatic hydrocarbon cyclic group having a carbon number of 6 to 30, an aromatic heterocyclic group having a carbon number of 2 to 30, or a group indicated by the previously shown formula (1-9).

Ax is more preferably an aromatic hydrocarbon cyclic group having a carbon number of 6 to 20 or an aromatic heterocyclic group having a carbon number of 4 to 20, and is even more preferably a group indicated by any one of the previously shown formulae (1-14), (1-20), (2-27) to (2-33), (2-35) to (2-43), (2-50), and (2-51).

Note that the rings may have one or a plurality of substituents as previously described. In a case in which a ring has a plurality of substituents, these substituents may be the same or different. Examples of these substituents include halogen atoms such as a fluorine atom and a chlorine atom; a cyano group; alkyl groups having a carbon number of 1 to 6 such as a methyl group, an ethyl group, and a propyl group; alkenyl groups having a carbon number of 2 to 6 such as a vinyl group and an allyl group; haloalkyl groups having a carbon number of 1 to 6 such as a trifluoromethyl group and a pentafluoroethyl group; N,N-dialkylamino groups having a carbon number of 1 to 12 such as a dimethylamino group; alkoxy groups having a carbon number of 1 to 6 such as a methoxy group, an ethoxy group, and an isopropoxy group; a nitro group; aromatic hydrocarbon cyclic groups having a carbon number of 6 to 20 such as a phenyl group and a naphthyl group; $-C(=O)-R^b$; $-O-C(=O)-R^b$; $-C(=O)-O-R^b$; and $-SO_2R^a$.

$R^b$ and $R^a$ have the same meaning here as previously described and preferable examples thereof are also the same as previously described.

Of these examples, halogen atoms, a cyano group, alkyl groups having a carbon number of 1 to 6, and alkoxy groups having a carbon number of 1 to 6 are preferable as substituents of the rings.

A group represented by the following formula (V) is even more preferable as Ax.

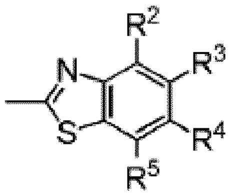

(V)

In formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, -O-C(=O)-$R^b$, or -C(=O)-O-$R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18. Of these examples, a case in which $R^2$ to $R^5$ are all hydrogen atoms and a case in which at least one of $R^2$ to $R^5$ is an optionally substituted alkoxy group having a carbon number of 1 to 6 and the rest of $R^2$ to $R^5$ are hydrogen atoms are preferable.

Moreover, C-$R^2$ to C-$R^5$ may be the same or different, and one or more of ring constituents C-$R^2$ to C-$R^5$ may be replaced by a nitrogen atom.

The following shows specific examples of groups resulting from one or more of C-$R^2$ to C-$R^5$ in the group represented by formula (V) being replaced by a nitrogen atom. However, examples of groups resulting from one or more of C-$R^2$ to C-$R^5$ being replaced by a nitrogen atom are not limited to these examples.

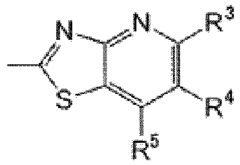
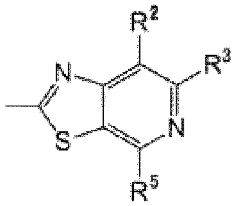
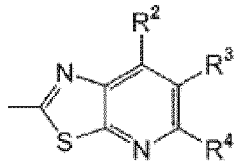
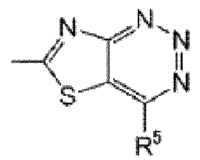

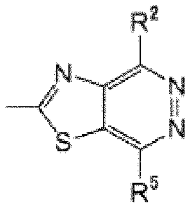
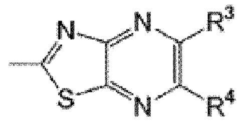
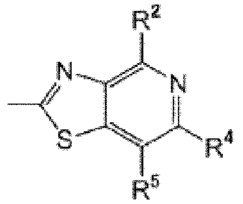
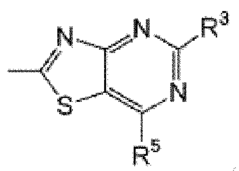

[In each formula, $R^2$ to $R^5$ have the same meaning as previously described and preferable examples thereof are also the same as previously described.]

Examples of the optionally substituted organic group having a carbon number of 1 to 30 of Ay in the groups represented by the formulae (II-1) to (II-6) include, but are not specifically limited to, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, $-SO_2R^a$, -O-C(=O)-$R^b$, -C(=O)-O-$R^b$, -C(=O)-$R^b$, -CS-NH-$R^b$, -NH-C(=O)-O-$R^b$, -O-C(=O)-NH-$R^b$, an optionally substituted aromatic hydrocarbon cyclic group having a carbon number of 6 to 30, and an optionally substituted aromatic heterocyclic group having a carbon number of 2 to 30.

$R^a$ and $R^b$ have the same meaning here as previously described and preferable examples thereof are also the same as previously described.

Examples of the alkyl group having a carbon number of 1 to 20 in the optionally substituted alkyl group having a carbon number of 1 to 20 of Ay, the alkenyl group having a carbon number of 2 to 20 in the optionally substituted alkenyl group having a carbon number of 2 to 20 of Ay, and the cycloalkyl group having a carbon number of 3 to 12 in the optionally substituted cycloalkyl group having a carbon number of 3 to 12 of Ay include the same specific examples as listed for the alkyl group having a carbon number of 1 to 20 in the optionally substituted alkyl group having a carbon number of 1 to 20 of $R^b$, the alkenyl group having a carbon number of 2 to 20 in the optionally substituted alkenyl group having a carbon number of 2 to 20 of $R^b$, and the cycloalkyl group having a carbon number of 3 to 12 in the optionally substituted cycloalkyl group having a carbon number of 3 to 12 of $R^b$. Moreover, the carbon number of the optionally substituted alkyl group having a carbon number of 1 to 20 is preferably 1 to 10, the carbon number of the optionally

substituted alkenyl group having a carbon number of 2 to 20 is preferably 2 to 10, and the carbon number of the optionally substituted cycloalkyl group having a carbon number of 3 to 12 is preferably 3 to 10.

Examples of the alkynyl group having a carbon number of 2 to 20 in the optionally substituted alkynyl group having a carbon number of 2 to 20 of Ay include an ethynyl group, a propynyl group, a 2-propynyl group (propargyl group), a butynyl group, a 2-butynyl group, a 3-butynyl group, a pentynyl group, a 2-pentynyl group, a hexynyl group, a 5-hexynyl group, a heptynyl group, an octynyl group, a 2-octynyl group, a nonanyl group, a decanyl group, and a 7-decanyl group.

Examples of possible substituents of the optionally substituted alkyl group having a carbon number of 1 to 20, the optionally substituted alkenyl group having a carbon number of 2 to 20, the optionally substituted cycloalkyl group having a carbon number of 3 to 12, or the alkynyl group having a carbon number of 2 to 20 of Ay include halogen atoms such as a fluorine atom and a chlorine atom; a cyano group; N,N-dialkylamino groups having a carbon number of 2 to 12 such as a dimethylamino group; alkoxy groups having a carbon number of 1 to 20 such as a methoxy group, an ethoxy group, an isopropoxy group, and a butoxy group; alkoxy groups having a carbon number of 1 to 12 that are substituted with an alkoxy group having a carbon number of 1 to 12 such as a methoxymethoxy group and a methoxyethoxy group; a nitro group; aromatic hydrocarbon cyclic groups having a carbon number of 6 to 20 such as a phenyl group and a naphthyl group; aromatic heterocyclic groups having a carbon number of 2 to 20 such as a triazolyl group, a pyrrolyl group, a furanyl group, and a thiophenyl group; cycloalkyl groups having a carbon number of 3 to 8 such as a cyclopropyl group, a cyclopentyl group, and a cyclohexyl group; cycloalkyloxy groups having a carbon number of 3 to 8 such as a cyclopentyloxy group and a cyclohexyloxy group; cyclic ether groups having a carbon number of 2 to 12 such as a tetrahydrofuranyl group, a tetrahydropyranyl group, a dioxolanyl group, and a dioxanyl group; aryloxy groups having a carbon number of 6 to 14 such as a phenoxy group and a naphthoxy group; fluoroalkyl groups having a carbon number of 1 to 12 in which one or more hydrogen atoms are replaced by fluorine atoms such as a trifluoromethyl group, a pentafluoroethyl group, and $-CH_2CF_3$; a benzofuryl group; a benzopyranyl group; a benzodioxolyl group; a benzodioxanyl group; $-O-C(=O)-R^b$; $-C(=O)-R^b$; $-C(=O)-O-R^b$; $-SO_2R^a$; $-SR^b$; alkoxy groups having a carbon number of 1 to 12 that are substituted with $-SR^b$; and a hydroxy group. $R^a$ and $R^b$ have the same meaning here as previously described and preferable examples thereof are also the same as previously described.

The alkyl group having a carbon number of 1 to 20, the alkenyl group having a carbon number of 2 to 20, the cycloalkyl group having a carbon number of 3 to 12, or the alkynyl group having a carbon number of 2 to 20 of Ay may have a plurality of the substituents listed above, and in such a case, these substituents may be the same or different.

Examples of the aromatic hydrocarbon cyclic group having a carbon number of 6 to 30 or aromatic heterocyclic group having a carbon number of 2 to 30 of Ay and substituents thereof include the same examples as listed for the aromatic hydrocarbon cyclic group or aromatic heterocyclic group of Ax and substituents thereof. The aromatic hydrocarbon cyclic group having a carbon number of 6 to 30 or the aromatic heterocyclic group having a carbon number of 2 to 30 of Ay may have a plurality of substituents selected from those listed above. In a case in which the aromatic hydrocarbon cyclic group or aromatic heterocyclic group of Ay has a plurality of substituents, these substituents may be the same or different. The carbon number of the aromatic hydrocarbon cyclic group of Ay is preferably 6 to 20, more preferably 6 to 18, and even more preferably 6 to 12. Moreover, the carbon number of the aromatic heterocyclic group of Ay is preferably 2 to 20, and more preferably 2 to 18.

Of the examples listed above, Ay is preferably a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 18, or an optionally substituted aromatic heterocyclic group having a carbon number of 2 to 18. Moreover, Ay is more preferably a hydrogen atom, an optionally substituted alkyl group having a carbon number of 1 to 18, an optionally substituted alkenyl group having a carbon number of 2 to 18, an optionally substituted alkynyl group having a carbon number of 2 to 18, an optionally substituted cycloalkyl group having a carbon number of 3 to 10, an optionally substituted aromatic hydrocarbon cyclic group having a carbon number of 6 to 12, or an optionally substituted aromatic heterocyclic group having a carbon number of 2 to 18. Furthermore, Ay is particularly preferably an optionally substituted alkyl group having a carbon number of 1 to 18, and is especially preferably an optionally substituted alkyl group having a carbon number of 2 to 12.

In the previously mentioned formula (I), $Y^0$, $Z^1$, and $Z^2$ each represent, independently of one another, a single bond, -O-, $-O-CH_2-$, $-CH_2-O-$, $-O-CH_2-CH_2-$, $-CH_2-CH_2-O-$, -C(=O)-O-, -O-C(=O)-, -C(=O)-S-, -S-C(=O)-, $-NR^{10}-C(=O)-$, $-C(=O)-NR^{10}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, or $-C\equiv C-$. $R^{10}$ represents a hydrogen atom or an alkyl group having a carbon number of 1 to 6.

Of these examples, $Z^1$ is preferably -C(=O)-O- and $Z^2$ is preferably -O-C(=O)-.

G in the previously mentioned formula (I) represents an optionally substituted alkylene group, an optionally

substituted alicyclic group, or an optionally substituted aromatic group, and is preferably an optionally substituted alkylene group having a carbon number of 1 to 20, an optionally substituted alicyclic group having a carbon number of 5 to 20, or an optionally substituted aromatic group having a carbon number of 2 to 20.

Examples of possible substituents of the alkylene group, alicyclic group, or aromatic group constituting G include halogen atoms such as a fluorine atom, a chlorine atom, and a bromine atom; alkyl groups having a carbon number of 1 to 6 such as a methyl group and an ethyl group; alkoxy groups having a carbon number of 1 to 5 such as a methoxy group and an isopropoxy group; a nitro group; and a cyano group. The alicyclic group, alicyclic group having a carbon number of 5 to 20, aromatic group, or aromatic group having a carbon number of 2 to 20 may have one or more substituents selected from the substituents described above. In a case in which the group has a plurality of substituents, these substituents may be the same or different.

Examples of the alkylene group of G include a methylene group, an ethylene group, an n-propylene group, an n-butylene group, and an n-hexylene group.

Specific examples of the alicyclic group of G include cycloalkanediyl groups having a carbon number of 5 to 20 such as cyclopentane-1,3-diyl, cyclohexane-1,4-diyl, cycloheptane-1,4-diyl, and cyclooctane-1,5-diyl; and bicycloalkanediyl groups having a carbon number of 5 to 20 such as decahydronaphthalene-1,5-diyl and decahydronaphthalene-2,6-diyl. Of these examples, the alicyclic group of G is preferably an optionally substituted cycloalkanediyl group having a carbon number of 5 to 20, more preferably a cyclohexanediyl group, and particularly preferably a cyclohexane-1,4-diyl group represented by the following formula (a). The alicyclic group of G may be a trans isomer represented by formula (a1), a cis isomer represented by formula (a2), or a mixture of the trans isomer and the cis isomer, but is preferably the trans isomer represented by formula (a1).

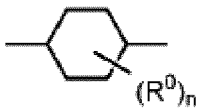
(a)

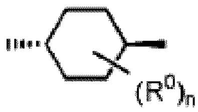
(a1)

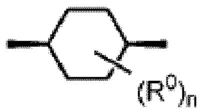
(a2)

(In the formulae, $R^0$ and n have the same meaning as previously described.)

Specific examples of the aromatic group of G include aromatic hydrocarbon cyclic groups having a carbon number of 6 to 20 such as a 1,4-phenylene group, a 1,4-naphthylene group, a 1,5-naphthylene group, a 2,6-naphthylene group, and a 4,4'-biphenylene group; and aromatic heterocyclic groups having a carbon number of 2 to 20 such as furan-2,5-diyl, thiophene-2,5-diyl, pyridine-2,5-diyl, and pyrazine-2,5-diyl. Of these examples, the aromatic group of G is preferably an aromatic hydrocarbon cyclic group having a carbon number of 6 to 20, more preferably a phenylene group, and particularly preferably a 1,4-phenylene group represented by the following formula (b).

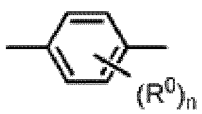
(b)

(In the formula, $R^0$ and n have the same meaning as previously described and preferable examples thereof are also the same as previously described. Note that in a case in which more than one $R^0$ is included, each $R^0$ may be the same or different.)

In the previously mentioned formula (I), $L^1$ and $L^2$ are each, independently of one another, an organic group that is either an alkylene group having a carbon number of 1 to 20 or a group in which at least one methylene group ($-CH_2-$) of an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom. Note that in the "group in which at least one methylene group ($-CH_2-$) of an alkylene group having a carbon number of 1 to 20 is replaced by -O- or -C(=O)-", it is preferable that consecutive methylene groups in the alkylene group are not replaced by -O- (i.e., an -O-O- structure is not formed) and that consecutive methylene groups in the alkylene group are not replaced by -C(=O)- (i.e., a -C(=O)-C(=O)- structure is not formed). Moreover, methylene groups ($-CH_2-$) at both ends of $L^1$ and $L^2$ are not replaced by -O- or -C(=O)-.

The organic groups of $L^1$ and $L^2$ are preferably each an alkylene group having a carbon number of 1 to 20 that is optionally substituted with a fluorine atom or a group represented by $-(CH_2)_j-C(=O)-O-(CH_2)_k-$ (j and k in the formula

each represent an integer of 2 to 12, and preferably each represent an integer of 2 to 8) that is optionally substituted with a fluorine atom, are more preferably each an alkylene group having a carbon number of 2 to 12 that is optionally substituted with a fluorine atom, are even more preferably each an unsubstituted alkylene group having a carbon number of 2 to 12, and are particularly preferably each a group represented by $-(CH_2)_l-$ (1 in the formula represents an integer of 2 to 12, and preferably represents an integer of 2 to 8).

In the previously mentioned formula (I), $A^1$, $A^2$, $B^1$, and $B^2$ each represent, independently of one another, an optionally substituted alicyclic group or an optionally substituted aromatic group, and preferably an optionally substituted alicyclic group having a carbon number of 5 to 20 or an optionally substituted aromatic group having a carbon number of 2 to 20. Examples of the optionally substituted alicyclic group and the optionally substituted aromatic group include the same examples as listed for the optionally substituted alicyclic group or optionally substituted aromatic group of G, and these groups may have a plurality of substituents selected from the listed examples. In a case in which the alicyclic group or aromatic group has a plurality of substituents, these substituents may be the same or different.

In terms of the combination of G and $A^2$ in the polymerizable compound (I), it is preferable that G is an optionally substituted aromatic hydrocarbon cyclic group having a carbon number of 6 to 20 or an optionally substituted cycloalkanediyl group having a carbon number of 5 to 20 and $A^2$ is an optionally substituted cycloalkanediyl group having a carbon number of 5 to 20, more preferable that G is an optionally substituted cyclohexanediyl group or an optionally substituted phenylene group and $A^2$ is an optionally substituted cyclohexanediyl group, and even more preferable that G is a group represented by formula (a) or formula (b) and $A^2$ is a group represented by formula (a). Note that it is particularly preferable that the group represented by formula (a) is the group represented by formula (a1).

In the previously mentioned formula (I), $Y^1$ to $Y^4$ each represent, independently of one another, a single bond, -O-, -C(=O)-, -C(=O)-O-, -O-C(=O)-, $-NR^{11}-C(=O)-$, $-C(=O)-NR^{11}-$, -O-C(=O)-O-, $-NR^{11}-C(=O)-O-$, $-O-C(=O)-NR^{11}-$, or $-NR^{11}-C(=O)-NR^{12}-$. $R^{11}$ and $R^{12}$ each represent, independently of one another, a hydrogen atom or an alkyl group having a carbon number of 1 to 6.

Of these examples, $Y^1$ to $Y^4$ are preferably each, independently of one another, -O-, -C(=O)-, -C(=O)-O-, -O-C(=O)-O-, or -O-C(=O)-.

In the previously mentioned formula (I), one of $P^1$ and $P^2$ represents a hydrogen atom or a polymerizable group and the other of $P^1$ and $P^2$ represents a polymerizable group. Examples of the polymerizable group of $P^1$ and $P^2$ include a group represented by $CH_2=CR^1-C(=O)-O-$ ($R^1$ represents a hydrogen atom, a methyl group, or a chlorine atom) such as an acryloyloxy group or a methacryloyloxy group, a vinyl group, a p-stilbene group, an acryloyl group, a methacryloyl group, a carboxyl group, a methylcarbonyl group, a hydroxy group, an amide group, an alkylamino group having a carbon number of 1 to 4, an amino group, an epoxy group, an oxetanyl group, an aldehyde group, an isocyanate group, and a thioisocyanate group. Of these polymerizable groups, a group represented by $CH_2=CR^1-C(=O)-O-$ such as the following formula (IV) is preferable, $CH_2=CH-C(=O)-O-$ (acryloyloxy group) or $CH_2=C(CH_3)-C(=O)-O-$ (methacryloyloxy group) is more preferable, and an acryloyloxy group is even more preferable. In a case in which two $R^1$ groups are present in the polymerizable compound indicated by formula (I), each $R^1$ may be the same or different. Moreover, $P^1$ and $P^2$ may be different, but are preferably the same polymerizable group.

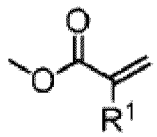
(I V)

[$R^1$ in formula (IV) represents a hydrogen atom, a methyl group, or a chlorine atom.]

In the previously mentioned formula (I), p and q are each, independently of one another, an integer of 0 to 2 (however, cases in which p = 0 and q = 1 and in which p = 1 and q = 2 are excluded). It is preferable that p = 0 or 1 and q = 0 or that p = 1 and q = 1, and more preferable that p = 0 and q = 0 or that p = 1 and q = 1.

Among the groups represented by the previously shown formulae (II-1) to (II-6), a group represented by formula (II-1) or (II-4) is preferable as Ar-D in the previously mentioned formula (I). In other words, the presently disclosed polymerizable compound is preferably a polymerizable compound indicated by the following formula (III-1) or (III-2).

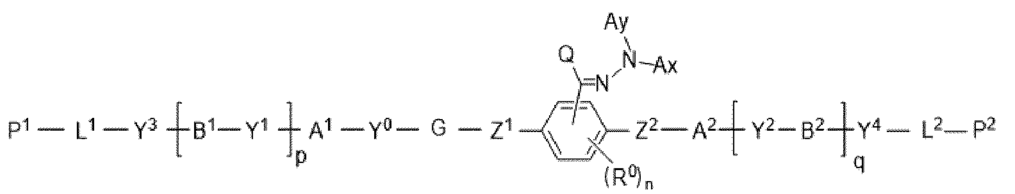
(III-1)

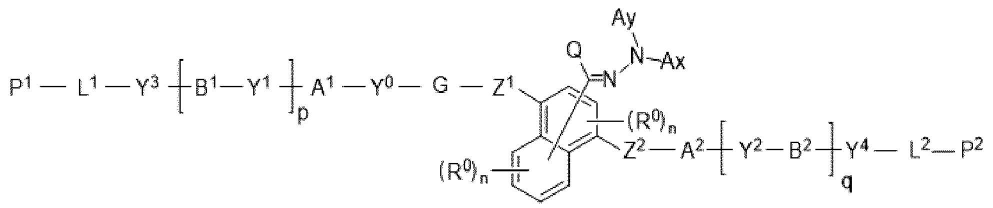

(III-2)

In formulae (III-1) and (III-2), $P^1$, $P^2$, $L^1$, $L^2$, $A^1$, $A^2$, $B^1$, $B^2$, $Y^0$ to $Y^4$, G, $Z^1$, $Z^2$, p, q, $R^0$, n, Ax, Ay, and Q have the same meaning as previously described and preferable examples thereof are also the same as previously described.

A polymerizable compound indicated by formula (III-1) or (III-2) enables production of an optical film or the like having even better wavelength dispersion characteristics at short wavelengths.

Moreover, the presently disclosed polymerizable compound is preferably a polymerizable compound indicated by the following formula (VI-1) or (VI-2).

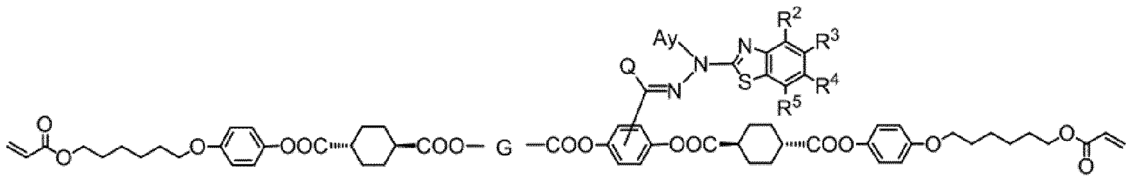

(VI-1)

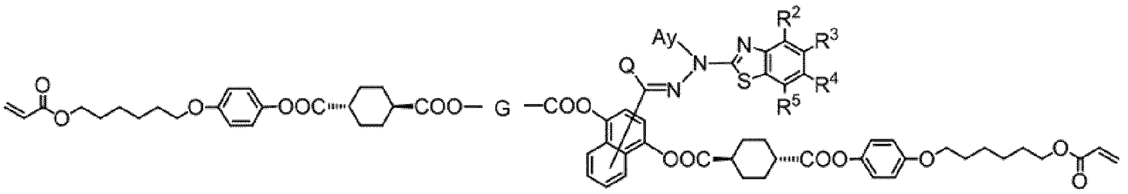

(VI-2)

In formulae (VI-1) and (VI-2), $R^2$ to $R^5$, G, Ay, and Q have the same meaning as previously described and preferable examples thereof are also the same as previously described.

The polymerizable compounds (I), (III-1), (III-2), (VI-1), and (VI-2) set forth above can be synthesized through a combination of known synthetic reactions. Specifically, these compounds can be synthesized with reference to methods described in various documents (for example, March's Advanced Organic Chemistry (Wiley); and Sandler and Karo, "Syntheses of Organic Compounds Classified by Functional Group", joint translation by Naoki INAMOTO (Hirokawa Publishing Company)).

(2) Polymerizable composition

The presently disclosed polymerizable composition contains the polymerizable compound set forth above and a polymerization initiator.

The presently disclosed polymerizable composition is useful as a production raw material for the presently disclosed polymer, optical film, and optically anisotropic body as described further below. Moreover, the presently disclosed polymerizable composition enables favorable production of an optical film or the like having good wavelength dispersion characteristics at short wavelengths.

The polymerization initiator is included from a viewpoint of more efficiently carrying out a polymerization reaction of the polymerizable compound contained in the polymerizable composition.

Examples of polymerization initiators that may be used include radical polymerization initiators, anionic polymerization initiators, and cationic polymerization initiators.

Although both thermal radical generators, which are compounds that generate active species that can initiate polymerization of the polymerizable compound upon heating, and photo-radical generators, which are compounds that generate active species that can initiate polymerization of the polymerizable compound upon exposure to exposure light such as visible light rays, ultraviolet rays (i-line, etc.), far ultraviolet rays, an electron beam, or X-rays, can be used as the radical polymerization initiator, use of a photo-radical generator is preferable.

Examples of photo-radical generators that may be used include acetophenone compounds, biimidazole compounds, triazine compounds, O-acyl oxime compounds, onium salt compounds, benzoin compounds, benzophenone compounds, α-diketone compounds, polynuclear quinone compounds, xanthone compounds, diazo compounds, and imide sulfonate compounds. These compounds are components that generate active radicals, active acid, or both active radicals and active acid upon photoexposure. One photo-radical generator may be used individually, or two or more photo-radical generators may be used in combination.

Specific examples of acetophenone compounds that may be used include 2-hydroxy-2-methyl-1-phenylpropan-1-one, 2-methyl-1-[4-(methylthio)phenyl]-2-morpholinopropan-1-one, 2-benzyl-2-dimethylamino-1-(4-morpholinophenyl)butan-1-one, 1-hydroxycyclohexyl phenyl ketone, 2,2-dimethoxy-1,2-diphenylethan-1-one, 1,2-octanedione, and 2-benzyl-2-dimethylamino-4'-morpholinobutyrophenone.

Specific examples of biimidazole compounds that may be used include 2,2'-bis(2-chlorophenyl)-4,4',5,5'-tetrakis(4-ethoxycarbonylphenyl)-1,2'-biim idazole, 2,2'-bis(2-bromophenyl)-4,4',5,5'-tetrakis(4-ethoxycarbonylphenyl)-1,2'-biim idazole, 2,2'-bis-(2-chlorophenyl)-4,4',5,5'-tetraphenyl-1,2'-biimidazole, 2,2'-bis-(2,4-dichlorophenyl)-4,4',5,5'-tetraphenyl-1,2'-biimidazole, 2,2'-bis-(2,4,6-trichlorophenyl)-4,4',5,5'-tetraphenyl-1,2'-biimidazole, 2,2'-bis-(2-bromophenyl)-4,4',5,5'-tetraphenyl-1,2'-biimidazole, 2,2'-bis-(2,4-dibromophenyl)-4,4',5,5'-tetraphenyl-1,2'-biimidazole, and 2,2'-bis-(2,4,6-tribromophenyl)-4,4',5,5'-tetraphenyl-1,2'-biimidazole.

In a situation in which a biimidazole compound is used as a photoinitiator (photo-radical generator) in the present disclosure, it is preferable that a hydrogen donor is used in combination therewith in terms that sensitivity can be further enhanced.

The term "hydrogen donor" refers to a compound that can donate a hydrogen atom to a radical generated from the biimidazole compound upon photoexposure. The hydrogen donor is preferably a mercaptan compound, an amine compound, or the like such as defined below.

Examples of mercaptan compounds that may be used include 2-mercaptobenzothiazole, 2-mercaptobenzoxazole, 2-mercaptobenzimidazole, 2,5-dimercapto-1,3,4-thiadiazole, and 2-mercapto-2,5-dimethylaminopyridine. Examples of amine compounds that may be used include 4,4'-bis(dimethylamino)benzophenone, 4,4'-bis(diethylamino)benzophenone, 4-diethylaminoacetophenone, 4-dimethylaminopropiophenone, ethyl 4-dimethylaminobenzoate, 4-dimethylaminobenzoic acid, and 4-dimethylaminobenzonitrile.

Examples of triazine compounds that may be used include halomethyl group-containing triazine compounds such as 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-(furan-2-yl)ethenyl]-4,6-bis(trichloromethyl)-s-triazine, 2-[2-(4-diethylamino-2-methylphenyl)ethenyl]-4,6-bis(trichloromethyl)-s-tria zine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, and 2-(4-n-butoxyphenyl)-4,6-bis(trichloromethyl)-s-triazine.

Specific examples of O-acyl oxime compounds that may be used include 1-[4-(phenylthio)phenyl]-heptan-1,2-dione 2-(O-benzoyloxime), 1-[4-(phenylthio)phenyl]-octan-1,2-dione 2-(O-benzoyloxime), 1-[4-(benzoyl)phenyl]-octan-1,2-dione 2-(O-benzoyloxime), 1-[9-ethyl-6-(2-methylbenzoyl)-9H-carbazol-3-yl]-ethanone 1-(O-acetyloxime), 1-[9-ethyl-6-(3-methylbenzoyl)-9H-carbazol-3-yl]ethanone 1-(O-acetyloxime), 1-(9-ethyl-6-benzoyl-9H-carbazol-3-yl)-ethanone 1-(O-acetyloxime), ethanone-1-[9-ethyl-6-(2-methyl-4-tetrathydrofuranylbenzoyl)-9H-carbazol-3 -yl]-1-(O-acetyloxime), ethanone-1-[9-ethyl-6-(2-methyl-4-tetrahydropyranylbenzoyl)-9H-carbazol-3-yl]-1-(O-acetyloxime), ethanone-1-[9-ethyl-6-(2-methyl-5-tetrahydrofuranylbenzoyl)-9H-carbazol-3-yl]-1-(O-acetyloxime), ethanone-1-[9-ethyl-6-(2-methyl-5-tetrahydropyranylbenzoyl)-9H-carbazol-3-yl]-1-(O-acetyloxime), ethanone-1-[9-ethyl-6-{2-methyl-4-(2,2-dimethyl-1,3-dioxolanyl)benzoyl}-9 H-carbazol-3-yl]-1-(O-acetyloxime), ethanone-1-[9-ethyl-6-(2-methyl-4-tetrahydrofuranylmethoxybenzoyl)-9H-car bazol-3-yl]-1-(O-acetyloxime), ethanone-1-[9-ethyl-6-(2-methyl-4-tetrahydropyranylmethoxybenzoyl)-9H-ca rbazol-3-yl]-1-(O-acetyloxime), ethanone-1-[9-ethyl-6-(2-methyl-5-tetrahydrofuranylmethoxybenzoyl)-9H-car bazol-3-yl]-1-(O-acetyloxime), ethanone-1-[9-ethyl-6-(2-methylbenzoyl)-9H-carbazol-3-yl]-1-(O-acetyloxim e), ethanone-1-[9-ethyl-6-(2-methyl-5-tetrahydropyranylmethoxybenzoyl)-9H-ca rbazol-3-yl]-1-(O-acetyloxime), and ethanone-1-[9-ethyl-6-{2-methyl-4-(2,2-dimethyl-1,3-dioxolanyl)methoxyben zoyl}-9H-carbazol-3-yl]-1-(O-acetyloxime).

A commercially available product may be used as a photo-radical generator. Specific examples include Irgacure 907 (product name), Irgacure 184 (product name), Irgacure 369 (product name), Irgacure 651 (product name), Irgacure 819 (product name), Irgacure 907 (product name), and Irgacure OXE02 (product name) produced by BASF, and ADEKA ARKLS N1919T (product name) produced by ADEKA Corporation.

Examples of anionic polymerization initiators that may be used include alkyllithium compounds; monolithium salts and monosodium salts of biphenyl, naphthalene, pyrene, and the like; and polyfunctional initiators such as dilithium salts and trilithium salts.

Examples of cationic polymerization initiators that may be used include proton acids such as sulfuric acid, phosphoric acid, perchloric acid, and trifluoromethanesulfonic acid; Lewis acids such as boron trifluoride, aluminum chloride, titanium tetrachloride, and tin tetrachloride; aromatic onium salts; and a combination of an aromatic onium salt and a reducing agent.

One of these polymerization initiators may be used individually, or two or more of these polymerization initiators may be used in combination.

The proportion in which the polymerization initiator is compounded in the presently disclosed polymerizable composition is normally 0.1 parts by mass to 30 parts by mass, and preferably 0.5 parts by mass to 10 parts by mass per 100 parts by mass of the previously described polymerizable compound.

Moreover, a surfactant is preferably compounded in the presently disclosed polymerizable composition in order to adjust surface tension. Although no specific limitations are placed on the surfactant, a non-ionic surfactant is normally preferable. The non-ionic surfactant may be a commercially available product and may, for example, be a non-ionic surfactant that is an oligomer including a fluorine-containing group, a hydrophilic group, and a lipophilic group. Examples include the SURFLON series (S242, S243, S386, S611, S651, etc.) produced by AGC Seimi Chemical Co., Ltd., the MEGAFACE series (F251, F554, F556, F562, RS-75, RS-76-E, etc.) produced by DIC Corporation, and the Ftergent series (FTX601AD, FTX602A, FTX601ADH2, FTX650A, etc.) produced by Neos Company Limited. One of these surfactants may be used individually, or two or more of these surfactants may be used in combination in a freely selected ratio.

The proportion in which the surfactant is compounded in the presently disclosed polymerizable composition is normally 0.01 parts by mass to 10 parts by mass, and preferably 0.01 parts by mass to 2 parts by mass per 100 parts by mass of all polymerizable compound.

Besides the polymerizable compound, the polymerization initiator, and the surfactant, the presently disclosed polymerizable composition may further contain other components to the extent that the effects disclosed herein are not affected. Examples of these other components include metals, metal complexes, dyes, pigments, fluorescent materials, phosphorescent materials, leveling agents, thixotropic agents, gelling agents, polysaccharides, ultraviolet absorbers, infrared absorbers, antioxidants, ion exchange resins, and metal oxides such as titanium oxide.

Moreover, other copolymerizable monomers may be used as other components. Specific examples include, but are not specifically limited to, 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4'-difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)biphenyl, 4-acryloyloxy-4'-propylcyclohexylphenyl, 4-methacryloyl-4'-butylbicyclohexyl, 4-acryloyl-4'-amyltolan, 4-acryloyl-4'-(3,4-difluorophenyl)bicyclohexyl, 4-amylphenyl 4-(2-acryloyloxyethyl)benzoate, 4-(4'-propylcyclohexyl)phenyl 4-(2-acryloyloxyethyl)benzoate, LC-242 (product name) produced by BASF, trans-1,4-bis[4-[6-(acryloyloxy)hexyloxy]phenyl]cyclohexanedicarboxylate, and other copolymerizable monomers such as compounds disclosed in JP 2007-002208 A, JP 2009-173893 A, JP 2009-274984 A, JP 2010-030979 A, JP 2010-031223 A, JP 2011-006360 A, JP 2010-24438 A, WO 2012/141245 A1, WO 2012/147904 A1, WO 2012/169424 A1, WO 2012/76679 A1, WO 2013/180217 A1, WO 2014/010325 A1, WO 2014/061709 A1, WO 2014/065176 A1, WO 2014/126113 A1, WO 2015/025793 A1, WO 2015/064698 A1, WO 2015/122384 A1, and WO 2015/122385 A1.

The proportion in which these other components are compounded is normally 0.005 parts by mass to 20 parts by mass per 100 parts by mass of all polymerizable compound.

The presently disclosed polymerizable composition can normally be produced by mixing/dissolving specific amounts of the polymerizable compound, the polymerization initiator, other components compounded as desired, and so forth in an appropriate organic solvent.

Examples of organic solvents that may be used include ketones such as cyclopentanone, cyclohexanone, and methyl ethyl ketone; acetic acid esters such as butyl acetate and amyl acetate; halogenated hydrocarbons such as chloroform, dichloromethane, and dichloroethane; and ethers such as 1,4-dioxane, cyclopentyl methyl ether, tetrahydrofuran, tetrahydropyran, and 1,3-dioxolane.

(3) Polymer

The presently disclosed polymer is obtained through polymerization of the previously described polymerizable compound or the previously described polymerizable composition.

Herein, the term "polymerization" is used to refer to a chemical reaction in a broad sense that is inclusive of a normal polymerization reaction and also a crosslinking reaction.

The presently disclosed polymer normally includes the following monomer unit (repeating unit (I)') derived from the polymerizable compound (I).

The following indicates, as one example, the structure of the repeating unit (I)' in a case in which the used polymerizable compound (I) has polymerizable groups represented by $CH_2=CR^1-C(=O)-O-$ as $P^1$ and $P^2$.

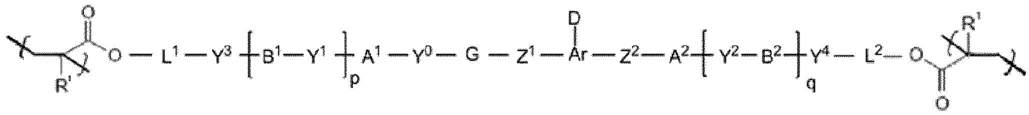

(I)'

[In formula (I)', Ar, D, G, $Z^1$, $Z^2$, $A^1$, $A^2$, $B^1$, $B^2$, $Y^1$, $Y^2$, $Y^3$, $Y^4$, $L^1$, $L^2$, $R^1$, p, and q have the same meaning as previously described.]

The presently disclosed polymer can favorably be used as a constituent material of an optical film or the like as a result of being produced using the polymerizable compound (I).

The presently disclosed polymer may be used in any form depending on the application, such as in the form of a film, a powder, or a layer of aggregated powder, without any specific limitations.

Specifically, a film of the polymer can favorably be used as a constituent material of the subsequently described optical film and optically anisotropic body, a powder of the polymer can be used for a paint, an anti-counterfeiting article, a security article, or the like, and a layer formed from a powder of the polymer can favorably be used as a constituent material of an optically anisotropic body.

The presently disclosed polymer can be more suitably produced by (α) carrying out a polymerization reaction of the polymerizable compound or the polymerizable composition in an appropriate organic solvent, subsequently isolating the target polymer, dissolving the obtained polymer in an appropriate organic solvent to prepare a solution, applying the solution onto an appropriate substrate to obtain an applied film, drying the applied film, and subsequently performing heating as desired, or (β) dissolving the polymerizable compound or the polymerizable composition in an organic solvent, applying the resultant solution onto a substrate by a commonly known application method, removing the solvent, and then carrying out a polymerization reaction through heating or irradiation with active energy rays.

The organic solvent used in the polymerization reaction in method (α) is not specifically limited so long as it is an inert organic solvent. Examples include aromatic hydrocarbons such as toluene, xylene, and mesitylene; ketones such as cyclohexanone, cyclopentanone, and methyl ethyl ketone; acetic acid esters such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Of these organic solvents, those having a boiling point of 60°C to 250°C are preferable, and those having a boiling point of 60°C to 150°C are more preferable from a viewpoint of having excellent handleability.

Examples of the organic solvent in which the isolated polymer is dissolved in method (α) and the organic solvent used in method (β) include ketone solvents such as acetone, methyl ethyl ketone, methyl isobutyl ketone, cyclopentanone, and cyclohexanone; ester solvents such as butyl acetate and amyl acetate; halogenated hydrocarbon solvents such as dichloromethane, chloroform, and dichloroethane; ether solvents such as tetrahydrofuran, tetrahydropyran, 1,2-dimethoxyethane, 1,4-dioxane, cyclopentyl methyl ether, and 1,3-dioxolane; and polar aprotic solvents such as N,N-dimethylformamide, N,N-dimethylacetamide, dimethyl sulfoxide, γ-butyrolactone, and N-methylpyrrolidone. Of these organic solvents, those having a boiling point of 60°C to 200°C are preferable in terms of ease of handling. These solvents may be used individually or as a combination of two or more types.

The substrate used in methods (α) and (β) may be made from a commonly known and typically used organic or inorganic material. Examples of organic materials that may be used include polycycloolefin (for example, ZEONEX® and ZEONOR® (ZEONEX and ZEONOR are registered trademarks in Japan, other countries, or both; produced by ZEON Corporation), ARTON® (ARTON is a registered trademark in Japan, other countries, or both; produced by JSR Corporation), and APEL® (APEL is a registered trademark in Japan, other countries, or both; produced by Mitsui Chemicals, Inc.)), polyethylene terephthalate, polycarbonate, polyimide, polyamide, polymethyl methacrylate, polystyrene, polyvinyl chloride, polytetrafluoroethylene, cellulose, cellulose triacetate, and polyethersulfone. Examples of inorganic materials that may be used include silicon, glass, and calcite.

The substrate that is used may be a single-layer substrate or a laminate.

The substrate is preferably a substrate formed from an organic material, and is more preferably a resin film obtained by shaping an organic material into the form of a film.

Examples of substrates that may be used also include substrates that can be used in preparation of the subsequently described optically anisotropic body.

Commonly known methods can be used as the method by which the solution of the polymer is applied onto the substrate in method (α) and the method by which the solution for polymerization reaction is applied onto the substrate in method (β). Specific examples of methods that may be used include curtain coating, extrusion coating, roll coating, spin coating, dip coating, bar coating, spray coating, slide coating, print coating, gravure coating, die coating, and cap coating.

The method of drying or solvent removal in methods (α) and (β) may be natural drying, heated drying, drying under reduced pressure, heated drying under reduced pressure, or the like.

The method by which polymerization of the polymerizable compound or polymerizable composition is carried out may, for example, be thermopolymerization or polymerization through irradiation with active energy rays. Polymerization through irradiation with active energy rays is preferable in terms that the reaction proceeds at room temperature without the need for heating. In particular, irradiation with light such as ultraviolet light is preferable due to the ease of operation.

The temperature during photoirradiation is preferably 30°C or lower. The photoirradiation intensity is normally within a range of 1 $W/m^2$ to 10 $kW/m^2$, and preferably within a range of 5 $W/m^2$ to 2 $kW/m^2$.

The polymer obtained as set forth above may be transferred from the substrate for use, may be peeled from the substrate and then used alone, or may be used as a constituent material or the like of an optical film or the like without being peeled from the substrate.

Moreover, polymer that is peeled from the substrate may be used after being pulverized by a known method to obtain a powder.

The number-average molecular weight of the presently disclosed polymer obtained as set forth above is preferably 500 to 500,000, and more preferably 5,000 to 300,000. A number-average molecular weight within any of the ranges set forth above is desirable because this provides high hardness and excellent handleability. The number-average molecular weight of the polymer can be measured by gel permeation chromatography (GPC) using monodisperse polystyrene as a standard sample and tetrahydrofuran as an eluent.

Through the presently disclosed polymer, it is possible to obtain an optical film or the like having good wavelength dispersion characteristics at short wavelengths.

(4) Optical film

The presently disclosed optical film includes a layer that is formed using the presently disclosed polymer and/or polymerizable compound and that has an optical function. The term "optical function" refers to simple transmission, reflection, refraction, birefringence, or the like. The presently disclosed optical film may be an optical film having the presently disclosed polymer as a main constituent material of a layer having an optical function or may be an optical film in which a layer having an optical function contains the presently disclosed polymerizable compound. In an optical film having the presently disclosed polymer as a constituent material, it is preferable that the presently disclosed polymer constitutes more than 50 mass% of a layer having an optical function when all components in the layer are taken to be 100 mass%. Moreover, an optical film containing the presently disclosed polymerizable compound preferably contains 0.01 mass% or more of the presently disclosed polymerizable compound when all components in a layer having an optical function are taken to be 100 mass%.

In terms of the form of the presently disclosed optical film, the optical film may be formed on an alignment substrate that optionally includes an alignment film (i.e., an "alignment substrate/(alignment film)/optical film" form), the optical film may be transferred onto a transparent substrate film or the like differing from an alignment substrate (i.e., a "transparent substrate film/optical film" form), or the optical film may be used as a single layer in a case in which the optical film is self-supporting (i.e., an "optical film" form).

The alignment film and the alignment substrate may the same as a substrate and an alignment film of the subsequently described optically anisotropic body.

The presently disclosed optical film can be produced by methods such as (A) applying a solution containing the presently disclosed polymerizable compound or a solution of the polymerizable composition onto an alignment substrate, drying the resultant applied film, performing heat treatment (liquid crystal alignment), and then carrying out photoirradiation and/or heating (polymerization), (B) applying a solution of a liquid crystal polymer obtained through polymerization of the presently disclosed polymerizable compound or polymerizable composition onto an alignment substrate, and optionally drying the resultant applied film, and (C) applying a solution containing the presently disclosed polymerizable compound and a resin onto an alignment substrate, and then drying the resultant applied film.

The presently disclosed optical film can be used for an optically anisotropic body, an alignment film for a liquid crystal display element, a color filter, a low-pass filter, a light polarizing prism, various light filters, and so forth.

The following values $\alpha$ to $\delta$ for the presently disclosed optical film that are determined from retardation at wavelengths of 400 nm, 410 nm, 420 nm, 430 nm, and 550 nm measured by an ellipsometer are preferably within specific ranges. Specifically, the $\alpha$ value is preferably 0.10 to 0.75, more preferably 0.15 or more, and even more preferably 0.35 or more. The $\beta$ value is preferably 0.40 to 0.75, and more preferably 0.55 or more. The $\gamma$ value is preferably 0.55 to 0.80, and more preferably 0.60 or more. The $\delta$ value is preferably 0.65 to 0.85, and more preferably 0.70 or more.

$$\alpha = (\text{Retardation at 400 nm})/(\text{Retardation at 550 nm})$$

$$\beta = (\text{Retardation at 410 nm})/(\text{Retardation at 550 nm})$$

$$\gamma = (\text{Retardation at 420 nm})/(\text{Retardation at 550 nm})$$

$$\delta = (\text{Retardation at 430 nm})/(\text{Retardation at 550 nm})$$

(5) Optically anisotropic body

The presently disclosed optically anisotropic body includes a layer having the presently disclosed polymer as a constituent material.

The presently disclosed optically anisotropic body can be obtained by, for example, forming an alignment film on a substrate and then forming a layer formed from the presently disclosed polymer (liquid crystal layer) on the alignment film. Note that the presently disclosed optically anisotropic body may be a body obtained by forming a layer formed from the presently disclosed polymer (liquid crystal layer) directly on a substrate or may be a body composed only of a layer formed from the presently disclosed polymer (liquid crystal layer).

The layer formed from the polymer may be a layer formed from a film-like polymer or may be an aggregate of a powder-like polymer.

The alignment film is formed on the surface of the substrate in order to regulate in-plane alignment of polymerizable liquid crystal compound in one direction.

The alignment film can be obtained by applying a solution containing a polymer such as a polyimide, polyvinyl alcohol, polyester, polyarylate polyamide imide, or polyetherimide (composition for alignment film) onto a substrate as a film, drying the film, and then performing rubbing or the like in one direction.

The thickness of the alignment film is preferably 0.001 $\mu$m to 5 $\mu$m, and more preferably 0.001 $\mu$m to 1.0 $\mu$m.

The method by which rubbing is performed is not specifically limited and may, for example, be a method in which the alignment film is rubbed in a given direction using a roll around which cloth or felt formed from synthetic fiber (for example, nylon) or natural fiber (for example, cotton) is wound. It is preferable to wash the alignment film with isopropyl alcohol or the like after the rubbing to remove fine powder (foreign matter) formed during the rubbing and to clean the surface of the alignment film.

Besides rubbing methods, a function of regulating in-plane alignment in one direction can be imparted through a method in which the surface of an alignment film is irradiated with polarized ultraviolet rays.

The substrate on which the alignment film is formed may, for example, be a glass substrate, a substrate formed from a synthetic resin film, or the like. Examples of synthetic resins that may be used include thermoplastic resins such as acrylic resin, polycarbonate resin, polyethersulfone resin, polyethylene terephthalate resin, polyimide resin, polymethyl methacrylate resin, polysulfone resin, polyarylate resin, polyethylene resin, polystyrene resin, polyvinyl chloride resin, cellulose diacetate, cellulose triacetate, and alicyclic olefin polymers.

Examples of alicyclic olefin polymers include cycloolefin random multicomponent copolymers described in JP H05-310845 A and the Specification of US 5179171 A, hydrogenated polymers described in JP H05-97978 A and the Specification of US 5202388 A, and thermoplastic dicyclopentadiene ring-opened polymers and hydrogenated products thereof described in JP H11-124429 A (WO 99/20676 A1).

The method by which a liquid crystal layer formed from the presently disclosed polymer is formed on the alignment film may, for example, be the same as any of the methods described in the section pertaining the presently disclosed polymer (methods ($\alpha$) and ($\beta$)).

Although no specific limitations are placed on the thickness of the liquid crystal layer that is obtained, the thickness is normally 1 $\mu$m to 10 $\mu$m.

The presently disclosed optically anisotropic body may be a retardation plate, a viewing angle enhancement plate, or the like, but is not specifically limited to these types of optically anisotropic bodies.

$\alpha$ to $\delta$ values for the presently disclosed optically anisotropic body that are determined by the same method as for the optical film in section (4) are preferably within the specific ranges described in section (4).

(6) Polarizer, etc.

The presently disclosed polarizer includes the presently disclosed optically anisotropic body and a polarizing film.

A specific example of the presently disclosed polarizer is a polarizer in which the presently disclosed optically anisotropic body in stacked on a polarizing film either directly or with another layer (for example, a glass sheet) in-between.

No specific limitations are placed on the method by which the polarizing film is produced. Examples of methods by which a PVA polarizing film can be produced include a method in which adsorption of iodine ions by a PVA film is carried out and then uniaxial stretching is performed, a method in which uniaxial stretching of a PVA film is performed and then adsorption of iodine ions is carried out, a method in which adsorption of iodine ions to a PVA film and uniaxial stretching are performed simultaneously, a method in which a PVA film is dyed using a dichroic dye and is then uniaxially stretched, a method in which a PVA film is uniaxially stretched and is then dyed using a dichroic dye, and a method in which dying of a PVA film using a dichroic dye and uniaxial stretching are performed simultaneously. Examples of methods by which a polyene polarizing film can be produced include commonly known methods such as a method in which a PVA film is uniaxially stretched and is then heated and dehydrated in the presence of a dehydration catalyst and a method in which a polyvinyl chloride film is uniaxially stretched and is then heated and dehydrated in the presence of a dehydrochlorination catalyst.

In the presently disclosed polarizer, the polarizing film and the presently disclosed optically anisotropic body may be in contact via an adhesive layer formed from an adhesive (inclusive of pressure-sensitive adhesives). The average thickness of the adhesive layer is normally 0.01 $\mu$m to 30 $\mu$m, and preferably 0.1 $\mu$m to 15 $\mu$m. The adhesive layer is preferably a layer having a tensile fracture strength of 40 MPa or less according to JIS K7113.

Examples of adhesives that may form the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene-butadiene-styrene copolymer (SBS copolymer) adhesives and adhesives that are hydrogenated products thereof (SEBS copolymers), ethylene adhesives such as ethylene-vinyl acetate copolymers and ethylene-styrene copolymers, and acrylic acid ester adhesives such as ethylene-methyl methacrylate copolymers, ethylene-methyl acrylate copolymers, ethylene-ethyl methacrylate copolymers, and ethylene-ethyl acrylate copolymers.

The presently disclosed polarizer has good wavelength dispersion characteristics at short wavelengths as a result of the presently disclosed optically anisotropic body being used therein.

The presently disclosed polarizer can be used to suitably produce a flat panel display in which a liquid crystal panel is used, an organic electroluminescence display in which an organic electroluminescence panel is used, or an antireflection film.

(7) Compound

The presently disclosed compound is useful as a production intermediate of the previously described polymerizable compound (I). One example of the compound is a compound indicated by the following formula (VII-1) or (VII-2). Hereinafter, a compound indicated by formula (VII-1) is also referred to as "compound (VII-1)" and a compound indicated by formula (VII-2) is also referred to as "compound (VII-2)".

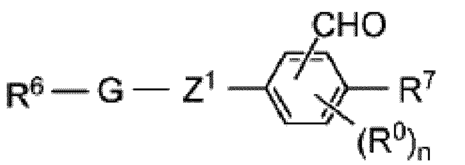

(VII-1)

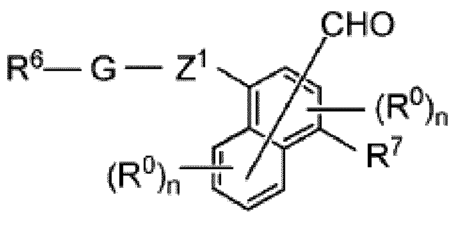

(VII-2)

In formulae (VII-1) and (VII-2), $Z^1$, G, and $R^0$ have the same meaning as previously described. Moreover, each n is independently an integer of 0 to 4. $R^6$ and $R^7$ each represent, independently of one another, $-OR^e$, $-CH_2OR^e$, $-CH_2CH_2OR^e$, $-C(=O)-OR^e$, $-CH_2-C(=O)-OR^e$, $-CH_2CH_2-C(=O)-OR^e$, a hydroxy group, a carboxyl group, $-CH_2-C(=O)-OH$, $-CH_2CH_2-C(=O)-OH$, $-CH_2OH$, $-CH_2CH_2OH$, or an amino group. $R^e$ represents a protecting group. It is preferable that $R^6$ and $R^7$ are each, independently of one another, $-OR^e$, $-CH_2OR^e$, $-CH_2CH_2OR^e$, $-CH_2OH$, $-CH_2CH_2OH$, or a hydroxy group, more preferably a combination in which $R^6$ is a hydroxy group, $-OR^e$, $-CH_2OR^e$, or $-CH_2CH_2OR^e$ and $R^7$ is a hydroxy group, $-CH_2OH$, or $-CH_2CH_2OH$, and particularly preferably a combination in which $R^6$ is a hydroxy group, $-OR^e$, $-CH_2OR^e$, or $-CH_2CH_2OR^e$ and $R^7$ is a hydroxy group.

Examples of the protecting group $R^e$ in formulae (VII-1) and (VII-2) include, but are not specifically limited to, a tetrahydropyranyl group, a methoxymethyl group, a 2-methoxyethoxymethyl group, a tert-butyldimethylsilyl group, a trimethylsilyl group, and a benzyl group. Of these examples, the protecting group $R^e$ is preferably a tetrahydropyranyl group, a 2-methoxyethoxymethyl group, or a tert-butyldimethylsilyl group.

The compound (VII-1) and the compound (VII-2) can be synthesized through a combination of known synthetic

reactions. Specifically, these compounds can be synthesized with reference to methods described in various documents (for example, March's Advanced Organic Chemistry (Wiley); and Sandler and Karo, "Syntheses of Organic Compounds Classified by Functional Group", joint translation by Naoki INAMOTO (Hirokawa Publishing Company)).

The compound (VII-1) is preferably (VII-1-1) or (VII-1-2), shown below, and is particularly preferably (VII-1-1). The compound (VII-2) is preferably (VII-2-1) or (VII-2-2), shown below, and is particularly preferably (VII-2-1). In terms of substituents, a structure in which n = 0 (i.e., a structure that does not include $R^0$) is preferable.

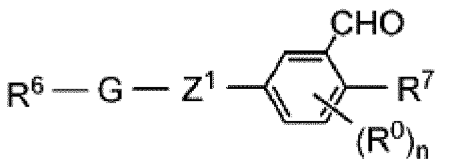

(VII-1-1)

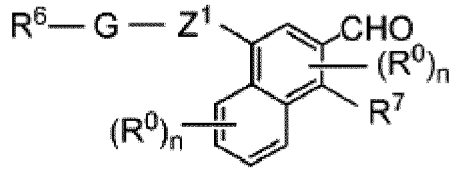

(VII-2-1)

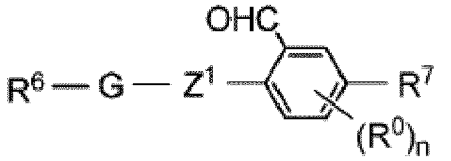

(VII-1-2)

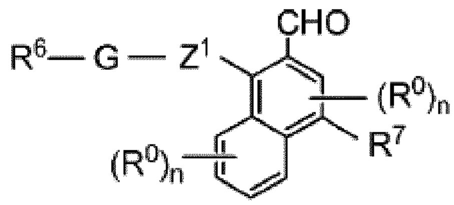

(VII-2-2)

Another example of the presently disclosed compound is a compound represented by the following formula (VIII-1) or (VIII-2). Hereinafter, a compound represented by formula (VIII-1) is also referred to as "compound (VIII-1)" and a compound represented by formula (VIII-2) is also referred to as "compound (VIII-2)".

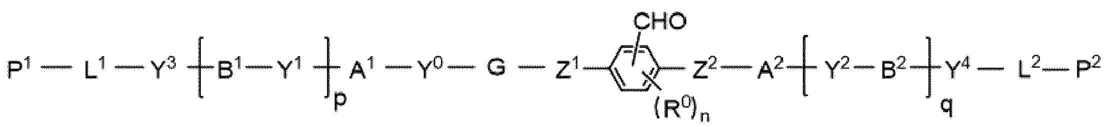

(VIII-1)

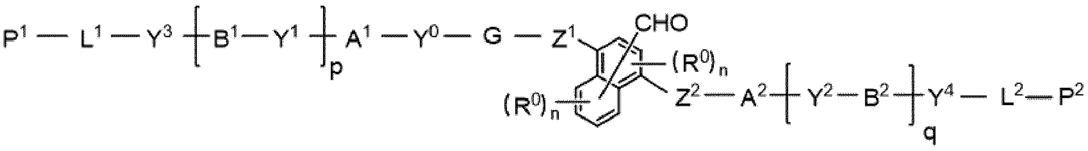

(VIII-2)

In formulae (VIII-1) and (VIII-2), $Z^1$, $Z^2$, G, $Y^0$ to $Y^4$, $A^1$, $A^2$, $B^1$, $B^2$, $L^1$, $L^2$, $P^1$, $P^2$, $R^0$, n, p, and q have the same meaning as previously described.

The compounds (VIII-1) and (VIII-2) can be synthesized through a combination of known synthetic reactions using the previously described compounds (VII-1) and (VII-2) as materials. Specifically, these compounds can be synthesized with reference to methods described in various documents (for example, March's Advanced Organic Chemistry (Wiley); and Sandler and Karo, "Syntheses of Organic Compounds Classified by Functional Group", joint translation by Naoki INAMOTO (Hirokawa Publishing Company)).

The compound (VIII-1) is preferably a compound represented by formula (VIII-1-1) or (VIII-1-2), shown below, and is particularly preferably a compound represented by formula (VIII-1-1). The compound (VIII-2) is preferably a compound represented by formula (VIII-2-1) or (VIII-2-2), shown below, and is particularly preferably a compound represented by (VIII-2-1). Moreover, n is preferably 0.

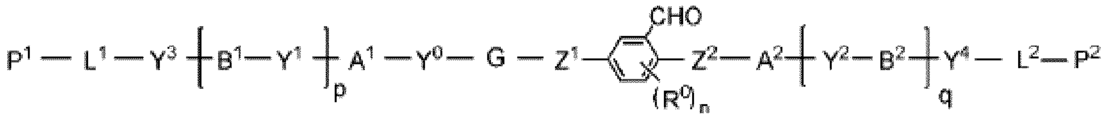

(VIII-1-1)

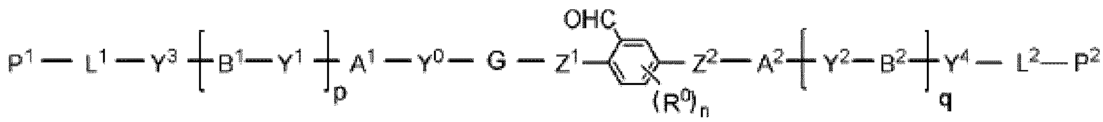

(VIII-1-2)

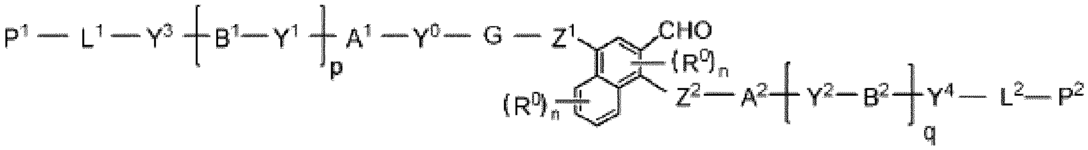

(VIII-2-1)

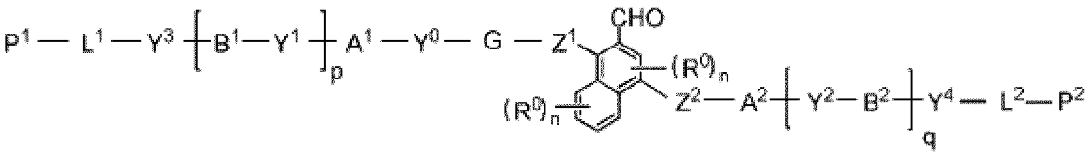

(VIII-2-2)

The compound (VIII-1-1) is preferably a compound represented by formula (X-1), shown below. Moreover, the compound (VIII-2-2) is preferably a compound represented by formula (X-2), shown below. Furthermore, among compounds represented by formulae (X-1) and (X-2), compounds represented by formulae (X-1-1) to (X-2-2) are even more preferable, and compounds represented by formulae (X-1-1) and (X-2-1) are particularly preferable.

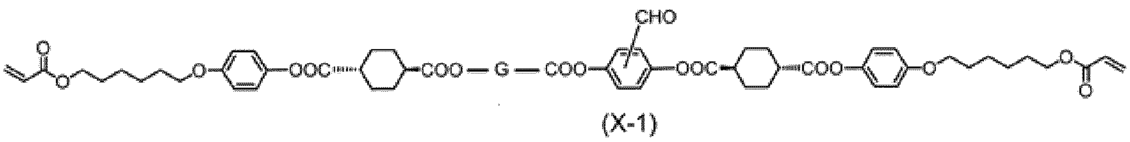

(X-1)

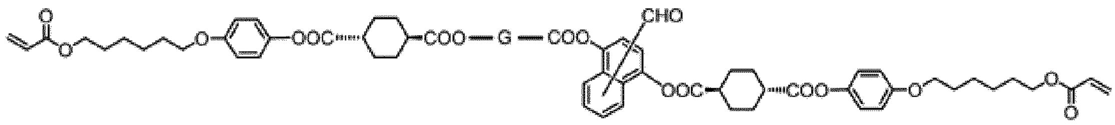

(X-2)

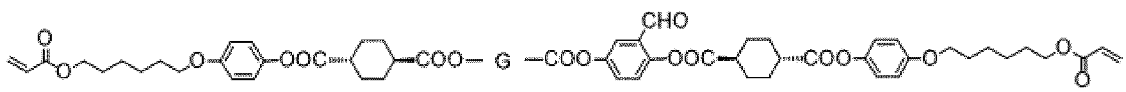

(X-1-1)

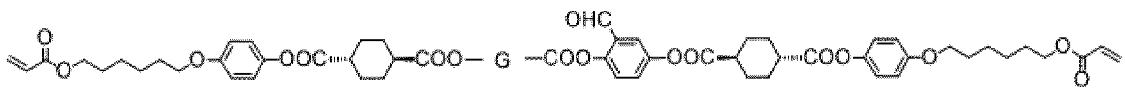

(X-1-2)

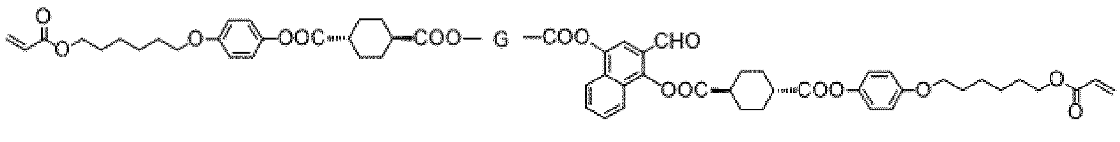

(X-2-1)

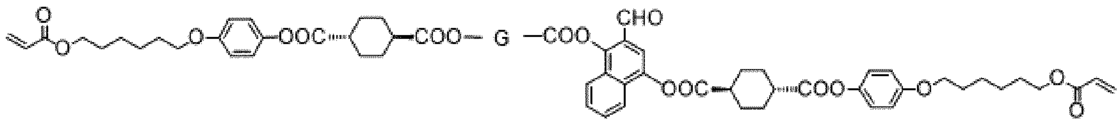

(X-2-2)

[In formulae (X-1), (X-2), and (X-1-1) to (X-2-2), G has the same meaning as previously described.]

EXAMPLES

The following provides a more detailed description of the present disclosure through examples. However, the present disclosure is not in any way limited by the following examples.

(Synthesis Example 1) Synthesis of compound 1

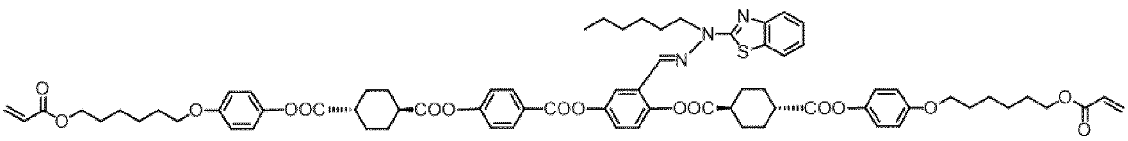

(Compound 1): Formula (XI-1)

Step 1: Synthesis of intermediate A

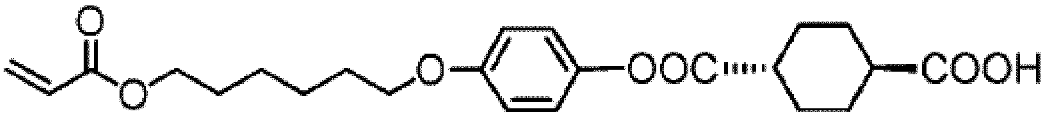

(Intermediate A)

A three-necked reaction vessel equipped with a thermometer was charged with 17.98 g (104.42 mmol) of trans-1,4-cyclohexanedicarboxylic acid and 180 mL of tetrahydrofuran (THF) in a stream of nitrogen. In addition, 6.58 g (57.43 mmol) of methanesulfonyl chloride was added into the reaction vessel and the reaction vessel was immersed in a water bath to attain a reaction liquid internal temperature of 20°C. Next, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C.

Next, 0.64 g (5.22 mmol) of 4-(dimethylamino)pyridine and 13.80 g (52.21 mmol) of 4-(6-acryloyloxy-hex-1-yloxy)phenol (produced by DKSH) were added to the resultant reaction liquid, and the reaction vessel was immersed in a water bath once again to attain a reaction liquid internal temperature of 15°C. Thereafter, 6.34 g (62.65 mmol) of triethylamine was added dropwise over 10 minutes while maintaining the reaction liquid internal temperature at 20°C to 30°C. After completion of the dropwise addition, the entire contents of the reaction vessel were further stirred for 2 hours at 25°C. Once the reaction ended, 1,000 mL of distilled water and 100 mL of saturated saline water were added to the reaction liquid, and two extractions were performed with 400 mL of ethyl acetate. The organic layers were collected and were dried with anhydrous sodium sulfate, and then sodium sulfate was filtered off. Solvent was removed from the filtrate by evaporation in a rotary evaporator and then the obtained residue was purified by silica gel column chromatography

(THF:toluene = 1:9 (volume ratio; same applies below)). Purification by silica gel column chromatography was repeated until the purity as analyzed by high-performance liquid chromatography was 99.5% or higher. As a result, 14.11 g (yield: 65 mol%) of intermediate A in the form of a white solid was obtained.

The structure of the target was identified by $^1$H-NMR. The results are shown below.
$^1$H-NMR (500 MHz, DMSO-$d_6$, TMS, δ ppm): 12.12 (s, 1H), 6.99 (d, 2H, J = 9.0 Hz), 6.92 (d, 2H, J = 9.0 Hz), 6.32 (dd, 1H, J = 1.5 Hz, 17.5 Hz), 6.17 (dd, 1H, J = 10.0 Hz, 17.5 Hz), 5.93 (dd, 1H, J = 1.5 Hz, 10.0 Hz), 4.11 (t, 2H, J = 6.5 Hz), 3.94 (t, 2H, J = 6.5 Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H)

Step 2: Synthesis of intermediate B

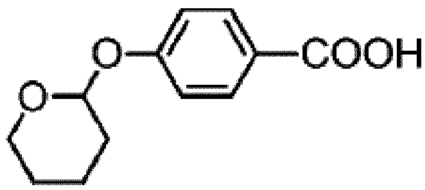

(Intermediate B)

In a three-necked reaction vessel equipped with a thermometer, 20 g (145 mmol) of 4-hydroxybenzoic acid and 14.62 g (145 mmol) of 3,4-dihydro-2H-pyran were added to 200 mL of tetrahydrofuran in a stream of nitrogen, and a homogeneous solution was obtained. The reaction vessel was immersed in a cold water bath to attain a reaction liquid internal temperature of 15°C. Next, 336 mg (1.45 mmol) of (±)-10-camphorsulfonic acid was added into the reaction vessel. Thereafter, the entire contents of the reaction vessel were returned to 25°C and were stirred for 6 hours. Once the reaction ended, 1 L of distilled water and 100 mL of saturated saline water were added to the resultant reaction liquid, and two extractions were performed with 300 mL of ethyl acetate. The organic layers were collected and were dried with anhydrous sodium sulfate, and then sodium sulfate was filtered off. Solvent was removed in a rotary evaporator and then recrystallization of the resultant residue was performed with ethyl acetate as a solvent. Precipitated crystals were collected by filtration. The obtained crystals were washed with cold ethyl acetate and then vacuum dried to yield 9.0 g (yield: 28 mol%) of intermediate B in the form of a white solid. The structure of the target was identified by $^1$H-NMR. The results are shown below.
$^1$H-NMR (500 MHz, DMSO-$d_6$, TMS, δ ppm): 12.66 (s, 1H), 7.89 (d, 2H, J = 9.0 Hz), 7.09 (d, 2H, J = 9.0 Hz), 5.58 (t, 1H, J = 3.5 Hz), 3.75-3.70 (m, 1H), 3.59-3.55 (m, 1H), 1.92-1.48 (m, 6H)

Step 3: Synthesis of intermediate C (one example of compound of formula (VII-1))

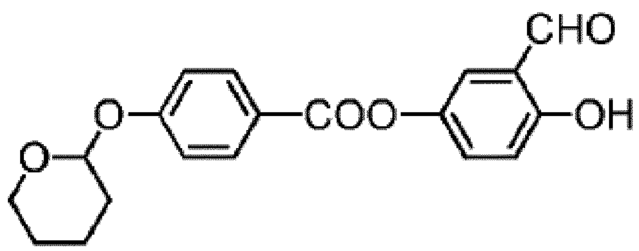

(Intermediate C)

In a three-necked reaction vessel equipped with a thermometer, 6.0 g (27 mmol) of the intermediate B synthesized in step 2, 3.73 g (27 mmol) of 2,5-dihydroxybenzaldehyde, and 330 mg (2.7 mmol) of N,N-dimethylaminopyridine were added to 110 mL of chloroform in a stream of nitrogen. The reaction vessel was vigorously stirred while slowly adding 4.09 g (32.4 mmol) of N,N'-diisopropylcarbodiimide dropwise at 25°C. Thereafter, the reaction vessel was stirred for 2 hours at 25°C to carry out a reaction. Once the reaction ended, 1 L of distilled water and 100 mL of saturated saline water were added to the resultant reaction liquid, and two extractions were performed with 500 mL of ethyl acetate. The

organic layers were collected and were washed with 500 mL of saturated saline water. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 97:3 (volume ratio)) to yield 4.5 g (yield: 49 mol%) of intermediate C in the form of a white solid. The structure of the target was identified by $^1$H-NMR. The results are shown below.
$^1$H-NMR (500 MHz, $CDCl_3$, TMS, $\delta$ ppm): 10.94 (s, 1H), 9.87 (d, 1H, J = 0.5 Hz), 8.13 (d, 2H, J = 9.0 Hz), 7.44 (d, 1H, J = 3.0 Hz), 7.37 (dd, 1H, J = 3.0 Hz, 9.0 Hz), 7.15 (d, 2H, J = 9.0 Hz), 7.02 (d, 1H, J = 9.0 Hz), 5.55 (t, 1H, J = 3.0 Hz), 3.89-3.84 (m, 1H), 3.66-3.62 (m, 1H), 2.07-1.95 (m, 1H), 1.93-1.83 (m, 2H), 1.77-1.57 (m, 3H)

Step 4: Synthesis of intermediate D (another example of compound of formula (VII-1))

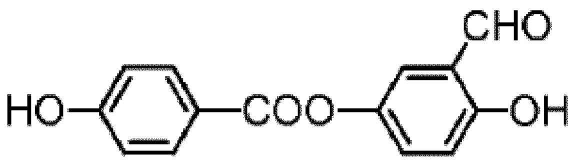

(Intermediate D)

In a three-necked reaction vessel equipped with a thermometer, 3.0 g (8.76 mmol) of the intermediate C synthesized in step 3 was added to 40 mL of a mixed solution of acetic acid/tetrahydrofuran/water (= 4/2/1 (mass ratio)) in a stream of nitrogen. The entire contents of the reaction vessel were subsequently heated to 45°C and were stirred for 6 hours. Once the reaction ended, 500 mL of distilled water was added to the resultant reaction liquid, and two extractions were performed with 200 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:ethyl acetate = 80:20 (volume ratio)) to yield 1.5 g (yield: 66 mol%) of intermediate D in the form of a white solid. The structure of the target was identified by $^1$H-NMR. The results are shown below.
$^1$H-NMR (500 MHz, THF-$d_8$, TMS, $\delta$ ppm): 10.91 (s, 1H), 10.07 (s, 1H), 9.36 (s, 1H), 8.15 (d, 2H, J = 9.0 Hz), 7.69 (d, 1H, J = 3.0 Hz), 7.51 (dd, 1H, J = 3.0 Hz, 9.0 Hz), 7.10 (d, 1H, J = 9.0 Hz), 6.98 (d, 2H, J = 9.0 Hz)

Step 5: Synthesis of intermediate E

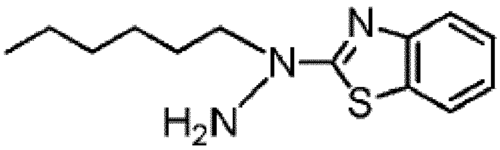

(Intermediate E)

In a four-necked reaction vessel equipped with a thermometer, 2.00 g (12.1 mmol) of 2-hydrazinobenzothiazole was dissolved in 20 mL of dimethylformamide in a stream of nitrogen. Next, 8.36 g (60.5 mmol) of potassium carbonate and 3.08 g (14.5 mmol) of 1-iodohexane were added to the solution and were stirred therewith for 7 hours at 50°C. Once the reaction ended, the reaction liquid was cooled to 20°C and was added into 200 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:ethyl acetate = 75:25 (volume ratio)) to yield 2.10 g (yield: 69.6 mol%) of intermediate E in the form of a white solid. The structure was identified by $^1$H-NMR. The results are shown below.
$^1$H-NMR (500 MHz, $CDCl_3$, TMS, $\delta$ ppm): 7.60 (dd, 1H, J = 1.0, 8.0 Hz), 7.53 (dd, 1H, J = 1.0, 8.0 Hz), 7.27 (ddd, 1H, J = 1.0, 8.0, 8.0 Hz), 7.06 (ddd, 1H, J = 1.0, 8.0, 8.0 Hz), 4.22 (s, 2H), 3.74 (t, 2H, J = 7.5 Hz), 1.69-1.76 (m, 2H), 1.29-1.42

(m, 6H), 0.89 (t, 3H, J = 7.0 Hz)

Step 6: Synthesis of compound 1

In a four-necked reaction vessel equipped with a thermometer, 1.0 g (3.87 mmol) of the intermediate D synthesized in step 4, 3.56 g (8.51 mmol) of the intermediate A synthesized in step 1, and 47 mg (0.39 mmol) of N,N-dimethylaminopyridine were added to 100 mL of chloroform in a stream of nitrogen. Dropwise addition of 1.12 g (8.9 mmol) of N,N'-diisopropylcarbodiimide to the solution was performed slowly at 25°C. The solution was then stirred for 3 hours at 25°C. Once the reaction ended, a rotary evaporator was used to remove solvent from reaction liquid obtained by sampling a portion of the resultant solution. The resultant residue was then purified by silica gel column chromatography (chloroform:tetrahydrofuran = 95:5 (volume ratio)), and the presence of intermediate F was confirmed by $^1$H-NMR. The results are shown below. Note that the intermediate F is a compound that, among compounds of formula (VIII-1), is one example of a compound indicated by formula (X-1).

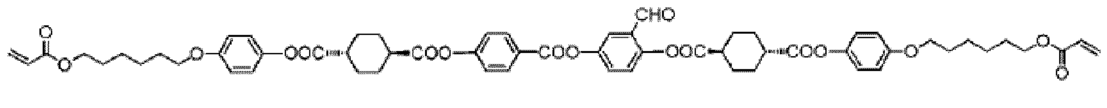

(Intermediate F)

$^1$H-NMR (500 MHz, $CDCl_3$, TMS, $\delta$ ppm): 10.10 (s, 1H), 8.24 (d, 2H, J = 8.5 Hz), 7.75 (d, 1H, J = 3.0 Hz), 7.52 (dd, 1H, J = 6.0 Hz, 9.0 Hz), 7.27-7.25 (m, 3H), 7.00-6.96 (m, 4H), 6.88-6.87 (d, 4H, J = 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.17 (t, 4H, J = 6.5 Hz), 3.94 (t, 4H, J = 6.5 Hz), 2.78-2.68 (m, 1H), 2.68-2.56 (m, 3H), 2.40-2.25 (m, 8H), 1.84-1.40 (m, 24H)

The remaining reaction liquid was cooled in an ice bath and then 1.16 g (4.6 mmol) of the intermediate E synthesized in step 5 and 14 mL of 1 N hydrochloric acid aqueous solution were added thereto. The solution was then stirred for 3 hours at 40°C. Once the reaction ended, the solution was added into 500 mL of 10 mass% sodium bicarbonate water, and two extractions were performed with 500 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:THF = 95:5 (volume ratio)) to yield 3.0 g (yield: 60%) of compound 1 in the form of a pale yellow solid. The structure of the target was identified by $^1$H-NMR. The results are shown below.

$^1$H-NMR (500 MHz, $CDCl_3$, TMS, $\delta$ ppm): 8.29 (d, 2H, J = 9.0 Hz), 7.89 (d, 1H, J = 3.0 Hz), 7.70 (s, 1H), 7.67-7.64 (m, 2H), 7.33 (dd, 1H, J = 1.0 Hz, 7.5 Hz), 7.30-7.25 (m, 2H), 7.23 (dd, 1H, J = 3.0 Hz, 9.0 Hz), 7.18-7.13 (m, 2H), 6.98 (d, 4H, J = 9.0 Hz), 6.88 (dd, 4H, J = 1.0 Hz, 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.32 (t, 2H, J = 7.5 Hz), 4.18 (t, 4H, J = 6.5 Hz), 3.95 (t, 4H, J = 6.5 Hz), 2.75-2.55 (m, 4H), 2.39-2.26 (m, 8H), 1.84-1.30 (m, 32H), 0.91 (t, 3H, J = 7.0 Hz)

(Synthesis Example 2) Synthesis of compound 2

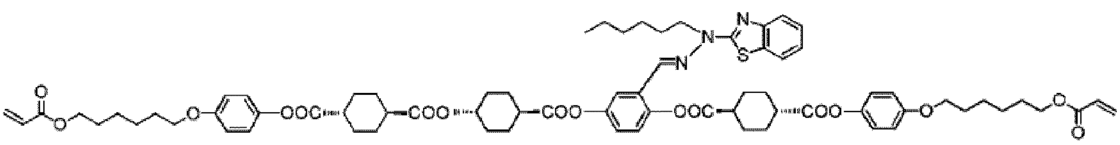

(Compound 2): Formula (XI-2)

Step 1: Synthesis of intermediate G (another example of compound of formula (VII-1))

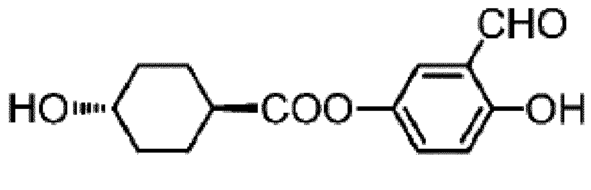

(Intermediate G)

In a three-necked reaction vessel equipped with a thermometer, 5.0 g (34.7 mmol) of trans-4-hydroxycyclohexanecarboxylic acid, 4.79 g (34.7 mmol) of 2,5-dihydroxybenzaldehyde, and 424 mg (3.47 mmol) of N,N-dimethylaminopyridine were added to 100 mL of chloroform in a stream of nitrogen. The reaction vessel was vigorously stirred while 5.25 g (41.6 mmol) of N,N'-diisopropylcarbodiimide was slowly added dropwise at 15°C. Thereafter, the reaction vessel was stirred for 8 hours at 25°C to carry out a reaction. Once the reaction ended, 1 L of distilled water and 100 mL of saturated saline water were added to the resultant reaction liquid, and two extractions were performed with 500 mL of ethyl acetate. The organic layers were collected and were washed with 500 mL of saturated saline water. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 75:25 (volume ratio)) to yield 5.0 g (yield: 55 mol%) of intermediate G in the form of a white solid. The structure of the target was identified by $^1$H-NMR. The results are shown below.
$^1$H-NMR (500 MHz, DMSO-$d_6$, TMS, $\delta$ ppm): 10.77 (s, 1H), 10.25 (s, 1H), 7.31 (d, 1H, J = 3.0 Hz), 7.26 (dd, 1H, J = 3.0 Hz, 9.0 Hz), 7.02 (d, 1H, J = 9.0 Hz), 4.62 (d, 1H, J = 4.0 Hz), 3.44-3.36 (m, 1H), 2.49-2.45 (m, 1H), 2.04-1.99 (m, 2H), 1.90-1.86 (m, 2H), 1.52-1.43 (m, 2H), 1.27-1.19 (m, 2H)

Step 2: Synthesis of compound 2

In a four-necked reaction vessel equipped with a thermometer, 2.5 g (9.53 mmol) of the intermediate G synthesized in step 1, 8.77 g (21 mmol) of intermediate A synthesized in the same way as in step 1 of Synthesis Example 1, and 232 mg (1.9 mmol) of N,N-dimethylaminopyridine were added to 150 mL of chloroform in a stream of nitrogen. Dropwise addition of 2.77 g (21.9 mmol) of N,N'-diisopropylcarbodiimide to the solution was performed slowly at 25°C. The solution was then stirred for 4 hours at 25°C. Once the reaction ended, a rotary evaporator was used to remove solvent from reaction liquid obtained by sampling a portion of the resultant solution. The resultant residue was then purified by silica gel column chromatography (chloroform:tetrahydrofuran = 95:5 (volume ratio)), and the presence of intermediate H was confirmed by $^1$H-NMR. The results are shown below. Note that intermediate H is a compound that, among compounds of formula (VIII-1), is one example of a compound indicated by formula (X-1).

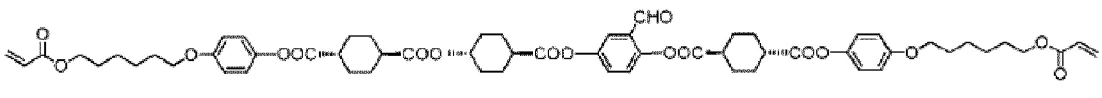

(Intermediate H)

$^1$H-NMR (500 MHz, $CDCl_3$, TMS, $\delta$ ppm): 10.07 (s, 1H), 7.60 (d, 1H, J = 3.0 Hz), 7.35 (dd, 1H, J = 3.0 Hz, 9.0 Hz), 7.20 (d, 1H, J = 9.0 Hz), 6.99-6.94 (m, 4H), 6.89-6.86 (m, 4H), 6.40 (dd, 2H, J = 0.5 Hz, 17.5 Hz), 6.12 (dd, 2H, J = 10.5 Hz, 17.5 Hz), 5.82 (dd, 2H, J = 0.5 Hz, 10.5 Hz), 4.78 (dddd, 1H, J = 4.0 Hz, 4.0 Hz, 10.0 Hz, 10.0 Hz), 4.172 (t, 2H, J = 6.5 Hz), 4.171 (t, 2H, J = 6.5 Hz), 3.941 (t, 2H, J = 6.5 Hz), 3.935 (t, 2H, J = 6.5 Hz), 2.76-2.65 (m, 1H), 2.63-2.48 (m, 3H), 2.38-2.05 (m, 13H), 1.83-1.43 (m, 28H)

The remaining reaction liquid was cooled in an ice bath and then 2.85 g (11.4 mmol) of intermediate E synthesized in the same way as in step 5 of Synthesis Example 1 and 1.1 g (4.77 mmol) of (±)-10-camphorsulfonic acid were added thereto. This solution was allowed to react for 4 hours at 45°C. Once the reaction ended, the solution was added into 1 L of 3 mass% sodium bicarbonate water, and two extractions were performed with 500 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 2.1 g (yield: 17 mol%) of compound 2 in the form of a pale yellow solid. The structure of the target was identified by $^1$H-NMR. The results are shown below.
$^1$H-NMR (500 MHz, $CDCl_3$, TMS, $\delta$ ppm): 7.73 (d, 1H, J = 3.0 Hz), 7.70-7.66 (m, 3H), 7.36-7.32 (m, 1H), 7.18-7.15 (m, 1H), 7.13-7.07 (m, 2H), 6.99-6.95 (m, 4H), 6.90-6.85 (m, 4H), 6.403 (dd, 1H, J = 1.5 Hz, 17.5 Hz), 6.401 (dd, 1H, J = 1.5 Hz, 17.5 Hz), 6.125 (dd, 1H, J = 10.5 Hz, 17.5 Hz), 6.124 (dd, 1H, J = 10.5 Hz, 17.5 Hz), 5.822 (dd, 1H, J = 1.5 Hz,

10.5 Hz), 5.820 (dd, 1H, J = 1.5 Hz, 10.5 Hz), 4.84-4.78 (m, 1H), 4.29 (t, 2H, J = 7.5 Hz), 4.174 (t, 2H, J = 6.5 Hz), 4.172 (t, 2H, J = 6.5 Hz), 3.944 (t, 2H, J = 6.5 Hz), 3.937 (t, 2H, J = 6.5 Hz), 2.72-2.49 (m, 4H), 2.37-2.08 (m, 13H), 1.83-1.30 (m, 36H), 0.900 (t, 3H, J = 7.0 Hz)

(Synthesis Example 3) Synthesis of compound 3

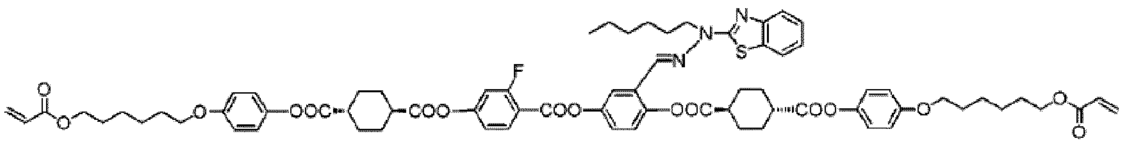

(Compound 3): Formula (XI-3)

Step 1: Synthesis of intermediate I

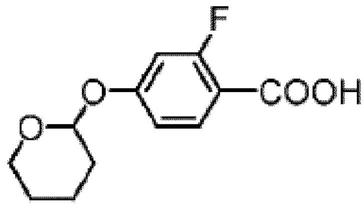

(Intermediate I)

In a three-necked reaction vessel equipped with a thermometer, 5 g (32.03 mmol) of 2-fluoro-4-hydroxybenzoic acid and 10.78 g (128.15 mmol) of 3,4-dihydro-2H-pyran were added to 50 mL of THF in a stream of nitrogen, and a homogeneous solution was obtained. The reaction vessel was immersed in a cold water bath to attain a reaction liquid internal temperature of 15°C. Next, 74 mg (0.32 mmol) of (±)-10-camphorsulfonic acid was added into the reaction vessel. The entire contents of the reaction vessel were then returned to 25°C and were stirred for 12 hours. Once the reaction ended, 400 mL of distilled water and 100 mL of saturated saline water were added to the resultant reaction liquid, and two extractions were performed with 200 mL of ethyl acetate. The organic layers were collected and were dried with anhydrous sodium sulfate, and then sodium sulfate was filtered off. Solvent was removed in a rotary evaporator and then the resultant residue was recrystallized from a mixed solvent of toluene and hexane. Recrystallization of the resultant solid was performed with ethyl acetate as a solvent. Precipitated crystals were collected by filtration. The obtained crystals were washed with cold ethyl acetate and then vacuum dried to yield 2.8 g (yield: 36.4 mol%) of intermediate I in the form of a white solid. The structure of the target was identified by $^1$H-NMR. The results are shown below.

$^1$H-NMR (500 MHz, DMSO-$d_6$, TMS, $\delta$ ppm): 13.1-12.8 (bs, 1H), 7.85-7.81 (m, 1H), 6.95-6.92 (m, 2H), 5.62 (t, 1H, J = 3.5 Hz), 3.73-3.68 (m, 1H), 3.61-3.58 (m, 1H), 1.88-1.51 (m, 6H)

Step 2: Synthesis of intermediate J (another example of compound of formula (VII-1))

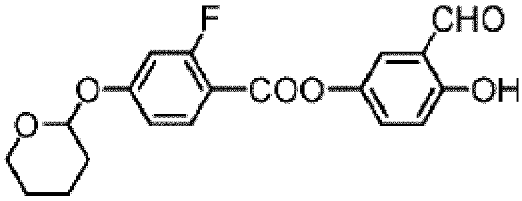

(Intermediate J)

In a three-necked reaction vessel equipped with a thermometer, 2.23 g (9.28 mmol) of the intermediate I synthesized in step 1, 1.28 g (9.28 mmol) of 2,5-dihydroxybenzaldehyde, and 113 mg (0.93 mmol) of N,N-dimethylaminopyridine were added to 80 mL of chloroform in a stream of nitrogen. The reaction vessel was vigorously stirred while 1.41 g (11.14 mmol) of N,N'-diisopropylcarbodiimide was slowly added dropwise at 25°C. Thereafter, the reaction vessel was stirred for 12 hours at 25°C to carry out a reaction. Once the reaction ended, 300 mL of distilled water and 50 mL of saturated saline water were added to the resultant reaction liquid, and two extractions were performed with 200 mL of ethyl acetate. The organic layers were collected and were washed with 500 mL of saturated saline water. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 98:2 (volume ratio)) to yield 2.0 g (yield: 59.8 mol%) of intermediate J in the form of a white solid. The structure of the intermediate J was identified by $^1$H-NMR. The results are shown below.
$^1$H-NMR (500 MHz, $CDCl_3$, TMS, $\delta$ ppm): 10.94 (s, 1H), 9.88 (s, 1H), 8.03 (m, 1H), 7.47 (d, 1H, J = 3.0 Hz), 7.39 (dd, 1H, J = 3.0 Hz, 9.0 Hz), 7.04 (d, 1H, J = 9.0 Hz), 6.95-6.89 (m, 2H), 5.52 (t, 1H, J = 3.0 Hz), 3.84 (dt, 1H, J = 3.0 Hz, 11.0 Hz), 3.68-3.64 (m, 1H), 2.05-1.96 (m, 1H), 1.92-1.88 (m, 2H), 1.78-1.61 (m, 3H)

Step 3: Synthesis of intermediate K (another example of compound of formula (VII-1))

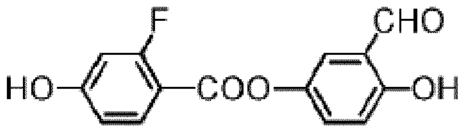

(Intermediate K)

In a three-necked reaction vessel equipped with a thermometer, 2.0 g (5.55 mmol) of the intermediate J synthesized in step 2 was added to 40 mL of a mixed solution of acetic acid/THF/water (= 4/2/1 (mass ratio)) in a stream of nitrogen. The entire contents of the reaction vessel were subsequently heated to 45°C and were stirred for 6 hours. Once the reaction ended, 300 mL of distilled water was added to the resultant reaction liquid, and two extractions were performed with 200 mL of ethyl acetate. The organic layers were collected and were washed twice with 300 mL of distilled water. The resultant organic layer was dried with anhydrous sodium sulfate, and then sodium sulfate was filtered off. Solvent was removed in a rotary evaporator and then the resultant residue was purified by silica gel column chromatography (toluene:ethyl acetate = 85:15 (volume ratio)) to yield 1.2 g (yield: 78 mol%) of intermediate K in the form of a white solid. The structure of the intermediate K was identified by $^1$H-NMR. The results are shown below.
$^1$H-NMR (500 MHz, THF-$d_8$, TMS, $\delta$ ppm): 10.91 (s, 1H), 10.08 (s, 1H), 9.79 (s, 1H), 8.08 (m, 1H), 7.69 (d, 1H, J = 3.0 Hz), 7.51 (dd, 1H, J = 3.0 Hz, 8.5 Hz), 7.10 (d, 1H, J = 8.5 Hz), 6.81 (dd, 1H, J = 2.5 Hz, 8.5 Hz), 6.73 (dd, 1H, J = 2.5 Hz, 12.5 Hz)

Step 4: Synthesis of compound 3

In a four-necked reaction vessel equipped with a thermometer, 1.2 g (4.34 mmol) of the intermediate K synthesized in step 3, 4.0 g (9.56 mmol) of intermediate A synthesized in the same way as in step 1 of Synthesis Example 1, and 117 mg (0.95 mmol) of N,N-dimethylaminopyridine were added to 80 mL of chloroform in a stream of nitrogen. Dropwise addition of 1.26 g (9.98 mmol) of N,N'-diisopropylcarbodiimide to the solution was performed slowly at 25°C. The solution was then stirred for 4 hours at 25°C. Once the reaction ended, the reaction liquid was cooled in an ice bath, and 1.30 g (5.21 mmol) of intermediate E synthesized in the same way as in step 5 of Synthesis Example 1 and 15.8

mL (15.75 mmol) of 1 N hydrochloric acid aqueous solution were added thereto. This solution was allowed to react for 4 hours at 40°C. Once the reaction ended, the solution was added into 200 mL of 3 mass% sodium bicarbonate water, and two extractions were performed with 200 mL of ethyl acetate. The organic layers were collected and were dried with anhydrous sodium sulfate, and then sodium sulfate was filtered off. Solvent was removed in a rotary evaporator and then the resultant residue was purified by silica gel column chromatography (toluene:tetrahydrofuran = 95:5 (volume ratio)) to yield 1.7 g (yield: 30 mol%) of compound 3 in the form of a pale yellow solid. The structure of the target was identified by $^1$H-NMR. The results are shown below.

$^1$H-NMR (500 MHz, $CDCl_3$, TMS, $\delta$ ppm): 8.21 (m, 1H), 7.90 (d, 1H, J = 3.0 Hz), 7.70 (s, 1H), 7.67-7.65 (m, 2H), 7.33 (dd, 1H, J = 1.5 Hz, 8.5 Hz), 7.26-7.23 (m, 1H), 7.18-7.13 (m, 2H), 7.10-7.06 (m, 2H), 7.00-6.97 (m, 4H), 6.90-6.87 (m, 4H), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.30 (t, 2H, J = 7.5 Hz), 4.18 (t, 4H, J = 6.5 Hz), 3.95 (t, 4H, J = 6.5 Hz), 2.73-2.55 (m, 4H), 2.34-2.28 (m, 8H), 1.83-1.31 (m, 32H), 0.91 (t, 3H, J = 7.5 Hz)

(Synthesis Example 4) Synthesis of compound 4

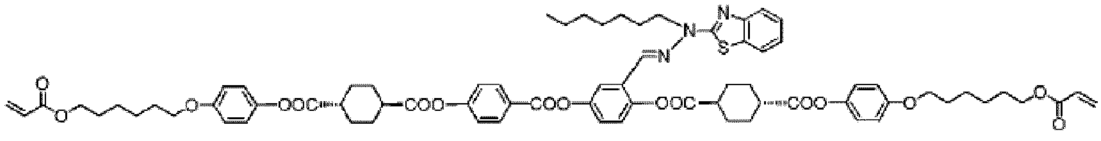

(Compound 4): Formula (XI-4)

Step 1: Synthesis of intermediate L

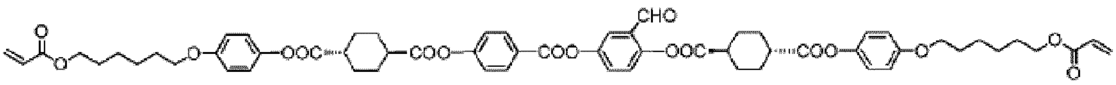

(Intermediate L)

In a four-necked reaction vessel equipped with a thermometer, 10 g (38.73 mmol) of intermediate D synthesized in the same way as in step 4 of Synthesis Example 1, 37.27 g (89.07 mmol) of intermediate A synthesized in the same way as in step 1 of Synthesis Example 1, and 473 mg (3.87 mmol) of N,N-dimethylaminopyridine were added to 400 mL of chloroform in a stream of nitrogen. Dropwise addition of 11.73 g (92.95 mmol) of N,N'-diisopropylcarbodiimide to the solution was performed slowly at 25°C. The solution was then stirred for 4 hours at 25°C. Once the reaction ended, solvent was removed from the reaction liquid in a rotary evaporator and then the resultant residue was purified by silica gel column chromatography (chloroform:tetrahydrofuran = 95:5 (volume ratio)). The resultant white solid was dissolved in 150 mL of THF and was then added dropwise to 1.5 L of methanol. Precipitated solid was filtered, washed with methanol, and subsequently vacuum dried to yield 22 g (yield: 53.7 mol%) of intermediate L in the form of a white solid. The structure of the intermediate L was identified by $^1$H-NMR. The results are shown below.

$^1$H-NMR (500 MHz, $CDCl_3$, TMS, $\delta$ ppm): 10.10 (s, 1H), 8.24 (d, 2H, J = 8.5 Hz), 7.75 (d, 1H, J = 3.0 Hz), 7.52 (dd, 1H, J = 6.0 Hz, 9.0 Hz), 7.27-7.25 (m, 3H), 7.00-6.96 (m, 4H), 6.88-6.87 (d, 4H, J = 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.17 (t, 4H, J = 6.5 Hz), 3.94 (t, 4H, J = 6.5 Hz), 2.78-2.68 (m, 1H), 2.68-2.56 (m, 3H), 2.40-2.25 (m, 8H), 1.84-1.40 (m, 24H)

Step 2: Synthesis of intermediate M

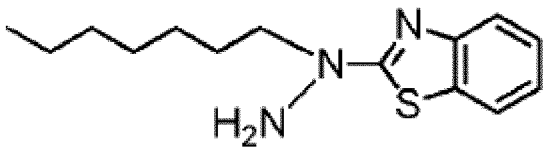

(Intermediate M)

In a four-necked reaction vessel equipped with a thermometer, 2.00 g (12.1 mmol) of 2-hydrazinobenzothiazole was dissolved in 20 mL of dimethylformamide in a stream of nitrogen. Next, 7.88 g (24.2 mmol) of cesium carbonate and 2.60 g (14.5 mmol) of 1-bromoheptane were added to the solution and were stirred therewith for 6 hours at 50°C. Once the reaction ended, the reaction liquid was cooled to 20°C and was added into 200 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:THF = 80:20 (volume ratio)) to yield 2.5 g (yield: 78.4%) of intermediate M in the form of a white solid. The structure of the intermediate M was identified by $^1$H-NMR. The results are shown below.
$^1$H-NMR (500 MHz, $CDCl_3$, TMS, $\delta$ ppm): 7.59 (dd, 1H, J = 1.5 Hz, 8.0 Hz), 7.53 (dd, 1H, J = 1.5 Hz, 8.0 Hz), 7.06-7.28 (m, 2H), 4.22 (s, 2H), 3.75 (t, 2H, J = 7.0 Hz), 1.29-1.38 (m, 10H), 0.88 (t, 3H, J = 7.0 Hz)

Step 3: Synthesis of compound 4

In a four-necked reaction vessel equipped with a thermometer, 2.0 g (1.89 mmol) of the intermediate L synthesized in step 1 and 696 mg (2.64 mmol) of the intermediate M synthesized in step 2 were dissolved in a mixed solvent of 5 mL of ethanol and 40 mL of tetrahydrofuran in a stream of nitrogen. Next, 87.8 mg (0.378 mmol) of (±)-10-camphorsulfonic acid was added to the solution and was stirred therewith for 2 hours at 50°C. Once the reaction ended, the reaction liquid was added into 300 mL of water, and an extraction was performed with 200 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (chloroform:THF = 95:5 (volume ratio)) to yield 1.58 g (yield: 64.1 mol%) of compound 4 in the form of a pale yellow solid. The structure of the target was identified by $^1$H-NMR. The results are shown below.
$^1$H-NMR (500 MHz, $CDCl_3$, TMS, $\delta$ ppm): 8.29 (d, 2H, J = 8.5 Hz), 7.89 (d, 1H, J = 3.0 Hz), 7.70 (s, 1H), 7.66 (m, 2H), 7.34-7.31 (m, 1H), 7.28 (d, 2H, J = 9.0 Hz), 7.23 (dd, 1H, J = 3.0 Hz, 9.0 Hz), 7.17 (d, 1H, J = 9.0 Hz), 7.16-7.13 (m, 1H), 6.99-6.96 (m, 4H), 6.90-6.87 (m, 4H), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.30 (t, 2H, J = 7.5 Hz), 4.18 (t, 4H, J = 6.5 Hz), 3.95 (t, 4H, J = 6.5 Hz), 2.76-2.58 (m, 4H), 2.38-2.28 (m, 8H), 1.81-1.29 (m, 34H), 0.88 (t, 3H, J = 7.0 Hz)

(Synthesis Example 5) Synthesis of compound 5

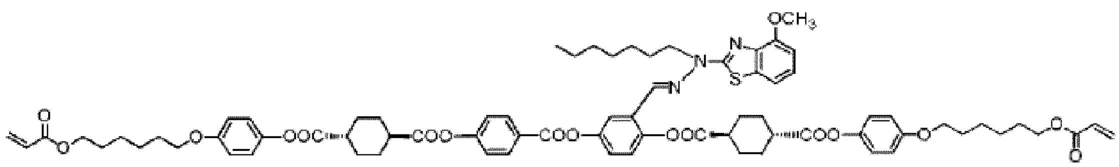

(Compound 5): Formula (XI-5)

Step 1: Synthesis of intermediate N

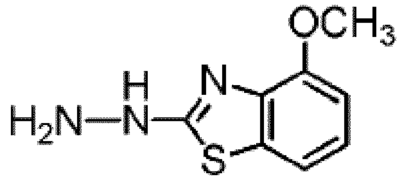

(Intermediate N)

In a four-necked reaction vessel equipped with a thermometer, 4.00 g (22.2 mmol) of 2-aminobenzothiazole was dissolved in 40 mL of ethylene glycol and 15 mL of water in a stream of nitrogen. Next, 11.1 g (222 mmol) of hydrazine monohydrate and 2.8 mL (33.3 mmol) of 12 N hydrochloric acid were added to the solution and were stirred therewith for 15 hours at 120°C. Once the reaction ended, the reaction liquid was cooled to 20°C and was added into 200 mL of 10% sodium bicarbonate water, and an extraction was performed with 800 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was recrystallized from ethyl acetate to yield 2.3 g (yield: 53.1 mol%) of intermediate N. The structure of the intermediate N was identified by $^1$H-NMR. The results are shown below.
$^1$H-NMR (500 MHz, DMSO-$d_6$, TMS, δ ppm): 8.93 (s, 1H), 7.27 (dd, 1H, J = 1.0 Hz, 8.0 Hz), 6.94 (dd, 1H, J = 8.0 Hz, 8.0 Hz), 6.82 (dd, 1H, J = 1.0 Hz, 8.0 Hz), 5.00 (s, 2H), 3.82 (s, 3H)

Step 2: Synthesis of intermediate O

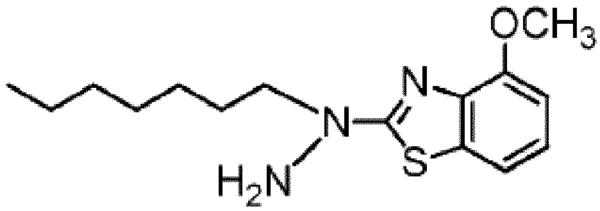

(Intermediate O)

In a four-necked reaction vessel equipped with a thermometer, 2.00 g (10.2 mmol) of the intermediate N synthesized in step 1 was dissolved in 20 mL of dimethylformamide in a stream of nitrogen. Next, 6.68 g (20.4 mmol) of cesium carbonate and 2.19 g (12.2 mmol) of 1-bromoheptane were added to the solution and were stirred therewith for 6 hours at 50°C. Once the reaction ended, the reaction liquid was cooled to 20°C and was added into 200 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:THF = 80:20 (volume ratio)) to yield 2.3 g (yield: 76.8 mol%) of intermediate O in the form of a white solid. The structure of the intermediate O was identified by $^1$H-NMR. The results are shown below.
$^1$H-NMR (500 MHz, $CDCl_3$, TMS, δ ppm): 7.22 (dd, 1H, J = 1.0 Hz, 8.0 Hz), 7.04 (dd, 1H, J = 8.0 Hz, 8.0 Hz), 6.81 (dd, 1H, J = 1.0 Hz, 8.0 Hz), 4.27 (s, 2H), 3.98 (s, 3H), 3.73 (t, 2H, J = 7.5 Hz), 1.76-1.70 (m, 2H), 1.40-1.23 (m, 8H), 0.88 (t, 3H, J = 7.0 Hz)

Step 3: Synthesis of compound 5

In a four-necked reaction vessel equipped with a thermometer, 2.0 g (1.89 mmol) of intermediate L synthesized in the same way as in step 1 of Synthesis Example 4 and 776 mg (2.65 mmol) of the intermediate O synthesized in step 2 were dissolved in a mixed solvent of 5 mL of ethanol and 40 mL of tetrahydrofuran in a stream of nitrogen. Next, 87.8 mg (0.378 mmol) of (±)-10-camphorsulfonic acid was added to the solution and was stirred therewith for 2 hours at 50°C. Once the reaction ended, the reaction liquid was added into 300 mL of water, and an extraction was performed with 200 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (chloroform:THF = 95:5 (volume ratio)) to yield 1.44

g (yield: 57.1 mol%) of compound 5 in the form of a pale yellow solid. The structure of the target was identified by $^1$H-NMR. The results are shown below.

$^1$H-NMR (500 MHz, $CDCl_3$, TMS, δ ppm): 8.29 (d, 2H, J = 9.0 Hz), 7.88 (d, 1H, J = 3.0 Hz), 7.68 (s, 1H), 7.28-7.26 (m, 3H), 7.22 (dd, 1H, J = 3.0 Hz, 9.0 Hz), 7.17 (d, 1H, J = 9.0 Hz), 7.11 (dd, 1H, J = 8.0 Hz, 8.0 Hz), 6.98 (d, 2H, J = 9.0 Hz), 6.98 (d, 2H, J = 9.0 Hz), 6.88 (d, 4H, J = 9.0 Hz), 6.84 (dd, 1H, J = 1.0 Hz, 8.0 Hz), 6.40 (dd, 2H, J = 1.5 Hz, 17.5 Hz), 6.13 (dd, 2H, J = 10.5 Hz, 17.5 Hz), 5.83 (dd, 2H, J = 1.5 Hz, 10.5 Hz), 4.35 (t, 2H, J = 7.5 Hz), 4.18 (t, 4H, J = 6.5 Hz), 4.01 (s, 3H), 3.95 (t, 4H, J = 6.5 Hz), 2.75-2.56 (m, 4H), 2.38-2.27 (m, 8H), 1.86-1.26 (m, 34H), 0.87 (t, 3H, J = 7.0 Hz)

(Synthesis Example 6) Synthesis of compound 6

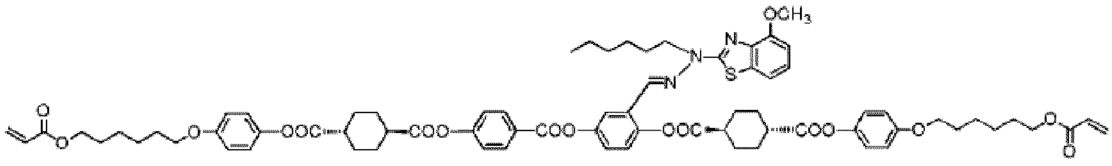

(Compound 6): Formula (XI-6)

Step 1: Synthesis of intermediate P

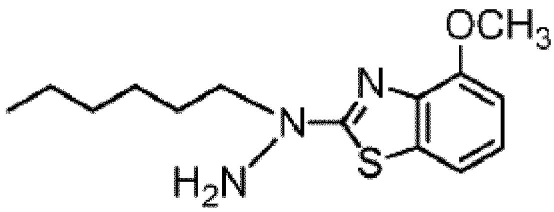

(Intermediate P)

In a four-necked reaction vessel equipped with a thermometer, 2.00 g (10.2 mmol) of intermediate N synthesized in the same way as in step 1 of Synthesis Example 5 was dissolved in 20 mL of dimethylformamide in a stream of nitrogen. Next, 6.68 g (20.4 mmol) of cesium carbonate and 2.0 g (12.2 mmol) of 1-bromohexane were added to the solution and were stirred therewith for 6 hours at 50°C. Once the reaction ended, the reaction liquid was cooled to 20°C and was added into 200 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a yellow solid. The yellow solid was purified by silica gel column chromatography (hexane:THF = 80:20 (volume ratio)) to yield 2.0 g (yield: 70.2 mol%) of intermediate P in the form of a white solid. The structure of the intermediate P was identified by $^1$H-NMR. The results are shown below.

$^1$H-NMR (500 MHz, $CDCl_3$, TMS, δ ppm): 7.22 (dd, 1H, J = 1.0 Hz, 8.0 Hz), 7.04 (dd, 1H, J = 8.0 Hz, 8.0 Hz), 6.81 (dd, 1H, J = 1.0 Hz, 8.0 Hz), 4.26 (s, 2H), 3.98 (s, 3H), 3.73 (t, 2H, J = 7.5 Hz), 1.75-1.69 (m, 2H), 1.41-1.27 (m, 6H), 0.89 (t, 3H, J = 7.0 Hz)

Step 2: Synthesis of compound 6

In a four-necked reaction vessel equipped with a thermometer, 2.0 g (1.89 mmol) of intermediate L synthesized in the same way as in step 1 of Synthesis Example 4 and 740 mg (2.65 mmol) of the intermediate P synthesized in step 1 were dissolved in a mixed solvent of 5 mL of ethanol and 40 mL of tetrahydrofuran in a stream of nitrogen. Next, 87.8 mg (0.378 mmol) of (±)-10-camphorsulfonic acid was added to the solution and was stirred therewith for 2 hours at 50°C. Once the reaction ended, the reaction liquid was added into 300 mL of water, and an extraction was performed with 200 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (chloroform:THF = 95:5 (volume ratio)) to yield 1.55

g (yield: 62.1 mol%) of compound 6 in the form of a pale yellow solid. The structure of the target was identified by $^1$H-NMR. The results are shown below.

$^1$H-NMR (500 MHz, $CDCl_3$, TMS, $\delta$ ppm): 8.29 (d, 2H, J = 9.0 Hz), 7.88 (d, 1H, J = 3.0 Hz), 7.69 (s, 1H), 7.28-7.26 (m, 3H), 7.23 (dd, 1H, J = 3.0 Hz), 7.17 (d, 1H, J = 9.0 Hz), 7.11 (dd, 1H, J = 8.0 Hz, 8.0 Hz), 6.98 (d, 4H, J = 9.0 Hz), 6.884 (d, 2H, J = 9.0 Hz), 6.881 (d, 2H, J = 9.0 Hz), 6.84 (dd, 1H, J = 1.0 Hz, 9.0 Hz), 6.40 (dd, 2H, J = 1.5 Hz, 17.5 Hz), 6.13 (dd, 2H, J = 10.5 Hz, 17.5 Hz), 5.82 (dd, 2H, J = 1.5 Hz, 10.5 Hz), 4.35 (t, 2H, J = 7.5 Hz), 4.18 (t, 4H, J = 6.5 Hz), 4.01 (s, 3H), 3.95 (t, 4H, J = 6.5 Hz), 2.74-2.56 (m, 4H), 2.38-2.27 (m, 8H), 1.83-1.29 (m, 32H), 0.90 (t, 3H, J = 7.0 Hz)

(Synthesis Example 7) Synthesis of compound 7

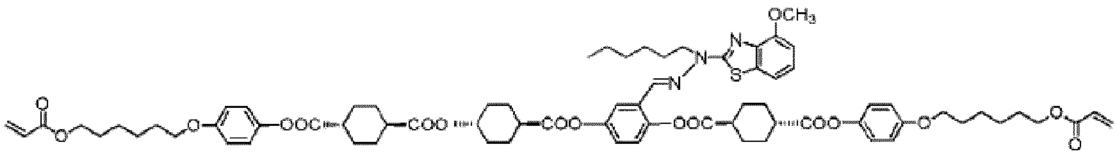

(Compound 7): Formula (XI-7)

Step 1: Synthesis of intermediate Q

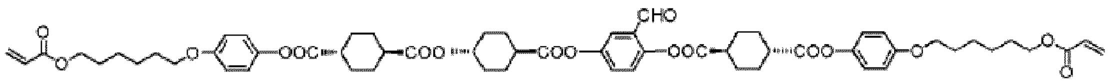

(Intermediate Q)

In a four-necked reaction vessel equipped with a thermometer, 10 g (37.84 mmol) of intermediate G synthesized in the same way as in step 1 of Synthesis Example 2, 36.4 g (87.03 mmol) of intermediate A synthesized in the same way as in step 1 of Synthesis Example 1, and 462 mg (3.78 mmol) of N,N-dimethylaminopyridine were added to 400 mL of chloroform in a stream of nitrogen. Dropwise addition of 11.46 g (90.82 mmol) of N,N'-diisopropylcarbodiimide to the solution was performed slowly at 25°C. The solution was then stirred for 4 hours at 25°C. Once the reaction ended, solvent was removed from the reaction liquid in a rotary evaporator and then the resultant residue was purified by silica gel column chromatography (chloroform:tetrahydrofuran = 95:5 (volume ratio)). The resultant white solid was dissolved in 150 mL of THF and was then added dropwise to 1.5 L of methanol.

Precipitated solid was filtered, washed with methanol, and subsequently vacuum dried to yield 18 g (yield: 44.7 mol%) of intermediate Q in the form of a white solid. The structure of the intermediate Q was identified by $^1$H-NMR. The results are shown below.

$^1$H-NMR (500 MHz, $CDCl_3$, TMS, $\delta$ ppm): 10.07 (s, 1H), 7.60 (d, 1H, J = 3.0 Hz), 7.35 (dd, 1H, J = 3.0 Hz, 9.0 Hz), 7.20 (d, 1H, J = 9.0 Hz), 6.99-6.94 (m, 4H), 6.89-6.86 (m, 4H), 6.40 (dd, 2H, J = 0.5 Hz, 17.5 Hz), 6.12 (dd, 2H, J = 10.5 Hz, 17.5 Hz), 5.82 (dd, 2H, J = 0.5 Hz, 10.5 Hz), 4.78 (dddd, 1H, J = 4.0 Hz, 4.0 Hz, 10.0 Hz, 10.0 Hz), 4.172 (t, 2H, J = 6.5 Hz), 4.171 (t, 2H, J = 6.5 Hz), 3.941 (t, 2H, J = 6.5 Hz), 3.935 (t, 2H, J = 6.5 Hz), 2.76-2.65 (m, 1H), 2.63-2.48 (m, 3H), 2.38-2.05 (m, 13H), 1.83-1.43 (m, 28H)

Step 2: Synthesis of compound 7

In a four-necked reaction vessel equipped with a thermometer, 2.0 g (1.88 mmol) of the intermediate Q synthesized in step 1 and 735 mg (2.63 mmol) of intermediate P synthesized in the same way as in step 1 of Synthesis Example 6 were dissolved in a mixed solvent of 5 mL of ethanol and 40 mL of tetrahydrofuran in a stream of nitrogen. Next, 87.3 mg (0.376 mmol) of (±)-10-camphorsulfonic acid was added to the solution and was stirred therewith for 2 hours at 50°C. Once the reaction ended, the reaction liquid was added into 300 mL of water, and an extraction was performed with 200 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (chloroform:THF = 95:5 (volume ratio)) to yield 1.77 g (yield: 71 mol%) of compound 7 in the form of a pale yellow solid. The structure of the target was identified by $^1$H-NMR. The results are shown below.

$^1$H-NMR (500 MHz, $CDCl_3$, TMS, δ ppm): 7.73 (d, 1H, J = 2.5 Hz), 7.66 (s, 1H), 7.31 (dd, 1H, J = 1.0 Hz, 8.0 Hz), 7.15-7.07 (m, 3H), 6.99-6.95 (m, 4H), 6.90-6.85 (m, 5H), 6.404 (dd, 1H, J = 1.5 Hz, 17.5 Hz), 6.401 (dd, 1H, J = 1.5 Hz, 17.5 Hz), 6.126 (dd, 1H, J = 10.5 Hz, 17.5 Hz), 6.124 (dd, 1H, J = 10.5 Hz, 17.5 Hz), 5.822 (dd, 1H, J = 1.5 Hz, 10.5 Hz), 5.820 (dd, 1H, J = 1.5 Hz, 10.5 Hz), 4.81 (dddd, 1H, J = 4.5 Hz, 4.5 Hz, 10.0 Hz, 10.0 Hz), 4.34 (t, 2H, J = 7.5 Hz), 4.175 (t, 2H, J = 6.5 Hz), 4.172 (t, 2H, J = 6.5 Hz), 4.02 (s, 3H), 3.944 (t, 2H, J = 6.5 Hz), 3.937 (t, 2H, J = 6.5 Hz), 2.72-2.49 (m, 4H), 2.37-2.17 (m, 8H), 2.16-2.08 (m, 4H), 1.83-1.29 (m, 37H), 0.90 (t, 3H, J = 7.0 Hz)

(Comparative Synthesis Example 1) Synthesis of compound X

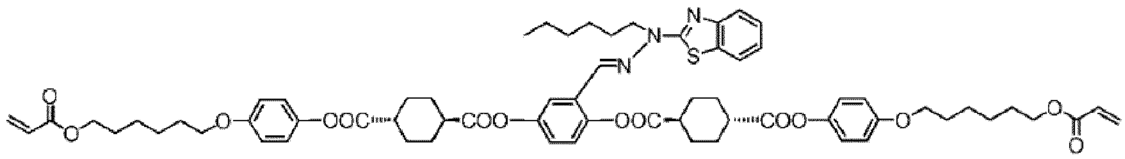

(Compound X)

Step 1: Synthesis of intermediate R

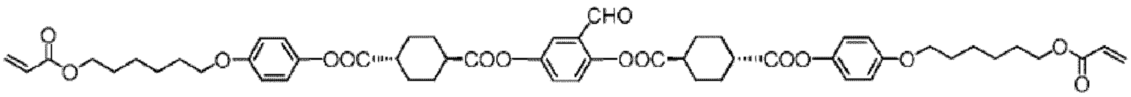

(Intermediate R)

A three-necked reaction vessel equipped with a thermometer was charged with 4.00 g (9.56 mmol) of intermediate A synthesized in the same way as in step 1 of Synthesis Example 1 and 60 mL of tetrahydrofuran in a stream of nitrogen, and a homogeneous solution was obtained. In addition, 1.12 g (9.78 mmol) of methanesulfonyl chloride was added into the reaction vessel and the reaction vessel was immersed in a water bath to attain a reaction liquid internal temperature of 20°C. Next, 1.01 g (9.99 mmol) of triethylamine was added dropwise over 5 minutes while maintaining the reaction liquid internal temperature at 20°C to 30°C. After completion of the dropwise addition, the entire contents of the reaction vessel were further stirred for 2 hours at 25°C. Next, 0.11 g (0.87 mmol) of 4-(dimethylamino)pyridine and 0.60 g (4.35 mmol) of 2,5-dihydroxybenzaldehyde were added to the resultant reaction liquid, and the reaction vessel was immersed in a water bath once again to attain a reaction liquid internal temperature of 15°C. Thereafter, 1.10 g (10.87 mmol) of triethylamine was added dropwise over 5 minutes while maintaining the reaction liquid internal temperature at 20°C to 30°C. After completion of the dropwise addition, the entire contents of the reaction vessel were further stirred for 2 hours at 25°C. Once the reaction ended, 400 mL of distilled water and 50 mL of saturated saline water were added to the reaction liquid, and two extractions were performed with 750 mL of ethyl acetate. The organic layers were collected and were dried with anhydrous sodium sulfate, and then sodium sulfate was filtered off. Solvent was removed from the filtrate by evaporation in a rotary evaporator and then the resultant residue was dissolved in 100 mL of tetrahydrofuran. Crystals were caused to precipitate by adding 500 mL of methanol to the solution and were then collected by filtration. The obtained crystals were washed with methanol and were then vacuum dried to yield 2.51 g (yield: 62 mol%) of intermediate R in the form of a white solid. The structure of the intermediate R was identified by $^1$H-NMR. The results are shown below.
$^1$H-NMR (500 MHz, DMSO-$d_6$, TMS, δ ppm): 10.02 (s, 1H), 7.67 (d, 1H, J = 3.0 Hz), 7.55 (dd, 1H, J = 3.0 Hz, 8.5 Hz), 7.38 (d, 1H, J = 8.5 Hz), 6.99-7.04 (m, 4H), 6.91-6.96 (m, 4H), 6.32 (dd, 2H, J = 1.5 Hz, 17.5 Hz), 6.17 (dd, 2H, J = 10.0 Hz, 17.5 Hz), 5.93 (dd, 2H, J = 1.5 Hz, 10.0 Hz), 4.11 (t, 4H, J = 6.5 Hz), 3.95 (t, 4H, J = 6.5 Hz), 2.56-2.81 (m, 4H), 2.10-2.26 (m, 8H), 1.50-1.76 (m, 16H), 1.33-1.49 (m, 8H)

Step 2: Synthesis of compound X

In a four-necked reaction vessel equipped with a thermometer, 697 mg (2.37 mmol) of intermediate E synthesized in the same way as in step 5 of Synthesis Example 1 and 2.00 g (2.13 mmol) of the intermediate R synthesized in step 1 were dissolved in a mixed solvent of 3 mL of ethanol and 20 mL of tetrahydrofuran in a stream of nitrogen.

Next, 55.1 mg (0.24 mmol) of (±)-10-camphorsulfonic acid was added to the solution and was stirred therewith for 5 hours at 40°C. Once the reaction ended, the reaction liquid was added into 150 mL of water, and an extraction was performed with 300 mL of ethyl acetate. The ethyl acetate layer was dried with anhydrous sodium sulfate. Sodium sulfate was filtered off and then ethyl acetate was evaporated under reduced pressure in a rotary evaporator to yield a white solid. The white solid was purified by silica gel column chromatography (toluene:ethyl acetate = 90:10 (volume ratio)) to yield 2.24 g (yield: 86.4 mol%) of compound X in the form of a white solid. The structure of the target was identified by $^{1}$H-NMR. The results are shown below.

H-NMR (400 MHz, $CDCl_3$, TMS, δ ppm): 7.75 (d, 1H, J = 2.5 Hz), 7.67-7.70 (m, 3H), 7.34 (ddd, 1H, J = 1.0 Hz, 7.0 Hz, 7.5 Hz), 7.17 (ddd, 1H, J = 1.0 Hz, 7.5 Hz, 7.5 Hz), 7.12 (d, 1H, J = 9.0 Hz), 7.10 (dd, 1H, J = 2.5 Hz, 9.0 Hz), 6.99 (d, 2H, J = 9.0 Hz), 6.98 (d, 2H, J = 9.0 Hz), 6.88 (d, 4H, J = 9.0 Hz), 6.40 (dd, 2H, J = 1.5 Hz, 17.0 Hz), 6.13 (dd, 2H, J = 10.5 Hz, 17.5 Hz), 5.82 (dd, 2H, J = 1.5 Hz, 10.5 Hz), 4.30 (t, 2H, J = 8.0 Hz), 4.18 (t, 4H, J = 6.5 Hz), 3.95 (t, 4H, J = 6.5 Hz), 2.58-2.70 (m, 4H), 2.31-2.35 (m, 8H), 1.66-1.82 (m, 18H), 1.31-1.54 (m, 14H), 0.90 (t, 3H, J = 7.0 Hz)

<Measurement of phase transition temperature>

A small amount of each of the compounds 1 to 7 and the compound X was, in a solid state, sandwiched between two glass substrates each provided with a polyimide alignment film that had been subjected to rubbing (produced by E.H.C. Co., Ltd.; product name: Alignment Treatment Glass Substrate). The substrates were placed on a hot plate and were heated from 50°C to 200°C before being allowed to cool back to 50°C. A polarizing optical microscope (ECLIPSE LV100POL produced by Nikon Corporation) was used to observe change in structure during heating and cooling in order to determine phase transition temperatures.

The measured phase transition temperatures are shown below in Table 1.

In Table 1, "C" represents crystal, "N" represents nematic, and "I" represents isotropic. Moreover, "crystal" indicates that the test compound is in a solid phase, "nematic" indicates that the test compound is in a nematic liquid crystal phase, and "isotropic" indicates that the test compound is in an isotropic liquid phase.

Table 1

| Compound number | Phase transition temperature |
|---|---|
| Compound 1 | $C \underset{\leq 50^{\circ}C}{\overset{135^{\circ}C}{\rightleftarrows}} N \xrightarrow{\geq 200^{\circ}C} I$ |
| Compound 2 | $C \underset{\leq 50^{\circ}C}{\overset{168^{\circ}C}{\rightleftarrows}} N \xrightarrow{\geq 200^{\circ}C} I$ |
| Compound 3 | $C \underset{\leq 50^{\circ}C}{\overset{133^{\circ}C}{\rightleftarrows}} N \xrightarrow{\geq 200^{\circ}C} I$ |
| Compound 4 | $C \underset{\leq 50^{\circ}C}{\overset{127^{\circ}C}{\rightleftarrows}} N \xrightarrow{\geq 200^{\circ}C} I$ |
| Compound 5 | $C \underset{\leq 50^{\circ}C}{\overset{147^{\circ}C}{\rightleftarrows}} N \xrightarrow{\geq 200^{\circ}C} I$ |
| Compound 6 | $C \underset{\leq 50^{\circ}C}{\overset{150^{\circ}C}{\rightleftarrows}} N \xrightarrow{\geq 200^{\circ}C} I$ |

(continued)

| Compound number | Phase transition temperature |
|---|---|
| Compound 7 | $C \underset{\leq 50^{\circ}C}{\overset{145^{\circ}C}{\rightleftarrows}} N \xrightarrow{\geq 200^{\circ}C} I$ |
| Compound X | $C \underset{\leq 50^{\circ}C}{\overset{96^{\circ}C}{\rightleftarrows}} N \xrightarrow{\geq 200^{\circ}C} I$ |

(Examples 1 to 7)

For each of the compounds 1 to 7 obtained in Synthesis Examples 1 to 7, a solution was obtained by dissolving 1.0 g of the compound, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in 2.05 g of 1,3-dioxolane and 1.37 g of cyclopentanone. The resultant solution was filtered using a disposable filter having a pore diameter of 0.45 μm. In this manner, polymerizable compositions 1 to 7 were obtained.

(Comparative Example 1)

A solution was obtained by dissolving 1.0 g of the compound X obtained in Comparative Synthesis Example 1, 43 mg of a photoinitiator ADEKA ARKLS N1919T (produced by ADEKA Corporation), and 300 mg of a mixed solvent of cyclopentanone and 1,3-dioxolane (mixing ratio (mass ratio): cyclopentanone/1,3-dioxolane = 4/6) containing 1 mass% of a surfactant MEGAFACE F-562 (produced by DIC Corporation) in 2.05 g of 1,3-dioxolane and 1.37 g of cyclopentanone. The resultant solution was filtered using a disposable filter having a pore diameter of 0.45 μm to obtain a polymerizable composition 1r.

<Measurement of retardation and evaluation of wavelength dispersion>

(i) Formation of liquid crystal layer by polymerizable composition

Each of the polymerizable compositions 1 to 7 and 1r was applied onto a transparent glass substrate provided with a polyimide alignment film that had been subjected to rubbing (product name: Alignment Treatment Glass Substrate; produced by E.H.C. Co., Ltd.) using a #4 wire bar so as to obtain an applied film. The applied film that was obtained was dried for 1 minute at a temperature indicated below in Table 2 and was then subjected to alignment treatment for 1 minute at a temperature indicated in Table 2 so as to form a liquid crystal layer. Thereafter, irradiation with 2000 $mJ/cm^2$ of ultraviolet rays was performed from the application surface side of the liquid crystal layer at a photoexposure temperature indicated in Table 2 so as to cause polymerization and thereby obtain an optically anisotropic body equipped with a transparent glass substrate as a sample for wavelength dispersion measurement. The film thickness of the optically anisotropic body was measured by using a needle to form a scratch in the optically anisotropic body of the transparent glass substrate-equipped optically anisotropic body, and then measuring a step at the scratch using a surface profiler Dektak 150 (produced by ULVAC, Inc.).

(ii) Measurement of retardation

A Mueller Matrix Polarimeter AxoScan (produced by Axometrics, Inc.) was used to measure retardation at wavelengths from 400 nm to 800 nm for each of the obtained samples.

(iii) Evaluation of wavelength dispersion

Wavelength dispersion was evaluated based on wavelength dispersion ratios calculated as follows using the measured retardation. The results are shown in Table 2.

Wavelength dispersion ratio at 400 nm: α value = (Retardation at 400 nm)/(Retardation at 550 nm)
Wavelength dispersion ratio at 410 nm: β value = (Retardation at 410 nm)/(Retardation at 550 nm)
Wavelength dispersion ratio at 420 nm: γ value = (Retardation at 420 nm)/(Retardation at 550 nm)
Wavelength dispersion ratio at 430 nm: δ value = (Retardation at 430 nm)/(Retardation at 550 nm)
Wavelength dispersion ratio at 440 nm = (Retardation at 440 nm)/(Retardation at 550 nm)
Wavelength dispersion ratio at 450 nm = (Retardation at 450 nm)/(Retardation at 550 nm)

Table 2

| | Polymerizable composition | Polymerizable compound | Drying temperature (°C) | Alignment treatment temperature (°C) | Temperature during photo exposure (°C) | Film thickness (μm) | Retardation at 550 nm (nm) | Wavelength dispersion ratio | | | | | |
|---|---|---|---|---|---|---|---|---|---|---|---|---|---|
| | | Used compound | | | | | | 400 nm | 410 nm | 420 nm | 430 nm | 440 nm | 450 nm |
| Example 1 | 1 | Compound 1 | 145 | 23 | 23 | 1.85 | 159.92 | 0.5057 | 0.6769 | 0.7731 | 0.8435 | 0.8753 | 0.8855 |
| Example 2 | 2 | Compound 2 | 175 | 23 | 23 | 1.43 | 96.03 | 0.3597 | 0.5730 | 0.6981 | 0.7631 | 0.8218 | 0.8154 |
| Example 3 | 3 | Compound 3 | 140 | 23 | 23 | 2.18 | 190.18 | 0.5141 | 0.6787 | 0.7745 | 0.8509 | 0.8823 | 0.9164 |
| Example 4 | 4 | Compound 4 | 140 | 23 | 23 | 2.27 | 189.00 | 0.4766 | 0.6486 | 0.7637 | 0.8336 | 0.8789 | 0.9073 |
| Example 5 | 5 | Compound 5 | 155 | 23 | 23 | 2.14 | 178.12 | 0.3254 | 0.4051 | 0.5919 | 0.6971 | 0.7703 | 0.8413 |
| Example 6 | 6 | Compound 6 | 160 | 23 | 23 | 2.09 | 175.61 | 0.2672 | 0.4064 | 0.5959 | 0.7066 | 0.7857 | 0.8452 |
| Example 7 | 7 | Compound 7 | 150 | 23 | 23 | 2.05 | 129.12 | 0.1531 | 0.4158 | 0.6021 | 0.7071 | 0.7744 | 0.8225 |
| Comparative Example 1 | 1r | Compound X | 110 | 23 | 23 | 1.92 | 141.88 | 0.0914 | 0.3787 | 0.5654 | 0.6711 | 0.7299 | 0.8088 |
| Ideal value | | | | | | | | 0.7273 | 0.7455 | 0.7636 | 0.7818 | 0.8000 | 0.8182 |

It can be seen from Table 2 that improvement was observed in Examples 1 to 7 (i.e., for optically anisotropic bodies formed using the polymerizable compositions 1 to 7 containing the compounds 1 to 7) in terms that deviation from the ideal values for wavelength dispersion ratios at short wavelengths (400 nm to 440 nm) decreased. In particular, wavelength dispersion characteristics noticeably improved at wavelengths from 400 nm to 430 nm.

INDUSTRIAL APPLICABILITY

The present disclosure provides a polymerizable compound that is useful in production of a polymerizable composition capable of forming an optical film or optically anisotropic body having good wavelength dispersion characteristics at short wavelengths.

Moreover, the present disclosure provides a polymerizable composition that is capable of forming an optical film or optically anisotropic body having good wavelength dispersion characteristics at short wavelengths.

Furthermore, the present disclosure provides a compound that is useful in production of the aforementioned polymerizable compound and in an optical film.

Also, the present disclosure provides an optical film and an optically anisotropic body having good wavelength dispersion characteristics at short wavelengths, and also a polarizer, a flat panel display, an organic electroluminescence (EL) display, and an antireflection film in which the optical film and optically anisotropic body are used.

## Claims

**1.** A polymerizable compound indicated by formula (I), shown below,

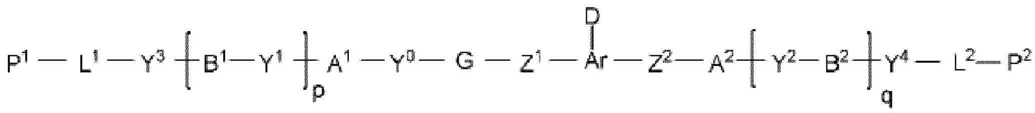

(I)

where, in formula (I):

Ar represents an optionally substituted aromatic hydrocarbon cyclic group or an optionally substituted aromatic heterocyclic group;

D represents an organic group having a carbon number of 1 to 67 and including at least one aromatic ring selected from the group consisting of an aromatic hydrocarbon ring and an aromatic heterocyclic ring;

$Y^0$, $Z^1$, and $Z^2$ each represent, independently of one another, a single bond, -O-, $-O-CH_2-$, $-CH_2-O-$, $-O-CH_2-CH_2-$, $-CH_2-CH_2-O-$, -C(=O)-O-, -O-C(=O)-, -C(=O)-S-, -S-C(=O)-, $-NR^{10}-C(=O)-$, $-C(=O)-NR^{10}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, or -C≡C-, where $R^{10}$ represents a hydrogen atom or an alkyl group having a carbon number of 1 to 6;

G represents an optionally substituted alicyclic group, an optionally substituted aromatic group, or an optionally substituted alkylene group;

$L^1$ and $L^2$ are each, independently of one another, an organic group that is either an alkylene group having a carbon number of 1 to 20 or a group in which at least one methylene group ($-CH_2-$) of an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-;

$A^1$, $A^2$, $B^1$, and $B^2$ each represent, independently of one another, an optionally substituted alicyclic group or an optionally substituted aromatic group;

$Y^1$ to $Y^4$ each represent, independently of one another, a single bond, -O-, -C(=O)-, -C(=O)-O-, -O-C(=O)-, $-NR^{11}-C(=O)-$, $-C(=O)-NR^{11}-$, -O-C(=O)-O-, $-NR^{11}-C(=O)-O-$, $-O-C(=O)-NR^{11}-$, or $-NR^{11}-C(=O)-NR^{12}-$, where $R^{11}$ and $R^{12}$ each represent, independently of one another, a hydrogen atom or an alkyl group having a carbon number of 1 to 6;

one of $P^1$ and $P^2$ represents a hydrogen atom or a polymerizable group and the other of $P^1$ and $P^2$ represents a polymerizable group; and

p and q are each, independently of one another, an integer of 0 to 2, with a proviso that cases in which p = 0 and q = 1 and in which p = 1 and q = 2 are excluded.

**2.** The polymerizable compound according to claim 1, wherein Ar-D is indicated by any one of formulae (II-1) to (II-6), shown below,

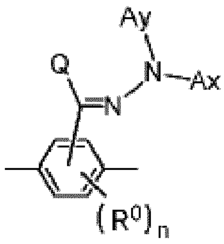
(II-1)

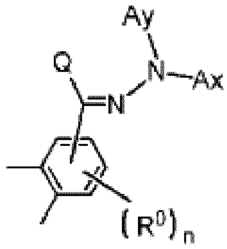
(II-2)

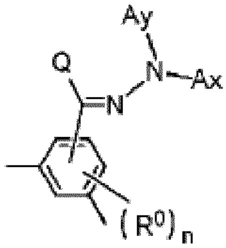
(II-3)

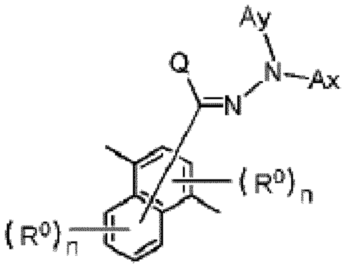
(II-4)

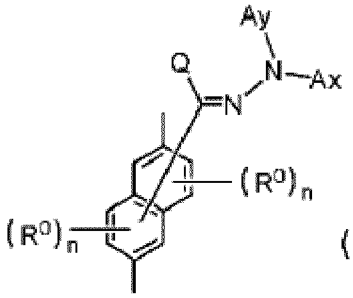
(II-5)

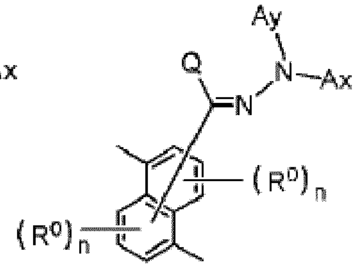
(II-6)

where, in formulae (II-1) to (II-6):

Ax represents an organic group including at least one aromatic ring selected from the group consisting of an aromatic hydrocarbon ring having a carbon number of 6 to 30 and an aromatic heterocyclic ring having a carbon number of 2 to 30, where the aromatic ring of Ax is optionally substituted;
Ay represents a hydrogen atom or an optionally substituted organic group having a carbon number of 1 to 30;
Q represents a hydrogen atom or an alkyl group having a carbon number of 1 to 6; and
$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, -C(=O)-$R^a$, -O-C(=O)-$R^a$, -C(=O)-O-$R^a$, or -$SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4.

**3.** The polymerizable compound according to claim 2, wherein the polymerizable compound is indicated by formula (III-1) or (III-2), shown below,

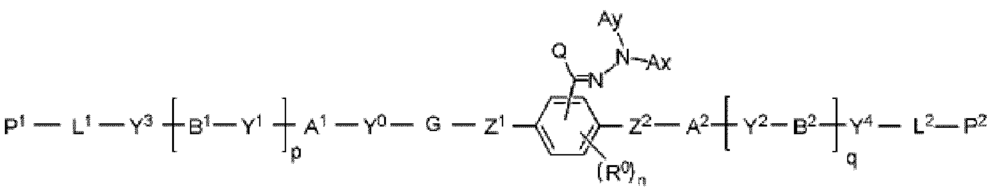
(III-1)

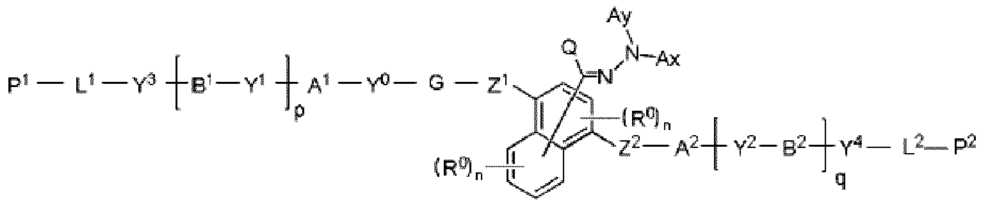

(III-2)

where, in formulae (III-1) and (III-2), $P^1$, $P^2$, $L^1$, $L^2$, $A^1$, $A^2$, $B^1$, $B^2$, $Y^0$ to $Y^4$, G, $Z^1$, $Z^2$, $R^0$, n, p, q, Ax, Ay, and Q have the same meaning as previously described.

**4.** The polymerizable compound according to claim 2 or 3, wherein Ay is a hydrogen atom, an optionally substituted alkyl group having a carbon number of 1 to 20, an optionally substituted alkenyl group having a carbon number of 2 to 20, an optionally substituted alkynyl group having a carbon number of 2 to 20, an optionally substituted cycloalkyl group having a carbon number of 3 to 12, an optionally substituted aromatic hydrocarbon cyclic group having a carbon number of 6 to 30, or an optionally substituted aromatic heterocyclic group having a carbon number of 2 to 30.

**5.** The polymerizable compound according to any one of claims 2 to 4, wherein Ax is indicated by formula (V), shown below,

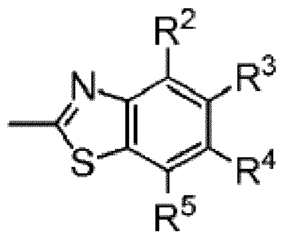

(V)

where, in formula (V), $R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, $C-R^2$ to $C-R^5$ may be the same or different, and one or more of ring constituents $C-R^2$ to $C-R^5$ may be replaced by a nitrogen atom.

**6.** The polymerizable compound according to any one of claims 1 to 5, wherein $P^1$ and $P^2$ are each, independently of one another, indicated by formula (IV), shown below,

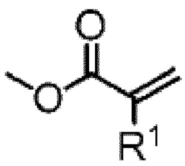

(IV)

where, in formula (IV), $R^1$ represents a hydrogen atom, a methyl group, or a chlorine atom.

**7.** The polymerizable compound according to any one of claims 1 to 6, wherein the polymerizable compound is indicated by formula (VI-1) or (VI-2), shown below,

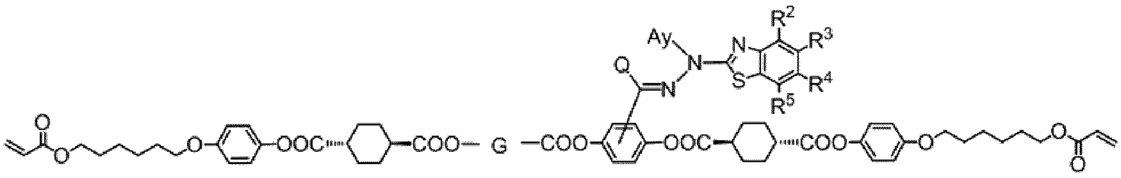

(VI-1)

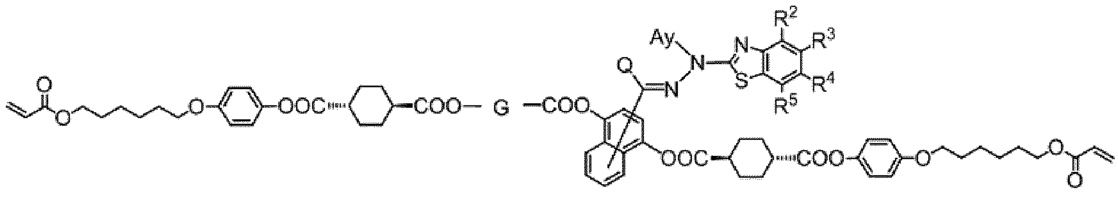

(VI-2)

where, in formulae (VI-1) and (VI-2):

$R^2$ to $R^5$ each represent, independently of one another, a hydrogen atom, a halogen atom, an alkyl group having a carbon number of 1 to 6, a cyano group, a nitro group, a fluoroalkyl group having a carbon number of 1 to 6, an alkoxy group having a carbon number of 1 to 6, $-OCF_3$, $-O-C(=O)-R^b$, or $-C(=O)-O-R^b$, where $R^b$ represents an optionally substituted alkyl group having a carbon number of 1 to 20, an optionally substituted alkenyl group having a carbon number of 2 to 20, an optionally substituted cycloalkyl group having a carbon number of 3 to 12, or an optionally substituted aromatic hydrocarbon cyclic group having a carbon number of 5 to 18, $C-R^2$ to $C-R^5$ may be the same or different, and one or more of ring constituents $C-R^2$ to $C-R^5$ may be replaced by a nitrogen atom;

Ay represents a hydrogen atom or an optionally substituted organic group having a carbon number of 1 to 30;

Q represents a hydrogen atom or an alkyl group having a carbon number of 1 to 6; and

G represents an optionally substituted alicyclic group, an optionally substituted aromatic group, or an optionally substituted alkylene group.

**8.** A polymerizable composition comprising:

the polymerizable compound according to any one of claims 1 to 7; and
a polymerization initiator.

**9.** A polymer obtained by polymerizing the polymerizable compound according to any one of claims 1 to 7.

**10.** An optical film comprising the polymer according to claim 9.

**11.** An optical film comprising the polymerizable compound according to any one of claims 1 to 7.

**12.** An optically anisotropic body comprising a layer containing the polymer according to claim 9.

**13.** A polarizer comprising:

the optically anisotropic body according to claim 12; and
a polarizing film.

**14.** A flat panel display comprising:

the polarizer according to claim 13; and
a liquid crystal panel.

**15.** An organic electroluminescence display comprising:

the polarizer according to claim 13; and

an organic electroluminescence panel.

**16.** An antireflection film comprising the polarizer according to claim 13.

**17.** A compound indicated by formula (VII-1) or (VII-2), shown below,

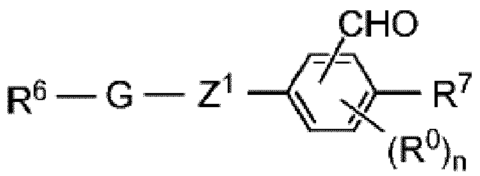

(VII-1)

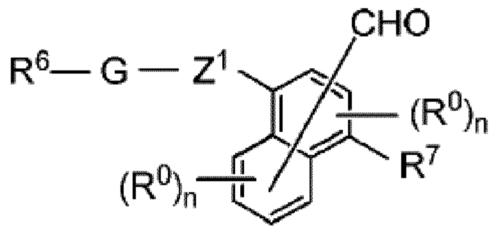

(VII-2)

where, in formulae (VII-1) and (VII-2):

$Z^1$ represents a single bond, -O-, $-O-CH_2-$, $-CH_2-O-$, $-O-CH_2-CH_2-$, $-CH_2-CH_2-O-$, -C(=O)-O-, -O-C(=O)-, -C(=O)-S-, -S-C(=O)-, $-NR^{10}-C(=O)-$, $-C(=O)-NR^{10}--$, $-CF_2-O-$, $-O-CF_2--CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, or -C≡C-, where $R^{10}$ represents a hydrogen atom or an alkyl group having a carbon number of 1 to 6;

G represents an optionally substituted alicyclic group, an optionally substituted aromatic group, or an optionally substituted alkylene group;

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4; and

$R^6$ and $R^7$ each represent, independently of one another, $-OR^e$, $-CH_2OR^e$, $-CH_2CH_2OR^e$, $-C(=O)-OR^e$, $-CH_2-C(=O)-OR^e$, $-CH_2CH_2-C(=O)-OR^e$, a hydroxy group, a carboxyl group, $-CH_2-C(=O)-OH$, $-CH_2CH_2-C(=O)-OH$, $-CH_2OH$, $-CH_2CH_2OH$, or an amino group, where $R^e$ represents a protecting group.

**18.** A compound indicated by formula (VIII-1) or (VIII-2), shown below,

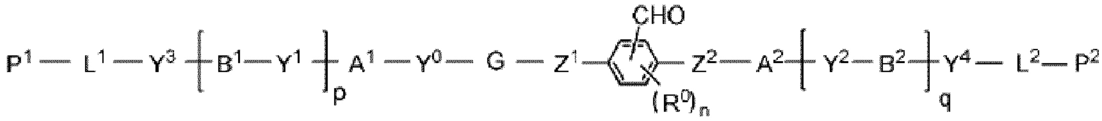

(VIII-1)

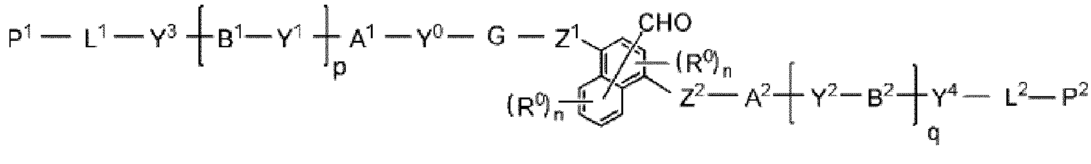

(VIII-2)

where, in formulae (VIII-1) and (VIII-2):

$Y^0$, $Z^1$, and $Z^2$ each represent, independently of one another, a single bond, -O-, $-O-CH_2-$, $-CH_2-O-$, $-O-CH_2-CH_2-$,

$-CH_2-CH_2-O--C(=O)-O-$, -O-C(=O)-, -C(=O)-S-, -S-C(=O)-, $-NR^{10}-C(=O)-$, $-C(=O)-NR^{10}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, $-CH_2-C(=O)-O-$, $-O-C(=O)-CH_2-$, $-CH_2-O-C(=O)-$, $-C(=O)-O-CH_2-$, or -C≡C-, where $R^{10}$ represents a hydrogen atom or an alkyl group having a carbon number of 1 to 6;

G represents an optionally substituted alicyclic group, an optionally substituted aromatic group, or an optionally substituted alkylene group;

$R^0$ represents a halogen atom, a cyano group, an alkyl group having a carbon number of 1 to 6, an alkenyl group having a carbon number of 2 to 6, a haloalkyl group having a carbon number of 1 to 6, an N,N-dialkylamino group having a carbon number of 2 to 12, an alkoxy group having a carbon number of 1 to 6, a nitro group, $-C(=O)-R^a$, $-C(=O)-O-R^a$, or $-SO_2R^a$, where $R^a$ represents an alkyl group having a carbon number of 1 to 6 or an aromatic hydrocarbon cyclic group having a carbon number of 6 to 20 that is optionally substituted with an alkyl group having a carbon number of 1 to 6 or an alkoxy group having a carbon number of 1 to 6, and each n is independently an integer of 0 to 4;

$L^1$ and $L^2$ are each, independently of one another, an organic group that is either an alkylene group having a carbon number of 1 to 20 or a group in which at least one methylene group ($-CH_2-$) of an alkylene group having a carbon number of 1 to 20 is replaced by -O- or -C(=O)-, where hydrogen atoms included in the organic groups of $L^1$ and $L^2$ may each be replaced by an alkyl group having a carbon number of 1 to 5, an alkoxy group having a carbon number of 1 to 5, or a halogen atom, and with a proviso that methylene groups ($-CH_2-$) at both ends of $L^1$ and $L^2$ are not replaced by -O- or -C(=O)-;

$A^1$, $A^2$, $B^1$, and $B^2$ each represent, independently of one another, an optionally substituted alicyclic group or an optionally substituted aromatic group;

$Y^1$ to $Y^4$ each represent, independently of one another, a single bond, -O-, -C(=O)-, -C(=O)-O-, -O-C(=O)-, $-NR^{11}-C(=O)-$, $-C(=O)-NR^{11}-$, -O-C(=O)-O-, $-NR^{11}-C(=O)-O-$, $-O-C(=O)-NR^{11}-$, or $-NR^{11}-C(=O)-NR^{12}-$, where $R^{11}$ and $R^{12}$ each represent, independently of one another, a hydrogen atom or an alkyl group having a carbon number of 1 to 6;

one of $P^1$ and $P^2$ represents a hydrogen atom or a polymerizable group and the other of $P^1$ and $P^2$ represents a polymerizable group; and

p and q are each, independently of one another, an integer of 0 to 2, with a proviso that cases in which p = 0 and q = 1 and in which p = 1 and q = 2 are excluded.

**19.** A compound indicated by formula (X-1) or (X-2), shown below,

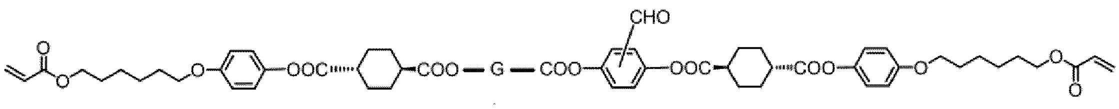

(X-1)

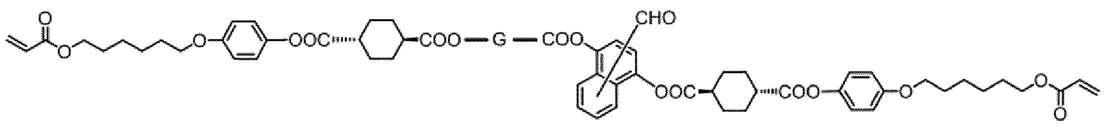

(X-2)

where, in formulae (X-1) and (X-2), G represents an optionally substituted alicyclic group, an optionally substituted aromatic group, or an optionally substituted alkylene group.

**20.** A compound indicated by any one of formulae (XI-1) to (XI-7), shown below.

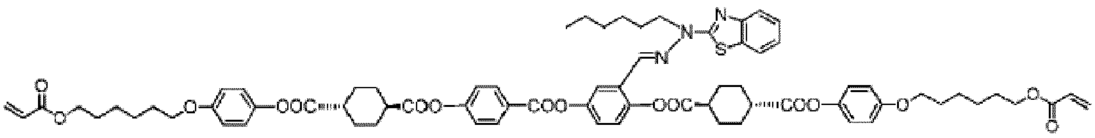

(XI-1)

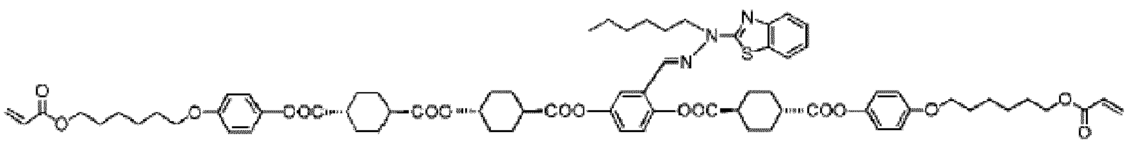

(XI-2)

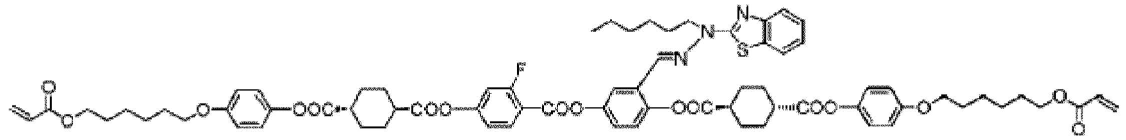

(XI-3)

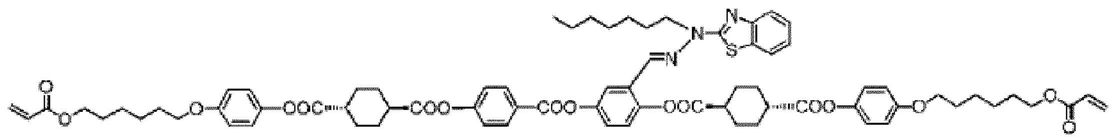

(XI-4)

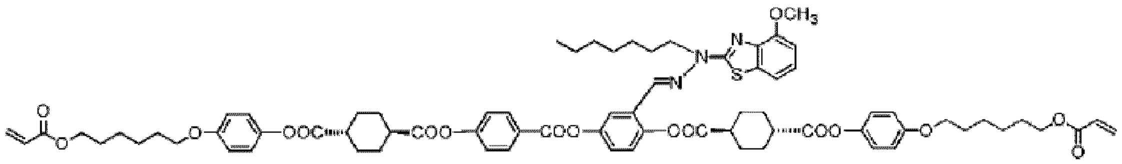

(XI-5)

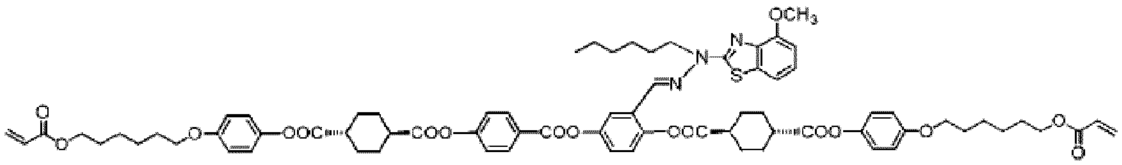

(XI-6)

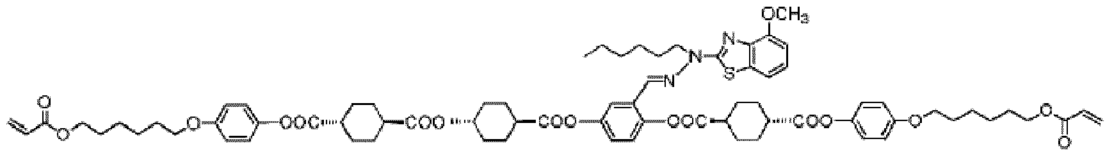

(XI-7)

## INTERNATIONAL SEARCH REPORT

International application No. PCT/JP2017/040466

A. CLASSIFICATION OF SUBJECT MATTER
Int.Cl. C07C69/76(2006.01)i, C07C69/75(2006.01)i, C07D277/82(2006.01)i, C08F20/38(2006.01)i, G02B1/111(2015.01)i, G02B5/30(2006.01)i, G02F1/13363(2006.01)i, H01L51/50(2006.01)i, H05B33/02(2006.01)i

According to International Patent Classification (IPC) or to both national classification and IPC

B. FIELDS SEARCHED

Minimum documentation searched (classification system followed by classification symbols)
Int.Cl. C07C69/76, C07C69/75, C07D277/82, C08F20/38, G02B1/111, G02B5/30, G02F1/13363, H01L51/50, H05B33/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | JP 2016-053709 A (FUJIFILM CORP.) 14 April 2016, claims 8-19, page 14-19, etc. & US 2015/0277006 A1 claims 8-19, page 7-12, etc. & CN 104950371 A & KR 10-2015-0113886 A | 1-16, 20 |
| X | JP 2016-012031 A (NIPPON ZEON CO., LTD.) 21 January 2016, paragraph [0087], preparation example 5, claims 7-10, etc. (Family: none) | 1-16, 20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February 2018 (01.02.2018) | 13 February 2018 (13.02.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/040466

C (Continuation). DOCUMENTS CONSIDERED TO BE RELEVANT

| Category* | Citation of document, with indication, where appropriate, of the relevant passages | Relevant to claim No. |
|---|---|---|
| X | WO 2013/046781 A1 (NIPPON ZEON CO., LTD.) 04 April 2013, paragraph [0006], respective preparation examples, claim 1, etc. & US 2014/0235857 A1, paragraph [0010], respective preparation examples, claim 1, etc. & EP 2762465 A1 & CN 103842334 A & KR 10-2014-0068960 A | 1-16, 20 |
| X | WO 2015/064581 A1 (NIPPON ZEON CO., LTD.) 07 May 2015, paragraph [0060], preparation example 5, claims 1-16, etc. & US 2016/0245972 A1, paragraph [0156], preparation example 5, claims 1-16, etc. & JP 2015-111257 A & EP 3064969 A1 & CN 105659122 A & KR 10-2016-0078356 A | 1-16, 20 |
| X | WO 2016/114066 A1 (DIC CORPORATION) 21 July 2016, paragraph [0007], pp. 34-99, claims 1-16, etc. & CN 107108770 A & KR 10-2017-0104992 A | 1-16, 20 |
| X | WO 2016/114346 A1 (DIC CORPORATION) 21 July 2016, paragraph [0013], pp. 33-100, claims 1-17, etc. & CN 107209309 A & KR 10-2017-0105016 A | 1-16, 20 |
| X | WO 2016/114252 A1 (DIC CORPORATION) 21 July 2016, paragraph [0011], pp. 33-100, claims 1-16, etc. & CN 107207652 A & KR 10-2017-0105041 A | 1-16, 20 |
| X | WO 2016/114253 A1 (DIC CORPORATION) 21 July 2016, paragraph [0012], pp. 33-100, claims 1-17, etc. & CN 107108775 A & KR 10-2017-0105015 A | 1-16, 20 |
| X | WO 2016/114254 A1 (DIC CORPORATION) 21 July 2016, paragraph [0015], pp. 34-100, claims 1-13, etc. & CN 107209308 A & KR 10-2017-0105042 A | 1-16, 20 |
| X | WO 2016/114347 A1 (DIC CORPORATION) 21 July 2016, paragraph [0014], pp. 32-53, 93-161, claims 1-22, etc. & CN 107209307 A & KR 10-2017-0105012 A | 1-16, 20 |
| X | WO 2016/114348 A1 (DIC CORPORATION) 21 July 2016, paragraph [0007], pp. 29-54, 93-162, claims 1-24, etc. (Family: none) | 1-16, 20 |
| X | WO 2016/121602 A1 (NIPPON ZEON CO., LTD.) 04 August 2016, paragraph [0076], preparation example 6, claims 1-13, etc. & CN 107111044 A & KR 10-2017-0105496 A | 1-16, 20 |

Form PCT/ISA/210 (continuation of second sheet) (January 2015)

INTERNATIONAL SEARCH REPORT

International application No. PCT/JP2017/040466

C (Continuation). DOCUMENTS CONSIDERED TO BE RELEVANT

| Category* | Citation of document, with indication, where appropriate, of the relevant passages | Relevant to claim No. |
|---|---|---|
| X | WO 2016/136901 A1 (NIPPON ZEON CO., LTD.) 01 September 2016, paragraph [0070], example 1, claims 1-11, etc. & CN 107209315 A & TW 201640144 A | 1-16, 20 |
| X | WO 2016/171169 A1 (NIPPON ZEON CO., LTD.) 27 October 2016, paragraph [0151], preparation example 5, claims 1-18, etc. & TW 201718245 A | 1-16, 20 |
| X | WO 2016/171041 A1 (NIPPON ZEON CO., LTD.) 27 October 2016, paragraphs [0076], [0173], pp. 85-86, claim 7, etc. & TW 201641556 A | 1-20 |
| X | JP 2015-200877 A (FUJIFILM CORP.) 12 November 2015, claims 1-17, pp. 17-30, examples, etc. & US 2015/0277007 A1, claims 1-17, pp. 8-26, examples, etc. & CN 104950373 A & KR 10-2015-0113855 A | 1-20 |
| X | JP 2016-098258 A (NIPPON ZEON CO., LTD.) 30 May 2016, claims 1-11, examples, etc. (Family: none) | 1-20 |
| X | JP 2016-081035 A (FUJIFILM CORP.) 16 May 2016, claims 1-19, pp. 28-32, examples, etc. & US 2016/0108315 A1, claims 1-19, pp. 9-14, examples, etc. & CN 105524625 A | 1-20 |
| X | JP 2016-113583 A (DIC CORPORATION) 23 June 2016, claims 1-9, pp. 25-41, examples, etc. (Family: none) | 1-20 |
| X | JP 2016-128403 A (JNC CORPORATION) 14 July 2016, claims 1-21, pp. 31-43, examples, etc. (Family: none) | 1-20 |
| X | JP 2016-190828 A (NIPPON ZEON CO., LTD.) 10 November 2016, claims 1-5, page 5, examples, etc. (Family: none) | 1-20 |

Form PCT/ISA/210 (continuation of second sheet) (January 2015)

INTERNATIONAL SEARCH REPORT

International application No.
PCT/JP2017/040466

C (Continuation). DOCUMENTS CONSIDERED TO BE RELEVANT

| Category* | Citation of document, with indication, where appropriate, of the relevant passages | Relevant to claim No. |
|---|---|---|
| X | WO 2016/056542 A1 (DIC CORPORATION) 14 April 2016, paragraph [0007], pp. 34-64, examples, claims 1-11, etc. & US 2017/0306233 A1, pp. 24-69, examples, claims 1-11, etc. & KR 10-2017-0068529 A | 1-20 |
| X | WO 2012/147904 A1 (NIPPON ZEON CO., LTD.) 01 November 2012, paragraphs [0010], [0090], examples, claims 1-14, etc. & US 2014/0142266 A1, paragraphs [0035], [0104], examples, claims 1-14, etc. & JP 2017-32987 A & EP 2703385 A1 & CN 103492363 A & KR 10-2014-0020296 A & CN 106278943 A | 1-20 |
| X | WO 2014/010325 A1 (NIPPON ZEON CO., LTD.) 16 January 2014, paragraphs [0010], [0117], examples, claims 1-18, etc. & US 2015/0175564 A1, paragraphs [0011], [0093], examples, claims 1-18, etc. & JP 2016-84349 A & JP 2017-167553 A & EP 2871192 A1 & KR 10-2015-0036047 A & CN 104603165 A & CN 107253935 A | 1-20 |
| X | WO 2014/065176 A1 (NIPPON ZEON CO., LTD.) 01 May 2014, paragraphs [0010], [0076], examples, claims 1-12, etc. & US 2015/0274872 A1, paragraphs [0012], [0082], examples, claims 1-12, etc. & EP 2913349 A1 & CN 104755513 A & KR 10-2015-0079579 A | 1-20 |
| X | WO 2014/065243 A1 (NIPPON ZEON CO., LTD.) 01 May 2014, pp. 4, 29-30, examples, claims 1-11, etc. & US 2015/0277010 A1, pp. 2, 12, examples, claims 1-11, etc. & EP 2910986 A1 & CN 104737044 A & KR 10-2015-0073177 A | 1-20 |
| X | WO 2014/132978 A1 (FUJIFILM CORP.) 04 September 2014, paragraphs [0060], [0125], examples, claims 1-16, etc. & TW 201439612 A | 1-16, 20 |
| X | WO 2015/025793 A1 (NIPPON ZEON CO., LTD.) 26 February 2015, paragraphs [0010], [0097], examples, claims 1-12, etc. & US 2016/0200841 A1, paragraphs [0034], [0105], examples, claims 1-12, etc. & EP 3037444 A1 & CN 105452311 A & KR 10-2016-0048816 A | 1-20 |
| X | WO 2015/064698 A1 (NIPPON ZEON CO., LTD.) 07 May 2015, paragraph [0011], pp. 21, 24, examples, claims 1-12, etc. & US 2016/0257659 A1, paragraph [0015], pp 8-10, examples, claims 1-12, etc. & EP 3064516 A1 & CN 105745233 A & KR 10-2016-0084397 A | 1-20 |

Form PCT/ISA/210 (continuation of second sheet) (January 2015)

INTERNATIONAL SEARCH REPORT

International application No.
PCT/JP2017/040466

C (Continuation). DOCUMENTS CONSIDERED TO BE RELEVANT

| Category* | Citation of document, with indication, where appropriate, of the relevant passages | Relevant to claim No. |
|---|---|---|
| X | WO 2015/141784 A1 (NIPPON ZEON CO., LTD.) 24 September 2015, paragraphs [0002]-[0003], [0007], page 8, examples, etc. & US 2017/0015639 A1, paragraphs [0002]-[0004], [0031], pp. 3-4, examples, etc. & EP 3121168 A1 & CN 106068257 A | 1-20 |
| X | WO 2016/104317 A1 (DIC CORPORATION) 30 June 2016, paragraphs [0007], [0371], pp. 139-210, examples, claims 1-38 etc. & CN 107108458 A & KR 10-2017-0101194 A | 1-20 |
| X | WO 2016/114211 A1 (DIC CORPORATION) 21 July 2016, paragraphs [0007], [0088], pp. 55-65, examples, claims 1-10, etc. & CN 107108473 A & KR 10-2017-0102242 A | 1-20 |
| X | WO 2016/136533 A1 (DIC CORPORATION) 01 September 2016, paragraph [0007], pp. 58-85, 89, examples, claims 1-9, etc. & CN 107207419 A | 1-20 |
| X | WO 2016/159193 A1 (NIPPON ZEON CO., LTD.) 06 October 2016, paragraphs [0007], page 4, examples, claim 1, etc. & JP 2017-52761 A & TW 201700450 A | 1-20 |
| PX | WO 2016/194999 A1 (FUJIFILM CORP.) 08 December 2016, pp. 10-17, 21-26, examples, claims 1-10, etc. (Family: none) | 1-20 |
| PX | JP 6055569 B1 (NIPPON ZEON CO., LTD.) 27 December 2016, examples, claims 1-22, etc. (Family: none) | 1-20 |
| PX | WO 2017/038266 A1 (DIC CORPORATION) 09 March 2017, paragraph [0022], pp. 107-113, examples, claims 1-12, etc. (Family: none) | 1-20 |
| PX | WO 2017/068860 A1 (DIC CORPORATION) 27 April 2017, paragraphs [0007], pp. 39-65, examples, claims 1-10, etc. (Family: none) | 1-20 |

Form PCT/ISA/210 (continuation of second sheet) (January 2015)

INTERNATIONAL SEARCH REPORT

International application No. PCT/JP2017/040466

C (Continuation). DOCUMENTS CONSIDERED TO BE RELEVANT

| Category* | Citation of document, with indication, where appropriate, of the relevant passages | Relevant to claim No. |
|---|---|---|
| PX | JP 6146526 B1 (NIPPON ZEON CO., LTD.) 14 June 2017, paragraph [0008], examples, claims 1-19, etc. (Family: none) | 1-20 |
| PX | WO 2017/098988 A1 (DIC CORPORATION) 15 June 2017, paragraph [0027], pp. 64-83, examples, claims 1-13, etc. (Family: none) | 1-20 |
| PX | WO 2017/098952 A1 (DIC CORPORATION) 15 June 2017, paragraph [0067], pp. 33-38, examples, claims 1-15, etc. (Family: none) | 1-20 |
| PX | WO 2017/154588 A1 (DIC CORPORATION) 14 September 2017, paragraph [0038], examples, claims 1-18, etc. (Family: none) | 1-20 |
| PX | WO 2017/169839 A1 (DIC CORPORATION) 05 October 2017, pp. 25-26, examples, claims 1-12, etc. (Family: none) | 1-20 |
| EX | WO 2017/199862 A1 (NIPPON ZEON CO., LTD.) 23 November 2017, paragraph [0006], examples, claims 1-27, etc. (Family: none) | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A1
- JP 2007002208 A
- JP 2009173893 A
- JP 2009274984 A
- JP 2010030979 A
- JP 2010031223 A
- JP 2011006360 A
- JP 2010024438 A
- WO 2012141245A1 A
- WO 2012147904 A1
- WO 2012169424 A1
- WO 201276679 A1
- WO 2013180217 A1
- WO 2014061709 A1
- WO 2014065176 A1
- WO 2014126113 A1
- WO 2015025793 A1
- WO 2015064698 A1
- WO 2015122384 A1
- WO 2015122385 A1
- JP H05310845 A
- US 5179171 A
- JP H0597978 A
- US 5202388 A
- JP H11124429 A
- WO 9920676 A1


### Non-patent literature cited in the description


- March's Advanced Organic Chemistry. Wiley
- **SANDLER ; KARO.** Syntheses of Organic Compounds Classified by Functional Group. Hirokawa Publishing Company